(19) Europäisches Patentamt
European Patent Office
Office européen des brevets

(11) EP 2 898 895 A1

(12) **EUROPEAN PATENT APPLICATION**

(43) Date of publication:
29.07.2015 Bulletin 2015/31

(51) Int Cl.:
$A61K\ 38/26^{(2006.01)}$      $A61K\ 38/28^{(2006.01)}$
$A61P\ 3/10^{(2006.01)}$      $A61P\ 5/50^{(2006.01)}$

(21) Application number: 14197154.9

(22) Date of filing: 27.05.2011

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 28.05.2010 EP 10164368

(62) Document number(s) of the earlier application(s) in accordance with Art. 76 EPC:
11723919.4 / 2 575 865

(71) Applicant: Sanofi-Aventis Deutschland GmbH
65929 Frankfurt am Main (DE)

(72) Inventors:
• Becker, Reinhard
 65929 Frankfurt am Main (DE)
• Hauck, Gerrit
 65929 Frankfurt am Main (DE)

(74) Representative: Weickmann & Weickmann
Postfach 860 820
81635 München (DE)

Remarks:
This application was filed on 10-12-2014 as a divisional application to the application mentioned under INID code 62.

(54) **Pharmaceutical composition comprising AVE0010 and insulin glargine**

(57) Subject of the present invention is a pharmaceutical composition comprising (a) desPro$^{36}$Exendin-4 (1-39)-Lys$_6$-NH$_2$ or/and a pharmaceutically acceptable salt thereof, and (b) insulin glargine or/and a pharmaceutically acceptable salt thereof.

SEQUENCE LISTING

<110>   Sanofi-Aventis Deutschland GmbH

<120>   Pharmaceutical composition comprising AVE0010 and insulin
        glargine

<130>   48026P EP-WO-1

<140>   EP 14197154.9
<141>   2011-05-27

<150>   EP 10164368.2
<151>   2010-05-28

<160>   2

<170>   PatentIn version 3.5

<210>   1
<211>   44
<212>   PRT
<213>   Artificial

<220>
<223>   desPro36-Exendin-4(1-39)-Lys6-NH2

<400>   1

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Ser Lys Lys Lys Lys Lys Lys
            35                  40


<210>   2
<211>   39
<212>   PRT
<213>   Heloderma suspectum

<400>   2

His Gly Glu Gly Thr Phe Thr Ser Asp Leu Ser Lys Gln Met Glu Glu
1               5                   10                  15


Glu Ala Val Arg Leu Phe Ile Glu Trp Leu Lys Asn Gly Gly Pro Ser
            20                  25                  30


Ser Gly Ala Pro Pro Pro Ser
            35

**Description**

Subject of the present invention is a pharmaceutical composition comprising (a) desPro36Exendin-4(1-39)-Lys$_6$-NH$_Z$ (AVE0010, lixisenatide) or/and a pharmaceutically acceptable salt thereof, and (b) insulin glargine or/and a pharmaceutically acceptable salt thereof, wherein the concentration of compound (a) is in the range of 20 - 120 μg/ml, and wherein the concentration of compound (b) is in the range of 40 - 200 U/ml.

Insulin is a polypeptide having 51 amino acid residues. Insulin consists of the A chain having 21 amino acid residues, and the B chain having 30 amino acid residues. The chains are coupled by 2 disulfide bridges. Insulin formulations have been used for a long time for therapy of diabetes mellitus type 1 and 2. Recently, insulin derivatives and insulin analogues have been used.

However, control of diabetes mellitus by insulin alone may be insufficient and thus, additional measures may be required.

In the present invention, the relative bioavailability of insulin glargine and AVE0010 given separately but simultaneously versus given in mixes is assessed. Further, the activity of insulin glargine and AVE0010 given separately but simultaneously versus given in mixes as subcutaneous single dosing is compared.

Insulin glargine and lixisenatide are efficacious when given once daily, they are administered subcutaneously and share similar physicochemical features, such as good solubility at low pH. This would allow the preparation of a mix (on-site mixed, in-device mixed or premixed) solution in which lixisenatide is mixed with insulin glargine, so that separate simultaneous injections may be replaced by one single injection delivering both components as a mixed formulation. However, it is not clear if a combined formulation of lixisentatide and insulin glargine would be as effective as separate formulations.

In the present invention, assessed is the exposure and the glucodynamic activity of such a mixture consisting of an insulin glargine/lixisenatide pre-mix compared to the exposure and activity of both drugs when given separately.

As demonstrated by the Examples of the present invention, combined medication of insulin glargine and lixisenatide, given as an on-site mixed formulation, achieves pharmacokinetic equivalence and comparable glucodynamic effect compared to insulin glargine and lixisenatide given separately but simultaneously. Thus, the present invention demonstrates that insulin glargine and lixisenatide can be administered in a single formulation having an equivalent therapeutic effect compared to administration of separate formulations of insulin glargine and lixisenatide.

A first aspect of the present invention is a pharmaceutical composition comprising

(a) desPro$^{36}$Exendin-4(1-39)-Lys$_6$-NH$_2$ or/and a pharmaceutically acceptable salt thereof, and
(b) insulin glargine or/and a pharmaceutically acceptable salt thereof,

wherein the concentration of compound (a) is in the range of 20 - 120 μg/ml, and wherein the concentration of compound (b) is in the range of 40 - 200 U/ml.

It is preferred that the concentration of compound (a) is in the range of 20 - 70 μg/ml. It is also preferred that the concentration of the compound (b) is in the range of 60 - 100 U/ml, most preferred 100 U/ml.

It is also preferred that in the pharmaceutical composition, the concentration of desPro$^{36}$Exendin-4(1-39)-Lys$_6$-NH$_2$ is in the range of 0.2 to 1.0 μg per U insulin glargine, preferably 0.25 to 0.75 μg per U insulin glargine.

The compound desPro$^{36}$Exendin-4(1-39)-Lys$_6$-NH$_2$ (AVE0010, lixisenatide) is a derivative of Exendin-4. AVE0010 is disclosed as SEQ ID NO:93 in WO 01/04156:

SEQ ID NO: 1 AVE0010 (44 AS)

H-G-E-G-T-F-T-S-D-L-S-K-Q-M-E-E-E-A-V-R-L-F-I-E-W-L-K-N-G-G-P-S-S-G-A-
P-P-S-K-K-K-K-K-K-NH$_2$

SEQ ID NO: 2 Exendin-4 (39 AS)

H-G-E-G-T-F-T-S-D-L-S-K-Q-M-E-E-E-A-V-R-L-F-I-E-W-L-K-N-G-G-P-S-S-G-A-
P-P-P-S-NH$_2$

Exendins are a group of peptides which can lower blood glucose concentration. The Exendin analogue AVE0010 is characterised by C-terminal truncation of the native Exendin-4 sequence. AVE0010 comprises six C-terminal lysine residues not present in Exendin-4.

In the context of the present invention, AVE0010 includes pharmaceutically acceptable salts thereof. The person skilled in the art knows pharmaceutically acceptable salts of AVE0010. A preferred pharmaceutically acceptable salt of AVE0010 employed in the present invention is acetate.

AVE0010 or/and a pharmaceutically acceptable salt thereof may be administered in a suitable amount, for instance in an amount in the range of 5 to 15 $\mu$g per dose or 15 to 45 $\mu$g per dose.

In the present invention, AVE0010 or/and a pharmaceutically acceptable salt thereof may be administered in a daily dose in the range of 5 to 15 $\mu$g or in the range of 15 to 45 $\mu$g.

Insulin glargine (Lantus) is Gly(A21)-Arg(B31)-Arg(B32)-human insulin. In the context of the present invention, insulin glargine includes pharmaceutically acceptable salts thereof.

Insulin glargine or/and a pharmaceutically acceptable salt thereof may be administered in a suitable amount, for instance in an amount in the range of 10 to 80 U per dose, preferably 20 - 60 U per dose.

In the present invention, insulin glargine or/and a pharmaceutically acceptable salt thereof may be administered in a daily dose in the range of 10 to 80 U, preferably 20 - 60 U per dose.

The pharmaceutical composition of the present invention may be administered parenterally, e.g. by injection (such as by intramuscular or by subcutaneous injection). Suitable injection devices, for instance the so-called "pens" comprising a cartridge comprising the active ingredient, and an injection needle, are known.

The pharmaceutical composition of the present invention may be administered by one injection per day (once-a-day-dosage). The pharmaceutical composition of the present invention may be provided in a liquid composition suitable for parenteral administration.

A liquid composition employed herein may have an acidic or a physiologic pH. An acidic pH preferably is in the range of pH 1 - 6.8, pH 3.5 - 6.8, or pH 3.5 - 5. A physiologic pH preferably is in the range of pH 2.5 -8.5, pH 4.0 to 8.5, or pH 6.0 to 8.5. The pH may be adjusted by a pharmaceutically acceptable diluted acid (typically HCl) or pharmaceutically acceptable diluted base (typically NaOH).

The liquid composition employed herein may comprise a suitable preservative. A suitable preservative may be selected from phenol, m-cresol, benzyl alcohol and p-hydroxybenzoic acid ester. A preferred preservative is m-cresol.

The liquid composition employed herein may comprise a tonicity agent. A suitable tonicity agent may be selected from glycerol, lactose, sorbitol, mannitol, glucose, NaCl, calcium or magnesium containing compounds such as $CaCl_2$. The concentration of glycerol, lactose, sorbitol, mannitol and glucose may be in the range of 100 - 250 mM. The concentration of NaCl may be up to 150 mM. A preferred tonicity agent is glycerol. The subject to be treated with the pharmaceutical composition of the present invention may have a fasting plasma glucose concentration of at least 7 mmol/L or/and 2 hours postprandial plasma glucose of at least 11.1 mmol/L. The subject may have a HbA1c value in the range of 7% to 10%.

The subject to be treated with the pharmaceutical composition may be an adult subject. The subject may have an age in the range of 18 to 50 years.

The pharmaceutical composition of the present invention preferably is a composition for the treatment of a subject suffering from diabetes mellitus type 2, wherein diabetes mellitus type 2 is not adequately controlled by treatment with insulin alone, for instance with a dose of 10 to 80 U/day insulin for 3 months. In the present invention, a subject the diabetes mellitus type 2 of which is not adequately have a HbA1c value in the range of 7 % to 10%. A subject the diabetes mellitus type 2 of which is not adequately controlled may have a fasting plasma glucose concentration of at least 7 mmol/L or/and 2 hours postprandial plasma glucose of at least 11.1 mmol/L.

The pharmaceutical composition of the present invention may be used for the treatment of diabetes mellitus type 1 or 2 by administration of a dose of 0.25 - 1.5 U/kg insulin glargine and 0.05 - 0.5 $\mu$g/kg desPro$^{36}$Exendin-4(1-39)-Lys$_6$-NH$_2$.

In particular, the pharmaceutical composition of the present invention may be used for the treatment of diabetes mellitus type 2.

The subject to be treated according to the present invention may be an obese subject. In the present invention, an obese subject may have a body mass index of at least 30, in particular suffering from diabetes mellitus type 2

Another aspect of the present invention is a pharmaceutical combination comprising

(a) desPro$^{36}$Exendin-4(1-39)-Lys$_6$-NH$_2$ or/and a pharmaceutically acceptable salt thereof, and
(b) insulin glargine or/and a pharmaceutically acceptable salt thereof,
wherein the concentration of compound (a) is in the range of 20 - 120 $\mu$g/ml, and wherein the concentration of compound (b) is in the range of 40 - 200 U/ml.

The combination of the present invention may be administered as described herein in the context of the pharmaceutical composition of the present invention. Preferred concentrations of the compounds (a) and (b) are as described for the pharmaceutical composition of the present invention.

The combination of the present invention may be used in the treatment of diabetes mellitus type 1 or 2 by administration of a dose of 0.25 - 1.5 U/kg insulin glargine and 0.05 - 0.5 $\mu$g/kg desPro$^{36}$Exendin-4(1-39)-Lys$_6$-NH$_2$.

The combination of the present invention may be used for the treatment of diabetes mellitus type 2.

Another aspect of the present invention is the use of a combination of

(a) desPro$^{36}$Exendin-4(1-39)-Lys$_6$-NH$_2$ or/and a pharmaceutically acceptable salt thereof, and
(b) insulin glargine or/and a pharmaceutically acceptable salt thereof,

wherein the concentration of compound (a) is in the range of 20 - 120 μg/ml, and wherein the concentration of compound (b) is in the range of 40 - 200 U/ml, for the preparation of a medicament for the treatment of diabetes mellitus type 1 or 2, in particular diabetes mellitus type 2.

The compounds (a) and (b) may be used for the preparation of a pharmaceutical composition of a medicament for the treatment of diabetes mellitus type 1 or 2, in particular diabetes mellitus type 2, wherein diabetes mellitus type 1 or 2 is treated by administration of a dose of 0.25 - 1.5 U/kg insulin glargine and 0.05 - 0.5 μg/kg desPro$^{36}$Exendin-4(1-39)-Lys$_6$-NH$_2$.

Preferred concentrations of the compounds (a) and (b) as described for the pharmaceutical composition of the present invention may be used.

Yet another aspect of the present invention is a method of treatment of diabetes mellitus type 1 or/and type 2, in particular diabetes mellitus type 2, comprising administering to a subject in need thereof a composition comprising

(a) desPro$^{36}$Exendin-4(1-39)-Lys$_6$-NH$_2$ or/and a pharmaceutically acceptable salt thereof, and
(b) insulin glargine or/and a pharmaceutically acceptable salt thereof,

wherein the concentration of compound (a) is in the range of 20 - 120 μg/ml, and wherein the concentration of compound (b) is in the range of 40 - 200 U/ml.

In the method of the present inveniton, preferred concentrations of the compounds (a) and (b) as described for the pharmaceutical composition of the present invention may be administered.

In the method of the present invention, a pharmaceutical composition as described herein may be administered. Administration may be performed as described herein in the context of the pharmaceutical composition of the present invention.

In the method of the present invention, diabetes mellitus type 1 or 2 is treated by administration of a dose of 0.25 - 1.5 U/kg insulin glargine and 0.,05 - 0.5 μg/kg desPro$^{36}$Exendin-4(1-39)-Lys$_6$-NH$_2$.

The invention is further illustrated by the following figures and examples.

Legends

Figure 1 - Mean (SD) Lixisenatide plasma concentrations for treatment R1 and T1 on linear scales.

Figure 2 - Mean (SD) Lixisenatide plasma concentrations for treatment R2 and T2 on linear scales.

Figure 3 - Mean (SD) Insulin Glargine serum concentrations for treatment R1 and T1 on linear scales.

Figure 4 - Mean (SD) Insulin Glargine serum concentrations for freatment R2 and T2 on linear scales.

Figure 5 - GIR (mean raw and mean smoothed profiles) - treatment=R1 (separate).

Figure 6 - GIR (mean raw and mean smoothed profiles) - treatment=T1 (mixed).

Figure 7 - GIR (mean raw and mean smoothed profiles) - treatment=R2 (separate).

Figure 8 - GIR (mean raw and mean smoothed profiles) - treatment=T2 (mixed).

Figure 9 - Graphical design of the Example 1 study.

Figure 10 - Graphical design of the Example 2 study.

Figure 11 - Mean (SD) Lixisenatide plasma concentrations for treatment reference (separate), test (mix A), and test 2 (mix B) on linear scales.

Figure 12 - Mean (SD) Insulin Glargine serum concentrations for treatment reference (separate), test (mix A), and test 2 (mix B) on linear scales.

Figure 13 - GIR (mean raw and mean smoothed profiles) - treatment=R (separate). GIR = body weight standardized Glucose Infusion Rate. Treatments are dosings of 20$\mu$ g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

Figure 14 - GIR (mean raw and mean smoothed profiles) - treatment=T1 (mix A). GIR = body weight standardized Glucose Infusion Rate. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

Figure 15 - GIR (mean raw and mean smoothed profiles) - treatment=T2 (mix B). GIR = body weight standardized Glucose Infusion Rate. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

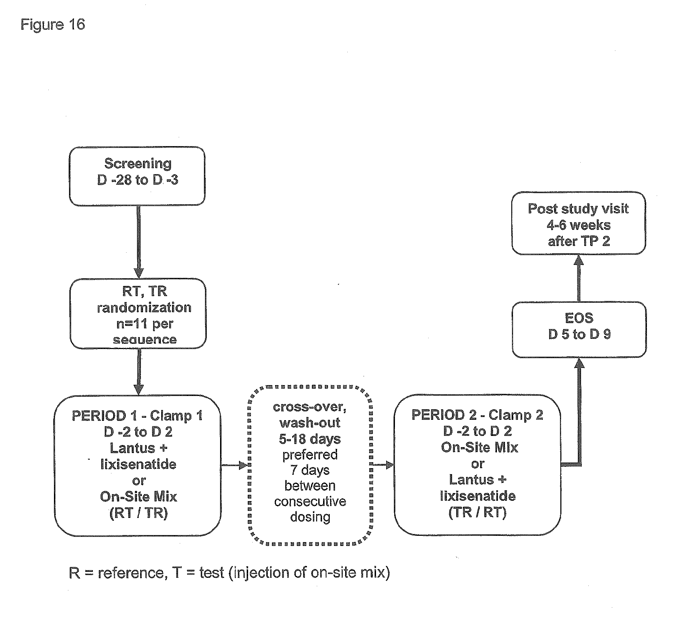

Figure 16 - Graphical study design.

EXAMPLE 1

SYNOPSIS

Title of the study: A randomized, cross-over, open euglycaemic clamp study on the relative bioavailability and activity of two fixed ratio premixed formulations of insulin glargine (Lantus) and AVE0010 compared to separate simultaneous injections of Lantus and AVE0010 in subjects with diabetes mellitus type 1.

Phase of development: Phase I

Primary objective

Assessment of relative bioavailability of insulin glargine and AVE0010 (lixisenatide) given separately simultaneously vs fixed ratio premixed insulin glargine / lixisenatide formulations as subcutaneous (SC) single dosing.

Secondary objectives

•    Comparison of the activity of insulin glargine and lixisenatide given separately simultaneously vs fixed ratio premixed insulin glargine /lixisenatide formulations as SC single dosing
•    Safety and tolerability of fixed ratio premixed insulin glargine - lixisenatide formulations

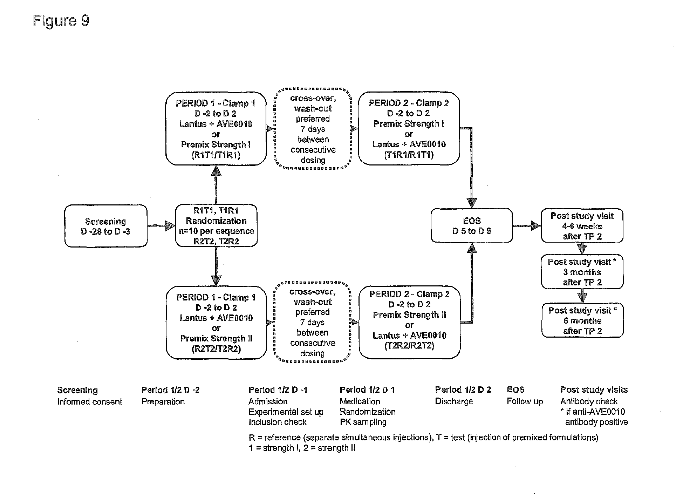

Methodology: Single-center, open, 2 parallel group cross-over euglycaemic clamp study with 2-periods and 2-sequences each of 5-18 days, preferred 7-day wash-out period between treatment periods, randomized for sequences R1 T1, T1 R1, R2 T2, T2 R2 (see Figure 9).
Treatment T1 was given as a single injection of the modified premixed lixisenatide-insulin glargine formulation of strength 1 (0.66 $\mu$g lixisenatide per 1 U insulin glargine) and treatment T2 as a single injection of the modified premixed lixisenatide-insulin glargine formulation of strength 2 (0.25 $\mu$g lixisenatide per 1 U insulin glargine). Reference treatments, R1 and R2, were given as separate but simultaneous injections of the commercialized Lantus U100 and current clinically developmental lixisenatide formulation (R1 and R2) with matching lixisenatide dosing (R1 with 0.66 $\mu$g lixisenatide per 1 U insulin glargine and R2 with 0.25 $\mu$g lixisenatide per 1 U insulin glargine).

Number of subjects

Planned: 40, Randomized: 43, Treated: 42 (total)

Evaluated

Pharmacokinetics: 42, Pharmacodynamics: 42, Safety: 42

Diagnosis and criteria for inclusion: Male and female subjects between 18 and 65 years of age with type 1 diabetes mellitus for more than one year; average total insulin dose of < 1.0 U/kg/day; body mass index between 18.0 and 30.0 kg/m$^2$ inclusive; fasting negative serum C-peptide (< 0.3 nmol/L); glycohemoglobin (HbA1c) ≤9%; stable insulin regimen for at least 2 months prior to the study (with respect to the safety of the subject and scientific integrity of the study)

Key exclusion criteria

- Any history or presence of clinically relevant cardiovascular, pulmonary, gastro-intestinal (such as pancreatitis), hepatic, renal, metabolic (apart from type 1 diabetes mellitus), hematological, neurological, psychiatric, systemic (affecting the body as a whole), ocular, gynecologic (if female), or infectious disease; any acute infectious disease or signs of acute illness

- More than one episode of severe hypoglycemia with seizure, coma or requiring assistance of another person during the previous 6 months

Investigational Product: Subcutaneous (SC) injection of 0.4 U/kg Lantus and 0.100 or 0.264 µg/kg lixisenatide separately simultaneously at opposite peri-umbilical sites within 1 min, or injection of premixed formulations strength I or strength II at one peri-umbilical site

• T1 (Test 1): injection of 0.4 U/kg insulin glargine and 0.264 µg/kg lixisenatide of a premixed formulation, strength I, containing 100 U/mL insulin glargine and 66 µg/mL lixisenatide, at one peri-umbilical site
• T2 (Test 2): injection of 0.4 U/kg insulin glargine and 0.100 µg/kg lixisenatide of a premixed formulation, strength II, containing 100 U/mL insulin glargine and 25 µg/mL lixisenatide, at one peri-umbilical site
• Dose Investigational Product

| Compound | Dose per kg | Form |
|---|---|---|
| Lantus U100 | 0.4 U | 100 U/mL for injection |
| Lixisenatide-insulin glargine, strength I | 0.264 µg + 0.4 U | 66 µg/mL + 100 U/mL for injection |
| Lixisenatide-insulin glargine, strength II | 0.100 µg + 0.4 U | 25 µg/mL + 100 U/mL for injection |

• Administration: Subcutaneous injection

Reference Therapy

• R1 (Reference 1): separate simultaneous injections of 0.4 U/kg Lantus U100 and 0.264 µg/kg lixisenatide (100 µg/mL) at opposite peri-umbilical sites
• R2 (Reference 2): separate simultaneous injections of 0.4 U/kg Lantus U100 and 0.100 µg/kg lixisenatide (100 µg/mL) at opposite peri-umbilical sites
• Dose Reference Therapy

| Compound | Dose per kg | Form |
|---|---|---|
| Lixisenatide (for R1) | 0.264 µg | 100 µg/mL for injection |
| Lixisenatide (for R2) | 0.100 µg | 100 µg/mL for injection |

• Administration: Subcutaneous injection

*Table 1 - Composition of Reference and Investigational Products*

| Components [a] | Strength 1 0.066 / 3.6378 | Strength 2 0.025 / 3.6378 | Lantus U100 100 U/mL | Lixisenatide 100 $\mu$g/mL | Function |
|---|---|---|---|---|---|
| | per mL [mg] | per mL [mg] | per mL [mg] | per mL [mg] | |
| AVE0010 | 0.0660 | 0.0250 | -- | 0.1 | Drug substance |
| Insulin glargine | 3.6378 | 3.6378 | 3.6378 | -- | Drug substance |
| Zinc chloride [b] | 0.0626 | 0.0626 | 0.0626 | -- | Stabilizing agent |
| Glycerol 85% | 20.000 | 20.000 | 20.000 | 18.000 | Tonicity agent |
| Methionine | 3.000 | 3.000 | -- | 3.000 | Stabilizing agent |
| Metacresol [c] | 2.700 | 2.700 | 2.700 | 2.700 | Antimicrobial preservative |
| Sodium acetate | -- | -- | -- | 3.5 | Buffering agent |
| Sodium hydroxide | q.s. pH 4.5 | q.s. pH 4.5 | q.s. pH 4.0 | q.s. pH 4.5 | Alkalizing agent |
| Hydrochloric acid, concentrated | q.s. pH 4.5 | q.s. pH 4.5 | q.s. pH 4.0 | q.s. pH 4.5 | Acidifying agent |
| Water for injection | Ad 1.0 mL | Ad 1.0 mL | Ad 1.0 mL | Ad 1.0 mL | Solvent |

[a] Components are listed according to their pharmcopoeial names. If more than one monograph exists, other names are given in brackets, along with the compendial origin. [b] Composition gives total zinc chloride amount from insulin glargine and from the manufacturing of the drug product. [c] For Metacresol, the common chemical name "m-cresol" is also used within this document.

Duration of treatment: Total study duration for one subject: about 1 month Duration of each part of the study for one subject

- Screening: 1 to 26 days (D-28 to D-3)
- Period 1: 2 days (1 overnight stay)
- Washout: 5 - 18 days, preferred 7 days between consecutive dosings
- Period 2: 2 days (1 overnight stay)
- End-of-study visit: 1 day between D5 and D9 of trial period 2
- Post-study visit / Anti AVE0010 anti-body check: 4 to 6 weeks after last dosing,

Duration of observation: The observation period started at the time the subject signed the informed consent form and ended with the first scheduled post study visit.

Criteria for evaluation

- Pharmacokinetics

    - Lixisenatide: The area under the plasma lixisenatide concentration curve (AUC) as $AUC_{last}$ and AUC, apparent clearance (CL/F), apparent volume of distribution (Vz/F), and terminal half life $t_1/_{2\lambda z}$ was derived, and peak concentration $C_{max}$, and time to $C_{max}$ ($T_{max}$) was observed.
    - Insulin glargine: The area under the plasma insulin glargine concentration curve (AUC) up to 24 h ($AUC_{0-24h}$) and the time to 50% of $AUC_{0-24h}$ was derived. In addition, $C_{max}$ and time to $C_{max}$ ($T_{max}$) was observed.

Pharmacokinetic sampling times and bioanalytical methods

PK sampling times

T0 defines time of injection of study medication.

Blood was collected for the determination of plasma lixisenatide concentrations at T0 and T0.25, T0.5, T1, T1.5, T2, T2.5, T3, T4, T5, T6, T8, T12 and T24 h after injection of study medication and for the determination of plasma insulin glargine concentrations at T0 and T0.25, T0.5, T1, T1.5, T2, T4, T6, T8, T10, T12, T14, T16, T18, T20, T22 and T24 h after injection of study medication.

Methods used to determine pharmacokinetic variables

The following pharmacokinetic (PK) parameters were calculated, using non-compartmental methods for lixisenatide and insulin glargine plasma concentrations after single dose:

| Parameters | Drug/Analyte | Definition/Calculation |
|---|---|---|
| $C_{max}$ | Lixisenatide / Insulin glargine | Maximum plasma concentration observed |
| $T_{max}$ | Lixisenatide / Insulin glargine | First time to reach $C_{max}$ |
| $AUC_{last}$ | Lixisenatide | Area under the plasma concentration versus time curve calculated using the trapezoidal method from time zero to the real time, $t_{last}$ (time corresponding to the last concentration above the limit of quantification, $C_{last}$) |
| $AUC_{0-24h}$ | Insulin glargine | Area under the plasma concentration versus time curve calculated using the trapezoidal method from time zero to 24 hours post dosing |
| AUC | Lixisenatide | Area under the plasma concentration versus time curve extrapolated to infinity according to the following equation: $AUC = AUC_{last} + \dfrac{C_{last}}{3Z}$ |

- Pharmacodynamics: For the pharmacodynamics (PD) of insulin glargine and lixisenatide given separately but simultaneously and given as premixed formulations, the blood glucose concentration and glucose infusion rate was continuously recorded during the clamp procedure.
  PD parameters: The area under the body weight standardized glucose infusion rate (GIR) within 24 h and the time to 50% of the total GIR-AUC within 24 h was calculated. In addition, the maximum of the smoothed GIR ($GIR_{max}$) and the time to $GIR_{max}$, $GIR-T_{max}$, was assessed.

- Safety: Adverse events (AEs) reported by the subject or noted by the Investigator; standard hematology and blood chemistry; urinalysis; vital signs and ECG; anti-AVE0010 antibodies; injections site tolerability.

Statistical methods:

Pharmacokinetics

Pharmacokinetic parameters are summarized by treatment using descriptive statistics. Main statistical analyses are performed separately for strength I (1) and strength II (2). Supplemental statistical analyses were performed to compare treatments across strengths, using a linear fixed effects model.

Lixisenatide: For log transformed AUC, $AUC_{last}$ and $C_{max}$ the relative bioavailability of the respective test treatment (premix vs separate) was assessed using a linear mixed effects model. Estimate and 90% confidence interval (CI) for the ratio of geometric means of the 2 treatments (premix vs separate) are provided for AUC, $AUC_{last}$ and $C_{max}$.

Insulin glargine: For log transformed $AUC_{0-24h}$ the relative bioavailability of the respective test treatment is assessed using a linear mixed effects model. Estimate and 90% CI for the ratio of geometric of the 2 treatments (premix and separate) are provided for $AUC_{0-24h}$. The times to 50% of $AUC_{0-24h}$ were compared non-parametrically between treatments.

Pharmacodynamics

Main statistical analyses were performed separately for strength I (1) and strength II (2).

For log transformed GIR-$AUC_{0-24h}$, the ratio of the 2 treatments (premix vs separate) were assessed using a linear mixed effects model. Estimate and 90% CI for the ratio of geometric means of the two treatments (premix vs separate) were provided for $AUC_{0-24h}$. $GIR_{max}$ was subject to corresponding analysis albeit a supplemental parameter.

The times to 50% of GIR-$AUC_{0-24h}$ were compared non-parametrically between treatments. GIR-$t_{max}$ was subject to corresponding analysis albeit a supplemental parameter. Supplemental statistical analyses were performed to compare treatments across strengths, using a linear fixed effects model and nonparametric analyses, respectively.

Safety and tolerability

The safety analysis is based on the review of the individual values (clinically significant abnormalities) and descriptive statistics by treatment.

For AEs, frequencies of treatment emergent AEs (TEAEs) classified by MedDRA system organ class and preferred term is tabulated by treatment.

For vital signs and ECG, frequency of subjects with abnormalities and potentially clinically significant abnormalities (PCSAs) are summarized by treatment.

Summary

Lixisenatide and insulin glargine were given subcutaneously as premixed lixisenatide-insulin glargine formulations, with modifications in pH and methionine content compared to Lantus U100, at 2 different strengths (Test treatment 1 with strength 1 = 0.66 μg lixisenatide per 1 U insulin glargine, and test treatment T2 with strength 2 = 0.25 μg lixisenatide per 1 U insulin glargine) and compared to separate but simultaneous subcutaneous injections of the commercialized Lantus U100 and current clinically developmental lixisenatide formulation with matching lixisenatide dosing (reference treatment R1 with 0.66 μg and reference treatment R2 with 0.25 μg lixisenatide per 1 U insulin glargine).

The primary objective of the study was to assess relative bioavailabilities of lixisenatide and insulin glargine and the secondary objective was to compare activities between test and reference treatments (T1 vs R1, T2 vs R2). In order to position findings equivalence criteria were employed. The conclusion of equivalence in the formal sense requires the CI of the ratio test/reference to be within the limits of 0.80 and 1.25. For the following, the term "comparable" or "almost equivalent" is used when the acceptance criteria are only marginally violated.

Lixisenatide

Total lixisenatide exposure (AUC) meets equivalence criteria for strength 2 (PE 0.97; 90% CI: 0.83 to 1.13) and almost for strength 1 (PE 0.92; 90% CI: 0.78 to 1.08) as compared to exposure with the current clinical developmental lixisenatide formulation, while maximum concentration was significantly reduced and delayed at either strength.

Additional information from supplemental analysis shows dose-proportionality in lixisenatide exposure, both for test (T1/T2) and reference treatments (R1/R2), and maximum concentration to increase less than proportional with dose.

Insulin glargine

Insulin glargine exposure ($AUC_{0-24h}$) almost meets equivalence criteria for either strength. The point estimates were 0.86 (90% CI: 0.77 to 0.96) and 0.88 (90% CI: 0.79 to 0.98) for the treatment ratios T1/R1 and T2/R2, respectively. Time to 50% of $AUC_{0-24h}$ with R1 and T1 as well as with R2 and T2 treatment was not different, indicating an otherwise unchanged PK profile.

Additional information from supplemental analysis showed insulin glargine exposure not to differ between T1 and T2, on the one hand and between reference treatments, R1 and R2, on the other.

Pharmacodynamic effects: In line with the observations on insulin glargine exposure, pharmacodynamic effects (GIR-$AUC_{0-24h}$) were estimated to be comparable between T1 and R1 (T1/R1: 0.95; 90% CI: 0.76 to 1.18), and similar for T2 and R2, acknowledging that the lower limit of the CI of the treatment ratio was 0.61 (T2/R2: 0.83; 90% CI: 0.61 to 1.12).

Safety: All treatments, R1, R2, T1 and T2 were well tolerated and judged safe for the study population.

Pharmacokinetic results

Lixisenatide: For $AUC_{last}$ and AUC the point estimates for treatment ratios (test/reference) were between 0.82 and 0.97, with only the latter calculated to be within formal equivalence limits.

Total lixisenatide exposure (AUC) meets equivalence criteria for strength 2 (PE 0.97; 90% CI: 0.83 to 1.13) and almost for strength 1 (PE 0.92; 90% CI: 0.78 to 1.08) as compared to exposure with the current clinical developmental lixisenatide formulation. $C_{max}$ of lixisenatide following T1 or T2 administration was less and later than after separate simultaneous administration, not meeting equivalence criteria. $C_{max}$ was 66% and 78% of the separate administrations and $t_{max}$ was achieved 1 h later at T1 and 0.75 h later at T2 as compared to R1 and R2, respectively.

Additional information from supplemental analysis shows dose-proportionality in lixisenatide exposure, both for test (T1/T2) and reference treatments (R1/R2), and maximum concentration to increase less than proportional with dose.

Insulin glargine: For $AUC_{0-24h}$, the point estimates were 0.86 (90% CI: 0.77 to 0.96) and 0.88 (90% CI: 0.79 to 0.98) for the treatment ratios T1/R1 and T2/R2, respectively, indicating, albeit slightly less, comparable insulin glargine exposure after T1 and T2. Time to 50% of $AUC_{0-24h}$ with R1 and T1 as well as with R2 and T2 treatment was not different.

Additional information from supplemental analysis showed insulin glargine exposure not to differ between T1 and T2, on the one hand and between reference treatments, R1 and R2, on the other.

Pharmacodynamic results

In line with the observations on insulin glargine exposure, pharmacodynamic effects (GIR-$AUC_{0-24h}$) were estimated to be comparable between T1 and R1 (T1/R1: 0.95; 90% CI: 0.76 to 1.18), and similar for T2 and R2, acknowledging that the lower limit of the CI of the treatment ratio was 0.61 (T2/R2: 0.83; 90% CI: 0.61 to 1.12). Time to 50% of GIR-$AUC_{0-24h}$ varied by approximately 1 h between treatments, indicating an otherwise unchanged PD profile.

Of note, there may be a modest imbalance in the total effect (GIR-$AUC_{0-24h}$) between R1 and R2, the latter being somewhat stronger (ratio R1/R2 0.86), while the effects after T1 and T2 appear to be similar (ratio T1/T2 0.95).

Safety results

All treatments, R1, R2, T1 and T2 were well tolerated and judged safe for the study population.

Headache was the most common AE (2, 2, 5 and 4 subjects on R1, T1, R2 and T2 respectively), known to be study specific (clamp procedure).

Drug specific AEs were gastrointestinal symptoms (2, 6, 1 and 1 subject(s) on R1, T1, R2 and T2 respectively), linked to lixisenatide.

Conclusions

Total lixisenatide exposure (AUC) meets equivalence criteria for strength 2 (PE 0.97; 90% CI: 0.83 to 1.13) and almost for strength 1 (PE 0.92; 90% CI: 0.78 to 1.08) as compared to exposure with the current clinical developmental lixisenatide formulation, while maximum concentration was significantly reduced and delayed at either strength of the test treatments.

Insulin glargine exposure ($AUC_{0-24h}$) almost meets equivalence criteria for either strength. The point estimates were 0.86 (90% CI: 0.77 to 0.96) and 0.88 (90% CI: 0.79 to 0.98) for the treatment ratios T1/R1 and T2/R2, respectively, at an otherwise unchanged PK profile.

In line with the observations on insulin glargine exposure, pharmacodynamic effects (GIR-$AUC_{0-24h}$) were estimated to be comparable between T1 and R1 (T1/R1: 0.95; 90% CI: 0.76 to 1.18), and similar for T2 and R2, acknowledging that the lower limit of the CI of the treatment ratio was 0.61 (T2/R2: 0.83; 90% CI: 0.61 to 1.12).

All treatments, R1, R2, T1 and T2 were well tolerated and judged as safe for the study population.

1. STUDY SUBJECTS

1.1 SUBJECTS ACCOUNTABILITY

*Table 2 - Overview of Study Populations - by Sequence*

|  | Strength 1 | Strength 2 | All |
|---|---|---|---|
| Safety population | 21 | 21 | 42 |
| Pharmacokinetic population | 21 | 21 | 42 |
|   - lixisenatide | 21 | 21 | 42 |
|   - insulin glargine | 21 | 21 | 42 |
| Pharmacodynamic population | 21 | 21 | 42 |

Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively.
R1 and R2 are separate simultaneous injections; T1 and T2 are injections of premixed formulation

## 1.2 STUDY DISPOSITION

*Table 3 - Overview of Subject Disposition*

| Status | Strength 1 | Strength 2 | All |
|---|---|---|---|
| Included | 21 | 22 | 43 |
| Randomized | 21 | 22 | 43 |
| Discontinued after randomization and before treatment | 0 | 1 | 1 |
|   Reason for discontinuation before treatment: Adverse event | 0 | 1 | 1 |
| Exposed | 21 | 21 | 42 |
|   Treated Period 1 | 21 | 21 | 42 |
|   Treated Period 2 | 21 | 20 | 41 |
| Discontinued treatment | 0 | 1 | 1 |
| Completed study treatment period | 21 | 20 | 41 |
| Reason for treatment/study discontinuation | | | |
|   Poor compliance to protocol | 0 | 1 | 1 |

Strength 1 (R1 and T1) and Strength 2 (R2 and T2) are treatments per kg body weight of 0.4 U/kg insulin glargine combined with 0.264 g/kg and 0.100 $\mu$g/kg lixisenatide respectively.
R1 and R2 are separate simultaneous injections; T1 and T2 are injections of premixed formulation

*Table 4 - Exclusion of Subjects for PK and PD Evaluation*

| Subject | Period | Treatment | PK Lixisenatide | PK insulin glargine | | PD | Safety | Reason for Exclusions |
|---|---|---|---|---|---|---|---|---|
|  |  |  |  |  | > 50% LOQ values [a] |  |  |  |
| 276-001-001 | P2 | T2 Premixed (II) | include | exclude | NA | include | include | not evaluable[c] |
| 276-001-005 | P2 | T1 Premixed (I) | exclude | exclude | NA | exclude | include | flawed injection; i.v. profile |
| 276-001-008 | P1 | T2 Premixed (II) | include | include | exclude | include | include | > 50% LOQ values (insulin glargine) [a] |

(continued)

| Subject | Period | Treatment | PK Lixisenatide | PK insulin glargine | | PD | Safety | Reason for Exclusions |
| | | | | | > 50% LOQ values [a] | | | |
|---|---|---|---|---|---|---|---|---|
| 276-001-008 | P2 | R2 Separate (II) | include | include | exclude | include | include | > 50% LOQ values (insulin glargine) [a] |
| 276-001-033 | P1 | R2 Separate (II) | include | include | exclude | include | include | > 50% LOQ values (insulin glargine) [a] |
| 276-001-033 | P2 | T2 Premixed (II) | include | include | exclude | include | include | > 50% LOQ values (insulin glargine) [a] |
| 276-001-036 | P1 | R1 Separate (1) | include | exclude | exclude | exclude | include | Late exposure to i.v. insulin b |
| 276-001-505 | P1 | R1 Separate (1) | include | include | exclude | include | include | > 50% LOQ values (insulin glargine) [a] |
| 276-001-505 | P2 | T1 Premixed (I) | include | include | exclude | include | include | > 50% LOQ values (insulin glargine) [a] |

[a] AUC and other PK parameter (apart from $C_{max}$ and $t_{max}$) calculation of insulin glargine
[b] insulin glulisine for correction of hyperglycemia
[c] unexplained erratic insulin glargine concentrations

1.3 DEMOGRAPHIC AND BASELINE CHARACTERISTICS

*Table 5 - Summary of Demographic Data - Safety Population*

| | Strength 1 (N=21) | Strength 2 (N=21) | All (N=42) |
|---|---|---|---|
| Sex [n (%)] | | | |
| Male | 19 (90.5%) | 19 (90.5%) | 38 (90.5%) |
| Female | 2 (9.5%) | 2 (9.5%) | 4 (9.5%) |
| Race [n (%)] | | | |
| Caucasian / white | 21 (100%) | 21 (100%) | 42 (100%) |
| Age (yrs) | | | |
| Number | 21 | 21 | 42 |
| Mean (SD) | 40.4 (8.9) | 41.0 (10.0) | 40.7 (9.3) |
| Median | 42.0 | 42.0 | 42.0 |
| Min : Max | 21 : 55 | 20:55 | 20:55 |
| Height (cm) | | | |
| Number | 21 | 21 | 42 |
| Mean (SD) | 177.2 (7.8) | 178.1 (7.8) | 177.6 (7.8) |

(continued)

| | Strength 1 (N=21) | Strength 2 (N=21) | All (N=42) |
|---|---|---|---|
| Median | 180.0 | 180.0 | 180.0 |
| Min : Max | 158 : 188 | 163: 196 | 158:196 |
| Weight (kg) | | | |
| Number | 21 | 21 | 42 |
| Mean (SD) | 79.71 (7.90) | 77.60 (9.69) | 78.65 (8.80) |
| Median | 81.00 | 76.50 | 78.65 |
| Min : Max | 62.2 : 94.6 | 55.7 : 93.8 | 55.7: 94.6 |
| BMI (kg/m$^2$) | | | |
| Number | 21 | 21 | 42 |
| Mean (SD) | 25.39 (1.92) | 24.43 (2.36) | 24.91 (2.18) |
| Median | 25.00 | 24.50 | 24.85 |
| Min : Max | 22.4 : 29.3 | 20.7 : 30.1 | 20.7 : 30.1 |

Note: Number corresponds to the count of subjects with non missing data used for the calculation of percentages. Strength 1 (R1 and T1) and Strength 2 (R2 and T2) are treatments per kg body weight of 0.4 U/kg insulin glargine combined with 0.264 g/kg and 0.100 μg/kg lixisenatide respectively.
R1 and R2 are separate simultaneous injections; T1 and T2 are injections of premixed formulation

### 1.4 DOSAGE AND DURATION

*Table 6 - Overview of Treatment Exposure - Safety Population*

| | Strength 1 | | Strength 2 | |
|---|---|---|---|---|
| Treatment duration (in days) | R1 (separate) (N=21) n (%) | T1 (mixed) (N=21) n (%) | R2 (separate) (N=21) n (%) | T2 (mixed) (N=20) n (%) |
| 1 | 21 (100%) | 21 (100%) | 21 (100%) | 20 (100%) |

n(%)=number and percentage of subject having the corresponding treatment duration. The denominator is N, the number of subjects treated within each group. Strength 1 (R1 and T1) and Strength 2 (R2 and T2) are treatments per kg body weight of 0.4 U/kg insulin glargine combined with 0.264 g/kg and 0.100 μg/kg lixisenatide respectively. R1 and R2 are separate simultaneous injections; T1 and T2 are injections of premixed formulation

### 2 PHARMACOKINETIC EVALUATION

### 2.1 PLASMA CONCENTRATIONS

### 2.1.1 Lixisenatide

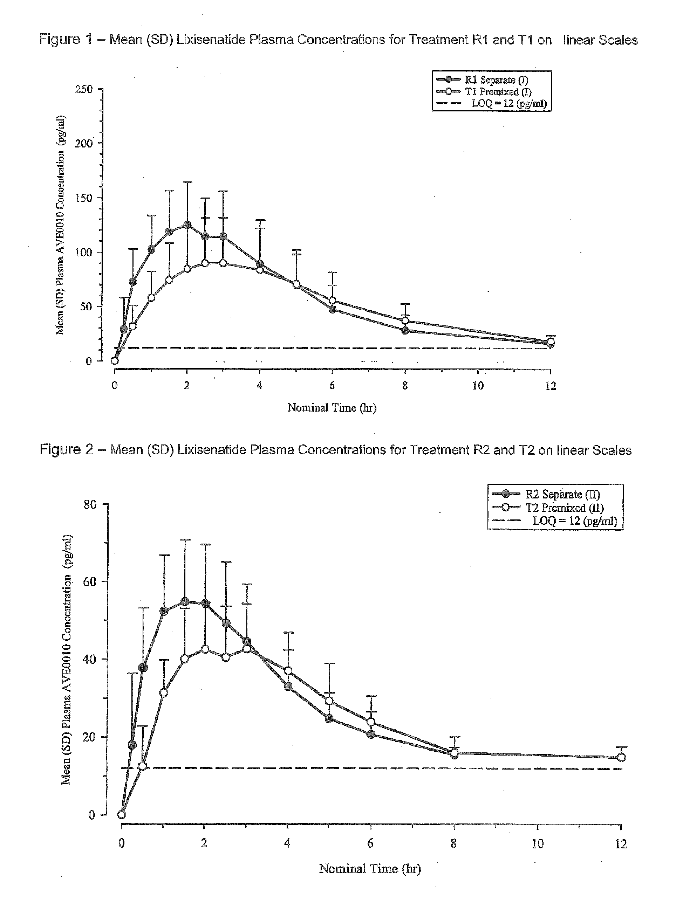

Mean (SD) plasma lixisenatide concentration-time profiles for each treatment are shown in Figure 1 and Figure 2.

### 2.1.2 Insuin Glargine

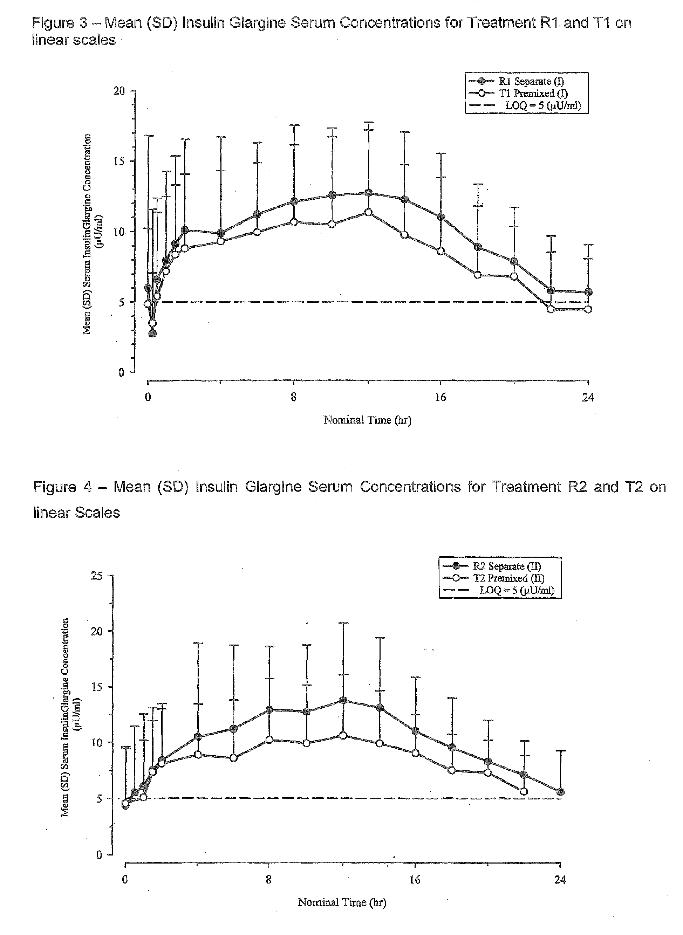

Mean (SD) serum insulin glargine concentration-time profiles for each treatment are shown in Figure 3 and Figure 4. For mean concentration calculation < LLOQ values were taken as zero.

### 2.2 PHARMACOKINETIC PARAMETERS

### 2.2.1 Lixisenatide

Pharmacokinetic parameters for lixisenatide for each treatment are summarized in the table below.

*Table 7 - Lixisenatide Pharmacokinetic Parameters*

| Mean ± SD (Geometric Mean) [CV%] | Plasma Lixisenatide | | | |
| --- | --- | --- | --- | --- |
| | 0.264 µg/kg Lixisenatide | | 0.100 µg/kg Lixisenatide | |
| | R1 Separate (I) | T1 Premixed (I) | R2 Separate (II) | T2 Premixed (II) |
| N | 21 | 20 [a] | 20 | 20 |
| $C_{max}$ (pg/ml) | 137 ± 42.4 (131) [31.0] | 96.8 ± 44.2 (87.3) [45.6] | 60.8 ± 14.0 (59.1) [23.1] | 47.3 ± 11.5 (45.9) [24.3] |
| $t_{max}$ [b] (hr) | 2.00 (1.00 - 4.00) | 3.00 (2.00 - 5.00) | 1.75 (0.50 - 3.00) | 2.50 (1.00 - 3.00) |
| $t_{lag}$ [b] (hr) | 0.00 (0.00 - 0.25) | 0.25 (0.00 - 0.50) | 0.00 (0.00 - 0.50) | 0.25 (0.25 - 0.50) |
| $t_{1/2z}$ (hr) | 2.18 ± 0.43 (2.14) [19.9] | 2.72 ± 0.66 (2.64) [24.2] | 2.20 ± 0.67 (2.11) [30.4] | 2.58 ± 1.17 (2.41) [45.4] |
| $AUC_{last}$ (pg•hr/ml) | 627 ± 236 (588) [37.7] | 559 ± 262 (491) [46.8] | 222 ± 65.2 (212) [29.4] | 213 ± 78.6 (196) [36.9] |
| AUC (pg•hr/ml) | 694 ± 242 (657) [34.9] | 664 ± 243 (620) [36.5] [c] | 280 ± 68.3 (270) [24.4] [d] | 273 ± 73.0 (263) [26.7] [e] |

[a] Profile of Subject 276001005 was excluded, [b] Median (Min - Max), [c] n=19, Subject 276001002 not included in calculation of summary statistics, [d] n=17, Subject 276001023, 276001030, 276001033 not included in calculation of summary statistics, [e] n=16, Subject 276001003, 276001008, 276001015, 276001028 not included in calculation of summary statistics.

2.2.2 Insulin glargine

Pharmacokinetic parameters for insulin glargine for each treatment are summarized in the table below. According to data handling conventions and justifications in the statistical analysis plan (SAP) the concentration of insulin glargine was to be set to zero at t=0 in all samples. Thereafter, < LLOQ concentration results were set to LLOQ/2, that is 2.5 µU/mL for calculation of $AUC_{0-24}$ and other PK parameters. For calculation of mean concentration values including $C_{max}$ < LLOQ concentration results were set to zero.

*Table 8 - Insulin Glargine Pharmacokinetic Parameters*

| Mean ± SD (Geometric Mean) [CV%] | Serum Insulin glargine | | | |
| --- | --- | --- | --- | --- |
| | 0.4 U/kg Insulin glargine | | 0.4 U/kg Insulin glargine | |
| | R1 Separate (I) | T1 Premixed (I) | R2 Separate (II) | T2 Premixed (II) |
| N | 20 [a] | 20 [b] | 21 | 19 [c] |
| $C_{max}$ (µU/ml) | 14.1 ± 5.93 (NA) [42.1] | 13.8 ± 6.99 (12.4) [50.5] | 15.7 ± 9.32 (13.6) [59.6] | 11.7 ± 5.13 (NA) [43.8] |
| $t_{max}$ [d] (hr) | 12.00 (0.25-16.00) | 10.00 (0.25-16.00) | 12.00 (2.00-16.00) | 10.00 (0.25-14.00) |
| $AUC_{0-24}$ (µU•hr/ml) | 255 ± 85.4 (242) [33] [e] | 221 ± 87.3 (206) [39] [e] | 267 ± 96.0 (251) [36] [f] | 221 ± 68.3 (211) [31] [g] |

[a] Profile of Subject 276001036 was excluded, [b] Profile of Subject 276001005 was excluded, [c] Profile of Subject 276001001 was excluded, [d] Median (Min - Max), [e] n=19, Subject 276001505 not included in calculation of summary statistics, [f] n=19, Subject 276001008, 276001033 not included in calculation of summary statistics, [g] n=17, Subject 276001008, 276001033 not included in calculation of summary statistics, NA = Not Applicable.

*Table 9 - Insulin Glargine Pharmacokinetic Parameters $T_{50\%}$-$AUC_{(0-24h)}$ for Insulin Glargine*

|  | Strength 1 | | Strength 2 | |
|---|---|---|---|---|
|  | R1 (separate) | T1 (mixed) | R2 (separate) | T2 (mixed) |
| $T_{50\%}$-$AUC_{(0-24h)}$ (h) | | | | |
| Number | 19 | 19 | 19 | 17 |
| Mean (SD) | 11.384 (0.980) | 11.128 (0.963) | 11.817 (0.970) | 11.560 (0.932) |
| Median | 11.750 | 11.340 | 11.760 | 11.610 |
| Min : Max | 8.56 : 12.64 | 9.36 : 12.94 | 8.48 : 12.99 | 9.85 : 13.47 |

AUC = Area under the insulin glargine concentration versus time curve. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation.

2.3 STATISTICAL ANALYSIS OF LIXISENATIDE PHARMACOKINETIC DATA

2.3.1 Analyses within Strength

*Table 10 - Strength 1 - Parametric Analyses - Treatment Ratios (T1/R1) - Estimates of Treatment Ratio with 90% Confidence Interval*

| Parameter | Comparison | Estimate | 90% CI |
|---|---|---|---|
| $C_{max}$ (pg/ml) | T1 (mixed) / R1 (separate) | 0.66 | (0.57 to 0.77) |
| $AUC_{last}$ (pg.hr/ml) | T1 (mixed) / R1 (separate) | 0.82 | (0.68 to 0.99) |
| AUC (pg.hr/ml) | T1 (mixed) / R1 (separate) | 0.92 | (0.78 to 1.08) |

R1 and T1 (Strength 1) are treatments per kg body weight of 0.4 U/kg insulin glargine and 0.264 $\mu$g/kg lixisenatide. R1 = separate simultaneous injections; T1 = injection of premixed formulation PK parameters are for lixisenatide (AVE0010).

*Table 11 - Strength 2 - Parametric Analyses - Treatment Ratios (T2/R2) - Estimates of Treatment Ratio with 90% Confidence Interval*

| Parameter | Comparison | Estimate | 90% CI |
|---|---|---|---|
| $C_{max}$ (pg/ml) | T2 (mixed) / R2 (separate) | 0.78 | (0.68 to 0.88) |
| $AUC_{last}$ (pg.hr/ml) | T2 (mixed) / R2 (separate) | 0.93 | (0.77 to 1.11) |
| AUC (pg.hr/ml) | T2 (mixed) / R2 (separate) | 0.97 | (0.83 to 1.13) |

R2 and T2 (Strength 2) are treatments per kg body weight of 0.4 U/kg insulin glargine and 0.100 $\mu$g/kg lixisenatide. R2 = separate simultaneous injections; T2 = injection of premixed formulation PK parameters are for lixisenatide (AVE0010).

2.3.2 Supplemental Analyses - across Strengths

*Table 12 - R1 versus R2 - Parametric Analyses - Treatment Ratios (R1/R2) - Estimates of Treatment Ratio with 90% Confidence Interval*

| Parameter | Comparison | Estimate | 90% CI |
|---|---|---|---|
| $C_{max}$ (pg/ml) | R1 (separate) / R2 (separate) | 2.20 | (1.89 to 2.56) |
| $AUC_{last}$ (pg.hr/ml) | R1 (separate) / R2 (separate) | 2.77 | (2.31 to 3.33) |

(continued)

| Parameter | Comparison | Estimate | 90% CI |
|---|---|---|---|
| AUC (pg.hr/ml) | R1 (separate) / R2 (separate) | 2.43 | (2.03 to 2.89) |

Lixisenatide dose ratio for R1/R2 is 2.64. R1 and R2 are treatments per kg body weight of 0.4 U/kg insulin glargine combined with 0.264 μg/kg and 0.100 μg/kg lixisenatide respectively. R1 and R2 are separate simultaneous injections. PK parameters are for lixisenatide (AVE0010).

*Table 13 - T1 versus T2 - Parametric Analyses - Treatment Ratios (T1/T2) - Estimates of Treatment Ratio with 90% Confidence Interval*

| Parameter | Comparison | Estimate | 90% CI |
|---|---|---|---|
| $C_{max}$ (pg/ml) | T1 (mixed) / T2 (mixed) | 1.90 | (1.55 to 2.34) |
| $AUC_{last}$ (pg.hr/ml) | T1 (mixed) / T2 (mixed) | 2.51 | (1.90 to 3.30) |
| AUC (pg.hr/ml) | T1 (mixed) / T2 (mixed) | 2.36 | (1.92 to 2.90) |

Lixisenatide dose ratio for T1/T2 is 2.64. T1 and T2 are treatments per kg body weight of 0.4 U/kg insulin glargine combined with 0.264 μg/kg and 0.100 μg/kg lixisenatide respectively. T1 and T2 are injections of premixed formulations. PK parameters are for lixisenatide (AVE0010).

2.4 STATISTICAL ANALYSIS OF INSULIN GLARGINE PHARMACOKINETIC DATA

2.4.1 Analyses within Strength

*Table 14 - Strength 1- Parametric Analyses - Treatment Ratios (T1/R1) - Estimates of Treatment Ratio with 90% Confidence Interval*

| Parameter | Comparison | Estimate | 90% CI |
|---|---|---|---|
| $AUC_{(0-24h)}$ (μU.hr/ml) | T1 (mixed) / R1 (separate) | 0.86 | (0.77 to 0.96) |

R1 and T1 (Strength 1) are treatments per kg body weight of 0.4 U/kg insulin glargine and 0.264 μg/kg lixisenatide. R1 = separate simultaneous injections; T1 = injection of premixed formulation. PK parameters are for insuline glargine.

*Table 15 - Strength 1- Nonparametric Analyses - Treatment Differences (T1-R1) - $T_{50\%}$-$AUC_{(0-24h)}$ [hours] - Estimate of Treatment Differences*

| Exact Hodges-Lehmann | | |
|---|---|---|
| Comparison | Point estimate | 90% confidence interval |
| T1 vs. R1 | -0.3 | (-0.8; 0.1) |

R1 and T1 (Strength 1) are treatments per kg body weight of 0.4 U/kg insulin glargine and 0.264 μg/kg lixisenatide. R1 = separate simultaneous injections; T1 = injection of premixed formulation. PK parameters are for insuline glargine.

*Table 16 - Strength 2 - Parametric Analyses - Treatment Ratios (T2/R2) - Estimates of Treatment Ratio with 90% Confidence Interval*

| Parameter | Comparison | Estimate | 90% CI |
|---|---|---|---|
| $AUC_{(0-24h)}$ (μU.hr/ml) | T2 (mixed) / R2 (separate) | 0.88 | (0.79 to 0.98) |

R2 and T2 (Strength 2) are treatments per kg body weight of 0.4 U/kg insulin glargine and 0.100 μg/kg lixisenatide. R2 = separate simultaneous injections; T2 = injection of premixed formulation PK parameters are for insuline glargine.

*Table 17 - Strength 2 - Nonparametric Analyses - Treatment Differences (T2-R2) - $T_{50\%}$-AUC$_{(0-24h)}$ [hours] - Estimate of Treatment Differences*

| Exact Hodges-Lehmann | | |
|---|---|---|
| Comparison | Point estimate | 90% confidence interval |
| T2 vs. R2 | -0.2 | (-0.7 ; 0.1) |
| R2 and T2 (Strength 2) are treatments per kg body weight of 0.4 U/kg insulin glargine and 0.100 μg/kg lixisenatide. R2 = separate simultaneous injections; T2 = injection of premixed formulation. PK parameters are for insuline glargine. | | |

2.4.2 Supplemental Analyses - across Strengths

*Table 18 - R1 versus R2 - Parametric Analyses - Treatment Ratios (R1/R2) - Estimates of Treatment Ratio with 90% Confidence Interval*

| Parameter | Comparison | Estimate | 90% CI |
|---|---|---|---|
| AUC$_{(0-24h)}$ (μU.hr/ml) | R1 (separate) / R2 (separate) | 0.96 | (0.79 to 1.16) |
| R1 and R2 are treatments per kg body weight of 0.4 U/kg insulin glargine combined with 0.264 μg/kg and 0.100 μg/kg lixisenatide respectively. R1 and R2 are separate simultaneous injections. PK parameters are for insuline glargine. | | | |

*Table 19 - R1 versus R2 - Nonparametric Analyses - Treatment Differences (R1-R2) - $T_{50\%}$-AUC$_{(0-24h)}$ [hours] - Estimate of Treatment Differences*

| Exact Hodges-Lehmann | | |
|---|---|---|
| Comparison | Point estimate | 90% confidence interval |
| R1 vs. R2 | -0.3 | (-0.9 ; 0.1) |
| R1 and R2 are treatments per kg body weight of 0.4 U/kg insulin glargine combined with 0.264 μg/kg and 0.100 μg/kg lixisenatide respectively. R1 and R2 are separate simultaneous injections. PK parameters are for insuline glargine. | | |

*Table 20 - T1 versus T2 - Parametric Analyses - Treatment Ratios (T1/T2) - Estimates of Treatment Ratio with 90% Confidence Interval*

| Parameter | Comparison | Estimate | 90% CI |
|---|---|---|---|
| AUC$_{(0-24h)}$ (μU.hr/ml) | T1 (mixed) / T2 (mixed) | 0.97 | (0.80 to 1.19) |
| T1 and T2 are treatments per kg body weight of 0.4 U/kg insulin glargine combined with 0.264 μg/kg and 0.100 μg/kg lixisenatide respectively. T1 and T2 are injections of premixed formulations. PK parameters are for insuline glargine. | | | |

*Table 21 - T1 versus T2 - Nonparametric Analyses - Treatment Differences (T1-T2) - $T_{50\%}$-AUC$_{(0-24h)}$ [hours] - Estimate of Treatment Differences*

| Exact Hodges-Lehmann | | |
|---|---|---|
| Comparison | Point estimate | 90% confidence interval |
| T1 vs. T2 | -0.4 | (-0.9 ; 0.2) |
| T1 and T2 are treatments per kg body weight of 0.4 U/kg insulin glargine combined with 0.264 μg/kg and 0.100 μg/kg lixisenatide respectively. T1 and T2 are injections of premixed formulations. PK parameters are for insuline glargine. | | |

3. PHARMACODYNAMIC EVALUATION

3.1 MAIN ANALYSIS - DESCRIPTIVE STATISTICS AND PLOTS

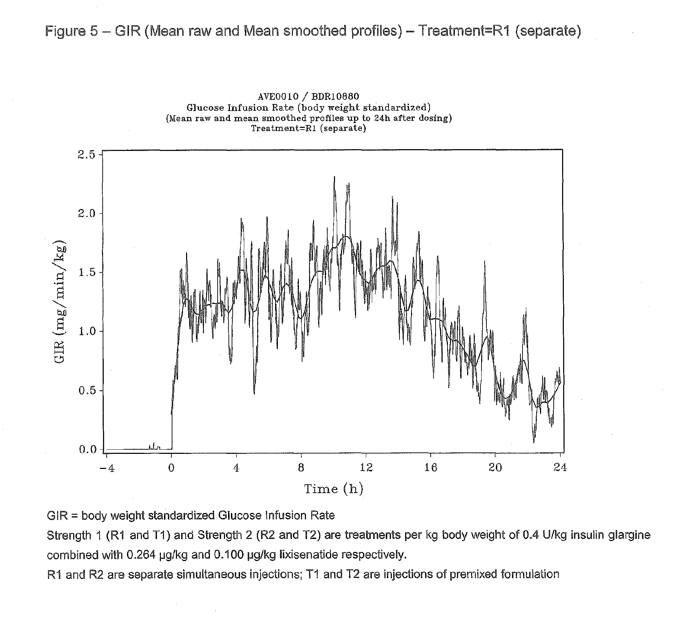

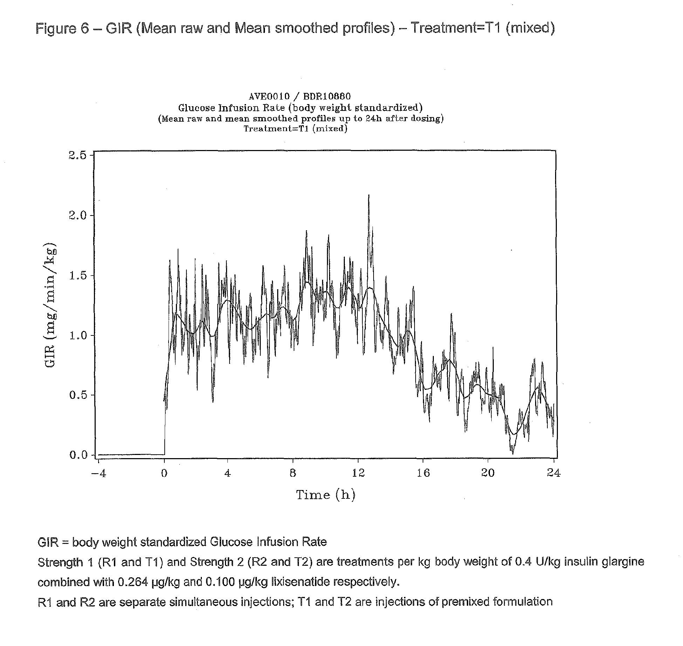

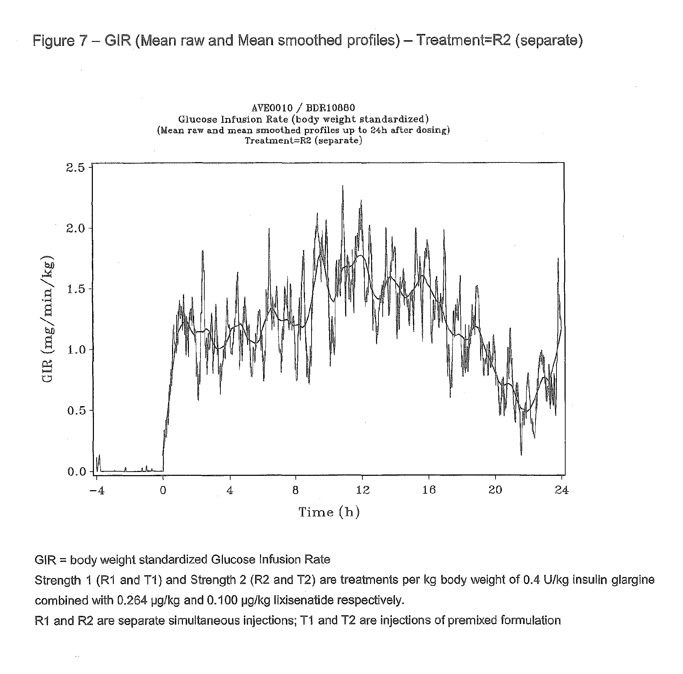

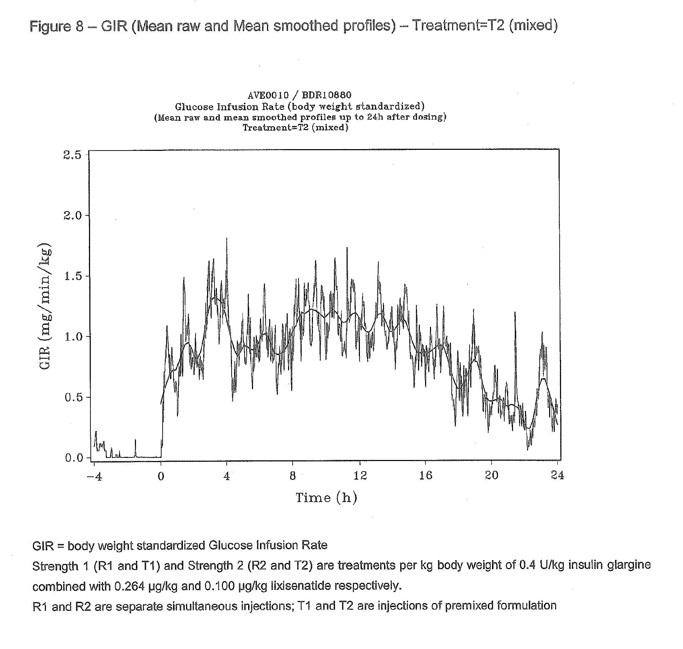

The time course of glucose infusion rate is plotted in Figures 5 to 8

*Table 22 - Descriptive Statistics of GIR-AUC$_{(0-24h)}$ (mglkg)*

| | Strength 1 | | Strength 2 | |
|---|---|---|---|---|
| | R1 (separate) | T1 (mixed) | R2 (separate) | T2 (mixed) |
| GIR-AUC$_{(0-24)}$ (mg/kg) | | | | |
| Number | 20 | 20 | 21 | 20 |
| Geometric Mean | 1232.78 | 1086.25 | 1341.21 | 1093.75 |
| CV% | 63.199 | 62.361 | 56.974 | 50.273 |
| Mean (SD) | 1639.51 (1036.15) | 1340.68 (836.06) | 1709.82 (974.16) | 1256.19 (631.53) |
| Median | 1785.40 | 1230.95 | 1560.50 | 1181.05 |
| Min : Max | 181.9 : 3938.8 | 265.3 : 3262.3 | 55.7 : 3842.1 | 341.1 : 2296.7 |

GIR = body weight standardized glucose infusion rate. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation

*Table 23 - Descriptive Statistics of Time (h) to 50% of GIR-AUC$_{(0-24h)}$*

| | Strength 1 | | Strength 2 | |
|---|---|---|---|---|
| | R1 (separate) | T1 (mixed) | R2 (separate) | T2 (mixed) |
| T$_{50\%}$-GIR-AUC$_{(0-24)}$ (h) | | | | |
| Number | 20 | 20 | 21 | 20 |
| Mean (SD) | 9.757 (2.455) | 8.887 (2.633) | 11.083 (2.917) | 10.206 (2.671) |
| Median | 10.540 | 9.330 | 11.470 | 10.460 |
| Min : Max | 3.03: 12.98 | 1.68 : 12.93 | 0.42: 14.82 | 3.30:13.83 |

GIR = body weight standardized glucose infusion rate. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation.

*Table 24 - Descriptive Statistics of GIR$_{max}$ (mg/kg/min)*

| | Strength 1 | | Strength 2 | |
|---|---|---|---|---|
| | R1 (separate) | T1 (mixed) | R2 (separate) | T2 (mixed) |
| GIR$_{max}$ (mg/kg/min) | | | | |
| Number | 20 | 20 | 21 | 20 |
| Geometric Mean | 2.697 | 2.575 | 2.862 | 2.397 |
| CV% | 47.0856 | 38.5356 | 37.4562 | 28.2595 |
| Mean (SD) | 2.954 (1.391) | 2.756 (1.062) | 3.044 (1.140) | 2.485 (0.702) |
| Median | 2.615 | 2.640 | 2.770 | 2.400 |
| Min : Max | 1.32 : 6.44 | 1.28 : 5.42 | 1.28 : 6.04 | 1.49 : 4.24 |

GIR = body weight standardized glucose infusion rate. GIR$_{max}$ and GIR-tmax are based on smoothed GIR profiles. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation.

*Table 25 - Descriptive Statistics of Time to GIR$_{max}$ (h)*

| | Strength 1 | | Strength 2 | |
|---|---|---|---|---|
| | R1 (separate) | T1 (mixed) | R2 (separate) | T2 (mixed) |
| GIR-t$_{max}$ (h) | | | | |
| Number | 20 | 20 | 21 | 20 |
| Mean (SD) | 9.989 (7.841) | 7.918 (6.600) | 11.371 (7.899) | 7.561 (4.484) |
| Median | 9.475 | 7.700 | 11.630 | 7.600 |
| Min : Max | 0.00 : 24.00 | 0.00:20.82 | 0.00 : 24.00 | 0.75 : 18.95 |

GIR = body weight standardized glucose infusion rate. GIR$_{max}$ and GIR-tmax are based on smoothed GIR profiles. Strength 1 (R1 and T1) and Strength 2 (R2 and T2) are treatments per kg body weight of 0.4 U/kg insulin glargine combined with 0.264 μg/kg and 0.100 μg/kg lixisenatide respectively. R1 and R2 are separate simultaneous injections; T1 and T2 are injections of premixed formulation.

3.2 MAIN ANALYSIS - COMPARATIVE ANALYSES WITHIN STRENGTH

*Table 26 - Strength 1- Parametric Analyses - Treatment Ratios (T1/R1) - Estimates of Treatment Ratio with 90% Confidence Interval*

| Parameter | Comparison | Estimate | 90% CI |
|---|---|---|---|
| GIR-AUC$_{(0-24h)}$ (mg/kg) | T1 (mixed) / R1 (separate) | 0.95 | (0.76 to 1.18) |
| GIR$_{max}$ (mg/kg/min) | T1 (mixed) / R1 (separate) | 0.99 | (0.86 to 1.14) |

R1 and T1 (Strength 1) are treatments per kg body weight of 0.4 U/kg insulin glargine and 0.264 μg/kg lixisenatide. R1 = separate simultaneous injections; T1 = injection of premixed formulation. GIRmax and GIR-tmax are based on smoothed GIR profiles.

*Table 27 - Strength 1- Nonparametric Analyses - Treatment Differences (T1-R1) - T$_{50\%}$-GIR-AUC($_{0-24h}$) [hours] - Estimate of Treatment Differences*

| Exact Hodges-Lehmann | | |
|---|---|---|
| Comparison | Point estimate | 90% confidence interval |
| T1 vs. R1 | -0.8 | (-1.9 ; 0.4) |

R1 and T1 (Strength 1) are treatments per kg body weight of 0.4 U/kg insulin glargine and 0.264 μg/kg lixisenatide. R1 = separate simultaneous injections; T1 = injection of premixed formulation

*Table 28 - Strength 2 - Parametric Analyses - Treatment Ratios (T2/R2) - Estimates of Treatment Ratio with 90% Confidence Interval*

| Parameter | Comparison | Estimate | 90% CI |
|---|---|---|---|
| GIR-AUC$_{(0-24h)}$ (mg/kg) | T2 (mixed) / R2 (separate) | 0.83 | (0.61 to 1.12) |
| GIR$_{max}$ (mg/kg/min) | T2 (mixed) / R2 (separate) | 0.85 | (0.76 to 0.95) |

R2 and T2 (Strength 2) are treatments per kg body weight of 0.4 U/kg insulin glargine and 0.100 μg/kg lixisenatide. R2 = separate simultaneous injections; T2 = injection of premixed formulation. GIRmax and GIR-tmax are based on smoothed GIR profiles.

*Table 29 - Strength 2 - Nonparametric Analyses - Treatment Differences (T2-R2) - $T_{50\%}$- GIR-AUC$_{(0-24h)}$ [hours] - Estimate of Treatment Differences*

| Exact Hodges-Lehmann | | |
|---|---|---|
| Comparison | Point estimate | 90% confidence interval |
| T2 vs. R2 | -0.8 | (-1.3;-0.4) |

R2 and T2 (Strength 2) are treatments per kg body weight of 0.4 U/kg insulin glargine and 0.100 μg/kg lixisenatide. R2 = separate simultaneous injections; T2 = injection of premixed formulation.

3.3 OTHER ANALYSIS

3.3.1 Analyses within Strength

*Table 30 - Strength 1 - Nonparametric Analyses - Treatment Differences (T1-R1) - GIR-$t_{max}$ [hours] - Estimate of Treatment Difference*

| Exact Hodges-Lehmann | | |
|---|---|---|
| Comparison | Point estimate | 90% confidence interval |
| T1 vs. R1 | -1.3 | (-5.1 ; 1.7) |

R1 and T1 (Strength 1) are treatments per kg body weight of 0.4 U/kg insulin glargine and 0.264 μg/kg lixisenatide. R1 = separate simultaneous injections; T1 = injection of premixed formulation. GIRmax and GIR-tmax are based on smoothed GIR profiles.

*Table 31 - Strength 2 - Nonparametric Analyses - Treatment Differences (T2-R2) - GIR-$t_{max}$ [hours] - Estimate of Treatment Differences*

| Exact Hodges-Lehmann | | |
|---|---|---|
| Comparison | Point estimate | 90% confidence interval |
| T2 vs. R2 | -3.8 | (-7.3 ; -0.1) |

R2 and T2 (Strength 2) are treatments per kg body weight of 0.4 U/kg insulin glargine and 0.100 μg/kg lixisenatide. R2 = separate simultaneous injections; T2 = injection of premixed formulation. GIRmax and GIR-tmax are based on smoothed GIR profiles.

3.3.2 Supplemental Analyses - across Strength

*Table 32 - R1 versus R2 - Parametric Analyses - Treatment ratios (R1/R2) - Estimates of Treatment Ratio with 90% Confidence Interval*

| Parameter | Comparison | Estimate | 90% CI |
|---|---|---|---|
| GIR-AUC$_{(0-24h)}$ (mg/kg) | R1 (separate) / R2 (separate) | 0.92 | (0.57 to 1.47) |
| GIR$_{max}$ (mg/kg/min) | R1 (separate) / R2 (separate) | 0.94 | (0.77 to 1.16) |

R1 and R2 are treatments per kg body weight of 0.4 U/kg insulin glargine combined with 0.264 μg/kg and 0.100 μg/kg lixisenatide respectively. R1 and R2 are separate simultaneous injections. GIRmax and GIR-tmax are based on smoothed GIR profiles.

*Table 33 - R1 versus R2 - Nonparametric Analyses - Treatment Differences (R1-R2) - $T_{50\%}$-GIR-AUC($_{0-24h}$) [hours] - Estimate of Treatment Differences*

| Exact Hodges-Lehmann | | |
|---|---|---|
| Comparison | Point estimate | 90% confidence interval |
| R1 vs. R2 | -1.3 | (-2.4 ; -0.3) |

R1 and R2 are treatments per kg body weight of 0.4 U/kg insulin glargine combined with 0.264 μg/kg and 0.100 μg/kg lixisenatide respectively. R1 and R2 are separate simultaneous injections.

*Table 34 - R1 versus R2 - Nonparametric Analyses - Treatment Differences (R1-R2) - GIR-$t_{max}$ [hours] - Estimate of Treatment Differences*

| Exact Hodges-Lehmann | | |
|---|---|---|
| Comparison | Point estimate | 90% confidence interval |
| R1 vs. R2 | -0.8 | (-6.0; 2.1) |

R1 and R2 are treatments per kg body weight of 0.4 U/kg insulin glargine combined with 0.264 μg/kg and 0.100 μg/kg lixisenatide respectively. R1 and R2 are separate simultaneous injections. GIRmax and GIR-tmax are based on smoothed GIR profiles.

*Table 35 - T1 versus T2 - Parametric Analyses - Treatment Ratios (T1/T2) - Estimates of Treatment Ratio with 90% Confidence interval*

| Parameter | Comparison | Estimate | 90% CI |
|---|---|---|---|
| GIR-AUC$_{(0-24h)}$ (mg/kg) | T1 (mixed) / T2 (mixed) | 0.99 | (0.70 to 1.40) |
| GIR$_{max}$ (mg/kg/min) | T1 (mixed) / T2 (mixed) | 1.07 | (0.90 to 1.28) |

T1 and T2 are treatments per kg body weight of 0.4 U/kg insulin glargine combined with 0.264 μg/kg and 0.100 μg/kg lixisenatide respectively. T1 and T2 are injections of premixed formulations. GIRmax and GIR-tmax are based on smoothed GIR profiles.

*Table 36 - T1 versus T2 - Nonparametric Analyses - Treatment Differences (T1-T2) - $T_{50\%}$-GIR-AUC$_{(0-24h)}$ [hours] - Estimate of Treatment Differences*

| Exact Hodges-Lehmann | | |
|---|---|---|
| Comparison | Point estimate | 90% confidence interval |
| T1 vs. T2 | -1.4 | (-2.5 ; -0.4) |

T1 and T2 are treatments per kg body weight of 0.4 U/kg insulin glargine combined with 0.264 μg/kg and 0.100 μg/kg lixisenatide respectively. T1 and T2 are injections of premixed formulations.

*Table 37- T1 versus T2 - Nonparametric Analyses - Treatment Differences (T1-T2) - GIR-$t_{max}$[hours]-Estimate of Treatment Differences*

| Exact Hodges-Lehmann | | |
|---|---|---|
| Comparison | Point estimate | 90% confidence interval |
| T1 vs. T2 | 0.0 | (-3.0 ; 3.0) |

T1 and T2 are treatments per kg body weight of 0.4 U/kg insulin glargine combined with 0.264 μg/kg and 0.100 μg/kg lixisenatide respectively. T1 and T2 are injections of premixed formulations. GIRmax and GIR-tmax are based on smoothed GIR profiles.

## 4. SAFETY EVALUATION

### 4.1 TREATMENT EMERGENT ADVERSE EVENTS

A total of 36 adverse events (AEs) occurred in 21 subjects.

The most common AE was headache with 15 subjects (a common observation in clamp settings regardless of medication), and 13 AEs for gastrointestinal symptoms in 9 subjects, which were considered lixisenatide-specific by the Investigator. There were 6 cases of nausea reported for 5 subjects. Other gastrointestinal events were single cases of vomiting (2 events in 1 subject), diarrhea (3 events in 2 subjects), heartburn (1 event in 1 subject) and gastrospasm (1 event in 1 subject).

Table 39 (evaluation by treatment) shows that headache was the most common AE (2, 2, 5 and 4 subjects on R1, T1, R2 and T2 respectively), known to be study specific (clamp procedure). Investigational product-specific AEs were gastrointestinal symptoms (2, 6, 1 and 1 subject(s) on R1, T1, R2 and T2 respectively), linked to lixisenatide.

All treatments, R1, R2, T1 and T2 were well tolerated and judged as safe for the study population.

*Table 38 - Overview of Treatment-Emergent Adverse Events - Safety Population*

| | Strength 1 | | Strength 2 | |
|---|---|---|---|---|
| | R1 (separat e) (N=21) n (%) | T1 (mixed) (N=21) n (%) | R2 (separat e) (N=21) n (%) | T2 (mixed) (N=20) n (%) |
| AnyTEAE | 5 (23.8%) | 8 (38.1%) | 7 (33.3%) | 5 (25.0%) |
| Any severe TEAE | 0 | 0 | 0 | 0 |
| Any serious TEAE | 0 | 0 | 0 | 0 |
| Any TEAE leading to permanent treatment discontinuation | 0 | 0 | 0 | 0 |

TEAE = Treatment Emergent Adverse Event. N = Number of subjects exposed, n(%) = Number and % of subjects with at least one TEAE. An adverse event is considered as treatment emergent if it occurred from the time of investigational product (IP) administration within the period up to 72 hours later. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation

*Table 39 - Number (%) of Subjects with TEAE by System Organ Class and Preferred Term - Safety Population*

| | Strength 1 | | Strength 2 | |
|---|---|---|---|---|
| Primary System Organ Class Preferred Term | R1 (separate) (N=21) n (%) | T1 (mixed) (N=21) n (%) | R2 (separate) (N=21) n (%) | T2 (mixed) (N=20) n (%) |
| Any class | 5 (23.8%) | 8 (38.1%) | 7 (33.3%) | 5 (25.0%) |
| Nervous system disorders | 2 (9.5%) | 2 (9.5%) | 5 (23.8%) | 4 (20.0%) |
| Headache | 2 (9.5%) | 2 (9.5%) | 5 (23.8%) | 4 (20.0%) |
| Gastrointestinal disorders | 2 (9.5%) | 6 (28.6%) | 1 (4.8%) | 1 (5.0%) |
| Diarrhoea | 0 | 1 (4.8%) | 1 (4.8%) | 1 (5.0%) |
| Nausea | 2 (9.5%) | 3 (14.3%) | 0 | 0 |
| Abdominal pain upper | 0 | 1 (4.8%) | 0 | 0 |
| Dyspepsia | 0 | 1 (4.8%) | 0 | 0 |
| Vomiting | 0 | 1 (4.8%) | 0 | 0 |
| Musculoskeletal and connective tissue disorders | 0 | 1 (4.8%) | 0 | 0 |
| Pain in extremity | 0 | 1 (4.8%) | 0 | 0 |
| General disorders and administration site conditions | 1 (4.8%) | 3 (14.3%) | 1 (4.8%) | 0 |

(continued)

| Primary System Organ Class Preferred Term | Strength 1 | | Strength 2 | |
|---|---|---|---|---|
| | R1 (separate) (N=21) n (%) | T1 (mixed) (N=21) n (%) | R2 (separate) (N=21) n (%) | T2 (mixed) (N=20) n (%) |
| Venipuncture site thrombosis | 0 | 0 | 1 (4.8%) | 0 |
| Catheter site pain | 0 | 1 (4.8%) | 0 | 0 |
| Injection site erythema | 0 | 1 (4.8%) | 0 | 0 |
| Malaise | 0 | 1 (4.8%) | 0 | 0 |
| Pyrexia | 0 | 1 (4.8%) | 0 | 0 |
| Injection site haematoma | 1 (4.8%) | 0 | 0 | 0 |

TEAE = Treatment Emergent Adverse Event. N = Number of subjects exposed, n(%) = Number and % of subjects with at least one TEAE. An adverse event is considered as treatment emergent if it occurred from the time of investigational product (IP) administration within the period up to 72 hours later. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation MedDRA version: 12.0

4.2 SERIOUS ADVERSE EVENTS

There were no serious TEAEs.

4.3 DISCONTINUATION DUE TO ADVERSE EVENTS

One randomized subject was withdrawn prior to dosing because of a trigeminus in the telemetric electrocardiogram (ECG) recording.

4.4 OTHER SAFETY PARAMETERS

*Table 40 - Number of Subjects with on-treatment Abnormalities (PCSA) in ECG Parameters - Safety Population*

| Electrocardiogram (PCSA definition) | Subjects with at least one PCSA (On treatment)/ Evaluable subjects[a] | | | |
|---|---|---|---|---|
| | Strength 1 | | Strength 2 | |
| | R1 (separate) (N=21)[b] | T1 (mixed) (N=21)[b] | R2 (separate) (N=21)[b] | T2 (mixed) (N=20)[b] |
| HR $\leq$ 40 bpm & deer. $\geq$ 20 bpm versus B | 0/21 | 0/21 | 0/21 | 0/20 |
| HR $\geq$ 100 bpm & incr. $\geq$ 20 bpm versus B | 0/21 | 0/21 | 0/21 | 0/20 |
| PR $\geq$ 220 ms | 0/21 | 0/21 | 0/21 | 0/20 |
| QRS $\geq$ 120 ms | 0/21 | 0/21 | 0/21 | 0/20 |
| 431 $\leq$ QTc $\leq$ 450 (Males) or 451 $\leq$ QTc $\leq$ 470 (Females) | 6/21 | 6/21 | 3/21 | 6/20 |
| QTc > 450 (Males) or QTc > 470 (Females) | 0/21 | 1/21 | 0/21 | 0/20 |
| QTc $\geq$ 500 | 0/21 | 0/21 | 0/21 | 0/20 |
| 30 $\leq$ delta QTc $\leq$ 60 ms | 0/21 | 0/21 | 0/21 | 1/20 |

(continued)

| Electrocardiogram (PCSA definition) | Subjects with at least one PCSA (On treatment)/ Evaluable subjects[a] | | | |
| | Strength 1 | | Strength 2 | |
| | R1 (separate) (N=21)[b] | T1 (mixed) (N=21)[b] | R2 (separate) (N=21)[b] | T2 (mixed) (N=20)[b] |
| Delta QTc > 60 ms | 0/21 | 0/21 | 0/21 | 0/20 |

PCSA = Potentially Clinically Significant Abnormality (Version of February 2009). [a] Count of subjects evaluable for a given parameter. [b] Total count of subjects exposed to study drugs (i.e. all subjects who took at least one dose of study drug). A PCSA is considered to be on-treatment if it occurred from the time of investigational product (IP) administration within the period up to 72 hours later. Strength 1 (R1 and T1) and Strength 2 (R2 and T2) are treatments per kg body weight of 0.4 U/kg insulin glargine combined with 0.264 μg/kg and 0.100 μg/kg lixisenatide respectively. R1 and R2 are separate simultaneous injections; T1 and T2 are injections of premixed formulation

*Table 41 - Number* of *Subjects with on-treatment Abnormalities (PCSA) in Vital Signs Parameters - Safety Population*

| Vital signs (PCSA definition) | Subjects with at least one PCSA (On treatment)/ Evaluable subjects [a] | | | |
| | Strength 1 | | Strength 2 | |
| | R1 (separate) (N=21)[b] | T1 (mixed) (N=21)[b] | R2 (separate) (N=21)[b] | T2 (mixed) (N=20)[b] |
| HR $\leq$ 40 bpm & deer. $\geq$ 20 bpm versus B | 0/21 | 0/21 | 0/21 | 0/20 |
| HR $\geq$ 100 bpm & incr. $\geq$ 20 bpm versus B | 0/21 | 0/21 | 0/21 | 0/20 |
| SBP $\leq$ 95 mmHg & decr. $\geq$ 20 mmHg versus B | 0/21 | 0/21 | 0/21 | 0/20 |
| SBP $\geq$ 140 mmHg & iner. $\geq$ 20 mmHg versus B | 2/21 | 2/21 | 1/21 | 1/20 |
| DBP $\leq$ 5 45 mmHg & decr. $\geq$ 10 mmHg versus B | 0/21 | 0/21 | 0/21 | 0/20 |
| DBP $\geq$ 90 mmHg & incr. $\geq$ 10 mmHg versus B | 1/21 | 0/21 | 0/21 | 0/20 |
| St - Su SBP $\leq$ -20 mmHg | 0/21 | 0/21 | 1/21 | 1/20 |
| St - Su DBP $\leq$ -10 mmHg | 0/21 | 0/21 | 0/21 | 0/20 |

PCSA = Potentially Clinically Significant Abnormality (Version of February 2009). [a] Count of subjects evaluable for a given parameter. [b] Total count of subjects exposed to study drugs (i.e. all subjects who took at least one dose of study drug). A PCSA is considered to be on-treatment if it occurred from the time of investigational product (IP) administration within the period up to 72 hours later. Strength 1 (R1 and T1) and Strength 2 (R2 and T2) are treatments per kg body weight of 0.4 U/kg insulin glargine combined with 0.264 μg/kg and 0.100 μg/kg lixisenatide respectively.

*Table 42 - Shift Table for Results of Anti-Lixisenatide Antibody Testing - up to first Post-Study Visit*

| Baseline | Strength 1 (N=21)[a] | | | Strength 2 (N=21)[a] | | |
| | pos.[b] | neg.[b] | mis.[b] | pos.[b] | neg.[b] | mis.[b] |
| Positive | 0/0 | 0/0 | 0/0 | 0/0 | 0/0 | 0/0 |
| negative | 0/21 | 21/21 | 0/21 | 0/21 | 21/21 | 0/21 |

(continued)

| Baseline | Strength 1 (N=21)[a] | | | Strength 2 (N=21)[a] | | |
|---|---|---|---|---|---|---|
| | pos.[b] | neg.[b] | mis.[b] | pos.[b] | neg.[b] | mis.[b] |
| missing | 0/0 | 0/0 | 0/0 | 0/0 | 0/0 | 0/0 |

[a] Total count of subjects exposed to study drugs (i.e., all subjects who took at least one dose of study drug). [b] After baseline. pos. = at least one positive result; neg. = all results negative; mis. = missing data. Strength 1: single dose treatments per kg body weight of 0.4 U/kg insulin glargine and 0.264 $\mu$g/kg lixisenatide. Strength 2: single dose treatments per kg body weight of 0.4 U/kg insulin glargine and 0.100 $\mu$g/kg lixisenatide.

*Table 43 - Frequency Table for Global Irritation Score and Pain - Safety Population*

| | Strength 1 | | Strength 2 | |
|---|---|---|---|---|
| | R1 (separate) (N=21) n (%) | T1 (mixed) (N=21) n (%) | R2 (separate) (N=21) n (%) | T2 (mixed) (N=20) n (%) |
| Global irritation score [1] | | | | |
| No | 20 (95.2%) | 20 (95.2%) | 21 (100%) | 20 (100%) |
| Hardly | 1 (4.8%) | 0 | 0 | 0 |
| Mild | 0 | 1 (4.8%) | 0 | 0 |
| Spontaneous pain | | | | |

| | Strength 1 | | Strength 2 | |
|---|---|---|---|---|
| | R1 (separate) (N=21) n (%) | T1 (mixed) (N=21) n (%) | R2 (separate) (N=21) n (%) | T2 (mixed) (N=20) n (%) |
| No | 21 (100%) | 21 (100%) | 21 (100%) | 20 (100%) |

n (%) = number and % of subjects with the maximum score. Maximum score per subject and treatment is analysed as well as pain at any time per subject and treatment. The denominator is N, the number of subjects treated within each group. [1] No = No reaction; Hardly = Hardly perceptible erythema; Mild = slight erythema with or without slight edema; Moderate = moderate erythema, edema, with or without papules; Intense = marked erythema, edema, induration, with or without papules; Severe = intense erythema with edema, vesicles or blisters. Strength 1 (R1 and T1) and Strength 2 (R2 and T2) are treatments per kg body weight of 0.4 U/kg insulin glargine combined with 0.264 $\mu$g/kg and 0.100 $\mu$g/kg lixisenatide respectively. R1 and R2 are separate simultaneous injections; T1 and T2 are injections of premixed formulation.

Additional Statistical Analysis of Pharmacokinetic Data - Analysis within Strength 2 - Subject 276001522 excluded

Subject 276001522 (strength 2) did not show sustained euglycaemia in both treatment periods despite confirmed exposure to both lixisenatide and insulin glargine. Conclusive results were not altered regardless of inclusion or exclusion of this subject.

*Table 44 - Lixisenatide (AVE0010) - Parametric Analyses - Treatment Ratios (T2/R2) - Estimates of Treatment Ratio with 90% Confidence Interval*

| Parameter | Comparison | Estimate | 90% CI |
|---|---|---|---|
| $C_{max}$ ($\mu$g/ml) | Subject 1522 excluded: T2 (mixed) / R2 (separate) | 0.76 | (0.67 to 0.86) |
| $AUC_{last}$ (pg.hr/ml) | Subject 1522 excluded: T2 (mixed) / R2 (separate) | 0.91 | (0.76 to 1.10) |

(continued)

| Parameter | Comparison | Estimate | 90% CI |
|---|---|---|---|
| AUC (pg.hr/ml) | Subject 1522 excluded: T2 (mixed) / R2 (separate) | 0.95 | (0.79 to 1.15) |

R2 and T2 (Strength 2) are treatments per kg body weight of 0.4 U/kg insulin glargine and 0.100 $\mu$g/kg lixisenatide. R2 = separate simultaneous injections; T2 = injection of premixed formulation. PK parameters are for lixisenatide (AVE0010).

*Table 45 - Insulin Glargine - Parametric Analyses - Treatment Ratios (T2/R2) - Estimates of Treatment Ratio with 90% Confidence Interval*

| Parameter | Comparison | Estimate | 90% CI |
|---|---|---|---|
| $AUC_{(0-24h)}$ ($\mu$U.hr/m)) | Subject 1522 excluded: T2 (mixed) / R2 (separate) | 0.88 | (0.78 to 0.98) |

R2 and T2 (Strength 2) are treatments per kg body weight of 0.4 U/kg insulin glargine and 0.100 $\mu$g/kg lixisenatide. R2 = separate simultaneous injections; T2 = injection of premixed formulation. PK parameters are for insuline glargine.

*Table 46 - Pharmacodynamics - Parametric Analyses - Treatment Ratios (T2/R2) - Estimates of Treatment Ratio with 90% Confidence Interval*

| Parameter | Comparison | Estimate | 90% CI |
|---|---|---|---|
| $GIR\text{-}AUC_{(0-24h)}$ (mg/kg) | Subject 1522 excluded: T2 (mixed) / R2 (separate) | 0.74 | (0.62 to 0.88) |
| $GIR_{max}$ (mg/kg/min) | Subject 1522 excluded: T2 (mixed) / R2 (separate) | 0.82 | (0.75 to 0.91) |

R2 and T2 (Strength 2) are treatments per kg body weight of 0.4 U/kg insulin glargine and 0.100 $\mu$g/kg lixisenatide. R2 = separate simultaneous injections; T2 = injection of premixed formulation. GIRmax and GIR-tmax are based on smoothed GIR profiles.

EXAMPLE 2

SYNOPSIS

Title of the study: A randomized, cross-over, open euglycemic clamp study on the relative bioavailability and activity of 0.4 U/kg insulin glargine and 20 $\mu$g AVE0010, given as on-site mixes in 2 strengths compared to separate simultaneous injections in subjects with diabetes mellitus type 1.

Phase of development: Exploratory

Primary objective

Assessment of relative bioavailability of insulin glargine and AVE0010 given separately but simultaneously versus given in 2 strengths of on-site mixes.

Secondary objectives

- Comparison of the activity of insulin glargine and AVE0010 given separately but simultaneously versus given in 2 strengths of on-site mixes as subcutaneous single dosing
- Safety and tolerability of insulin glargine - AVE0010 given in 2 strengths of in syringe mixes

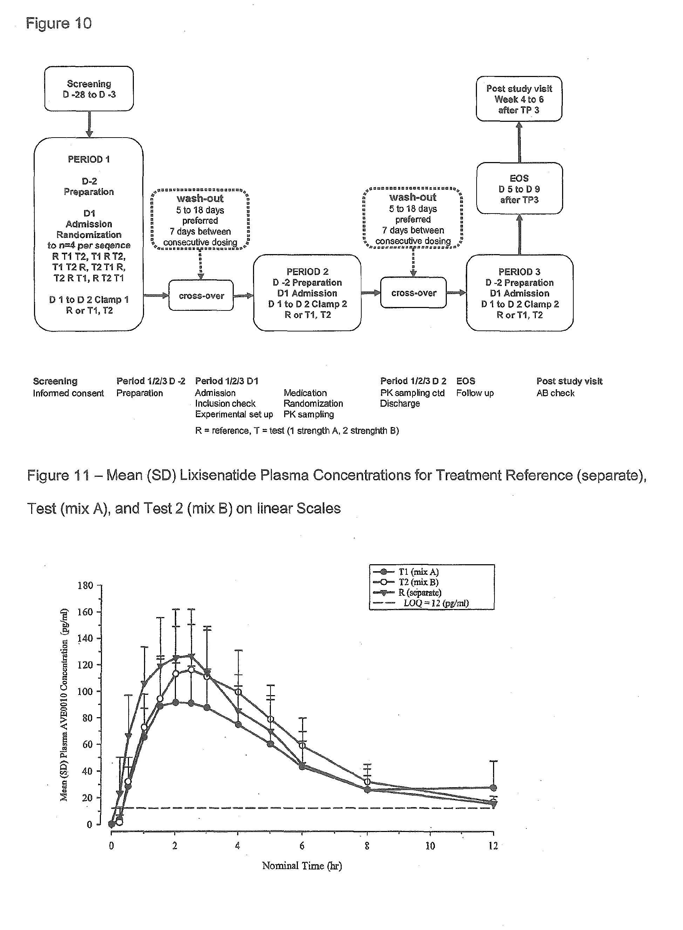

Methodology: Single-center, open, cross-over euglycaemic clamp study with 3 treatment periods and 6-sequences of 5-18 days, preferred 7-day wash-out period between treatment periods, randomized for sequences (4 subjects per sequence) R T1 T2, T1 R T2, T1 T2 R, T2 T1 R, T2 R T1, R T2 T1 (see Figure 10).

Test treatment T1 (Strength A), was given as an on-site mix of the current commercialized Lantus U100 formulation and a modified version of the current lixisenatide clinically developmental formulation (100 µg/mL, unbuffered) yielding an about U60 insulin glargine concentration.

Test treatment T2 (Strength B), was given as an on-site mix of a U300 insulin glargine formulation which was in clinical development and a modified version of the current lixisenatide clinically developmental formulation (100 µg/mL, unbuffered) yielding a U90 insulin glargine concentration.

Reference treatment (R) was given as separate simultaneous injections of Lantus U100 and current lixisenatide clinically developmental formulation (100 µg/mL, buffered) at opposite peri-umbilical sites.

Number of subjects

Planned: 24, Randomized: 26, Treated: 26

Evaluated

Pharmacokinetics: 26, Pharmacodynamics: 25, Safety: 26

Diagnosis and criteria for inclusion: Male and female subjects between 18 and 65 years of age with type 1 diabetes mellitus for more than one year; average total insulin dose of < 1.2 U/kg/day; body mass index between 18.0 and 30.0 kg/m$^2$ inclusive; fasting negative serum C-peptide (< 0.3 nmol/L); glycohemoglobin (HbA1c) ≤9%; stable insulin regimen for at least 2 months prior to study (with respect to the safety of the subject and scientific integrity of the study),

Key exclusion criteria

- Any history or presence of clinically relevant cardiovascular, pulmonary, gastrointestinal (such as pancreatitis), hepatic, renal, metabolic (apart from type 1 diabetes mellitus), hematological, neurological, psychiatric, systemic (affecting the body as a whole), ocular, gynecologic (if female), or infectious disease; any acute infectious disease or signs of acute illness

- More than one episode of severe hypoglycemia with seizure, coma or requiring assistance of another person during the past 6 months

Investigational product: Subcutaneous (SC) injection of 0.4 U/kg Lantus and 20 µg AVE0010 separate simultaneously at opposite peri-umbilical sites within 1 min, or injection as on-site mixes of Strength A or B at one peri-umbilical site.

• T1 (Test 1): injection of an on-site mix of Lantus U100 and AVE0010 (100 µg/mL, unbuffered) at one peri-umbilical site (Strength A)

• T2 (Test 2): injection of an on-site mix of insulin glargine (U300), AVE0010 (100 µg/mL, unbuffered) and placebo saline solution as needed at one peri-umbilical site (Strength B)

• Dose Investigational Product

| Compound | Dose | Form |
|---|---|---|
| AVE0010/Lantus Strength A | 20 µg 0.4 U/kg | 100 µg/mL unbuffered AVE0010, + 100 U/mL Lantus for injection |
| AVE0010/Lantus Strength B | 20 µg 0.4 U/kg | 100 µg/mL unbuffered AVE0010, + 300 U/mL Lantus for injection + solution* for injection |
| * pH 4.5 adjusted saline solution, for dilution to 90 U/mL | | |

• Administration: Subcutaneous injection

Reference Therapy

• R (Reference): separate simultaneous injections of Lantus U100 and AVE0010 (100 µg/mL) at opposite peri-umbilical sites

• Dose Reference Therapy

| Compound | Dose | Form |
|---|---|---|
| AVE0010 | 20 µg | 100 µg/mL for injection |
| Lantus U100 | 0.4 U/kg | 100 U/mL for injection |

• Administration: Subcutaneous injection

*Table 47- Composition of Reference and Investigational Products*

| Components [a] | Strength A [e] 0.1 / 3.6378 | Strength B [e] 0.1 / 3.6378 | Lantus U100 [f] 100 U/mL | Lixisenatide [f] 100 µg/mL | Function |
|---|---|---|---|---|---|
| | per mL [mg] | per mL [mg] | per mL [mg] | per mL [mg] | |
| AVE0010 | 0.044 - 0.033 | 0.070 - 0.045 | -- | 0.1 | Drug substance |
| Insulin glargine (U/mL) | 2.04 - 2.43 (57 - 66) | 3.27 (90) | 3.6378 | -- | Drug substance |
| Zinc chloride [b] | 0.0357 - 0.0413 | 0.0558 | 0.0626 | -- | Stabilizing agent |
| Glycerol 85% | 22.17 - 21.72 | 23.29 - 17.83 | 20.000 | 18.000 | Tonicity agent |
| Methionine | 1.304 - 1.034 | 1.421 - 2.077 | -- | 3.000 | Stabilizing agent |
| Metacresoic | 2.700 | 2.700 | 2.700 | 2.700 | Antimicrobial preservative |
| Sodium acetate | -- | -- | -- | 3.5 | Buffering agent |
| Sodium hydroxide | final pH 4.0<pH< 4.5 | final pH 4.0 < pH < 4.5 | q.s. pH 4.0 | q.s. pH 4.5 | Alkalizing agent |
| Hydrochloric acid, concentrated | | | q.s. pH 4.0 | q.s. pH 4.5 | Acidifying agent |
| Water for injection | -- | -- | Ad 1.0 mL | Ad 1.0 mL | Solvent |
| Placebo solution | | to yield 90 U/mL [d] | | | |

[a] Components are listed according to their pharmcopoeial names. If more than one monograph exists, other names are given in brackets, along with the compendial origin. [b] Composition gives total zinc chloride amount from insulin glargine and from the manufacturing of the drug products [c] For Metacresol, the common chemical name "m-cresol" is also used within this document. [d] Contains Metacresol but no Glycerol. [e] Strength A = T1; strength B = T2. [f] Reference = R

*Table 48 - Composition of Formulations used for on-site Preparations of Investigational Products*

| Components [a] | Lantus U100 100 U/mL | Lantus U300 300 U/mL | Lixisenatide [d] 100 µg/mL | Function |
|---|---|---|---|---|
| | per mL [mg] | per mL [mg] | per mL [mg] | |
| AVE0010 | -- | -- | 0.1 | Drug substance |
| Insulin glargine (U/mL) | 3.6378 | 10.9134 | -- | Drug substance |
| Zinc chloride [b] | 0.0626 | 0.1878 | -- | Stabilizing agent |
| Glycerol 85% | 20.000 | 20.000 | 25.000 | Tonicity agent |
| Methionine | -- | -- | 3.000 | Stabilizing agent |
| Metacresol [c] | 2.700 | 2.700 | 2.700 | Antimicrobial preservative |

(continued)

| Components [a] | Lantus U100 100 U/mL | Lantus U300 300 U/mL | Lixisenatide [d] 100 μg/mL | Function |
|---|---|---|---|---|
| | per mL [mg] | per mL [mg] | per mL [mg] | |
| Sodium acetate | -- | -- | -- | Buffering agent |
| Sodium hydroxide | q.s. pH 4.0 | q.s. pH 4.0 | q.s. pH 4.5 | Alkalizing agent |
| Hydrochloric acid, concentrated | q.s. pH 4.0 | q.s. pH 4.0 | q.s. pH 4.5 | Acidifying agent |
| Water for injection | Ad 1.0 mL | Ad 1.0 mL | Ad 1.0 m L | Solvent |

[a] Components are listed according to their pharmcopoeial names. If more than one monograph exists, other names are given in brackets, along with the compendial origin. [b] Composition gives total zinc chloride amount from insulin glargine and from the manufacturing of the drug product. [c] for Metacresol, the common chemical name "m-cresol" is also used within this document. [d] without buffer.

*Table 49 - AVE0090 Matching Placebo (needed for Dilution)*

| Components [a] | Percentage [%] | per mL [mg] | Per Unit (3 mL cartridge) [mg] | Function |
|---|---|---|---|---|
| Sodium chloride | 0.82 | 8.2 | 24.6 | Tonicity agent |
| Metacresol [b] | 0.27 | 2.7 | 8.1 | Antimicrobial preservative |
| Hydrochloric acid, concentrated | -- | q.s. pH 4.5 | q.s. pH 4.5 | Acidifying agent |
| Sodium hydroxide | -- | q.s. pH 4.5 | q.s. pH 4.5 | Alkalizing agent |
| Water for injection | q.s. 100 | q.s. 1 mL | q.s. 3 mL | Solvent |
| Nitrogen | Process aid forfiltration of the formulation | | | |

[a] Components are listed according to their pharmcopoeial names. If more than one monograph exists, other names are given in brackets, along with the compendial origin.
[b] for Metacresol, the common chemical name "m-cresol" is also used within this document.

Duration of treatment: Total study duration for one subject: about 3.5 months
Duration of each part of the study for one subject:

- Screening: 1 day during D-28 up to D-3
- Trial Period 1 (TP1): 2 days including one treatment day
- Washout: 5 - 18 days, preferred 7 days, between consecutive dosings
- TP2: 2 days including one treatment day
- Washout: 5 - 18 days, preferred 7 days ,between consecutive dosings
- TP3: 2 days including one treatment day
- End-of-study (EOS): 1 day between D5 and D9 after TP3
- Post-study visit / Anti AVE0010 anti-body check: 4 to 6 weeks after TP3

Duration of observation: The observation period started at the time the subject signed the informed consent and ended with the first scheduled post study visit.
Criteria for evaluation:

- Pharmacokinetics:

  - AVE0010: The area under the plasma AVE0010 concentration curve (AUC) as $AUC_{last}$ and AUC, apparent clearance (CL/F), apparent volume of distribution (Vz/F), and terminal half life $t_{1/2\lambda z}$ were derived, and peak concentration $C_{max}$, and time to $C_{max}$ ($T_{max}$) were observed.
  - Insulin glargine: The area under the plasma insulin glargine concentration curve (AUC) up to 24 h, $AUC_{0-24h}$ and the time to 50% of $AUC_{0-24h}$ were derived. In addition, $C_{max}$ and time to $C_{max}$ ($T_{max}$) were observed.

Pharmacokinetic sampling times and bioanalytical methods:

Pharmacokinetic sampling times

T0 defines time of injection of study medication.

Blood was collected for the determination of plasma AVE0010 concentrations at T0 and T0.25, T0.5, T1, T1.5, T2, T2.5, T3, T4, T5, T6, T8, T12 and T24 h after injection of study medication and for the determination of plasma insulin glargine concentrations at T0 and T0.25, T0.5, T1, T1.5, T2, T4, T6, T8, T10, T12, T14, T16, T18, T20, T22 and T24 h after injection of study medication.

Bioanalytical methods

| Analyte | AVE0010 | Insulin |
|---|---|---|
| Matrix | Plasma | Serum |
| Analytical Technique | Double-antibody sandwich ELISA | Radioimmunoassay |
| Lower Limit of Quantification | 12 pg/ml | 5.02 $\mu$U/mL 0.18 ng/mL or 30 pmol/L |
| Assay Range | 12- 220 pg/mL | 5.02-150 $\mu$U/mL |
| Assay Volume | 100 $\mu$L | 300 $\mu$L |
| Method Reference | VAA43648CH-IB-02 | VAL030/01 |

Methods used to determine pharmacokinetic variables

The following pharmacokinetic (PK) parameters were calculated, using non-compartmental methods for AVE0010 and insulin glargine plasma concentrations after single dose:

| Parameters | Drug/Analyte | Definition/Calculation |
|---|---|---|
| $C_{max}$ | AVE0010 / insulin glargine | Maximum plasma concentration observed |
| $t_{max}$ | AVE0010 / insulin glargine | First time to reach $C_{max}$ |
| $AUC_{last}$ | AVE0010 | Area under the plasma concentration versus time curve calculated using the trapezoidal method from time zero to the real time, $t_{last}$ (time corresponding to the last concentration above the limit of quantification, $C_{last}$) |
| $AUC_{0-24h}$ | Insulin glargine | Area under the plasma concentration versus time curve calculated using the trapezoidal method from time zero to 24 hours post dosing |
| AUC | AVE0010 | Area under the plasma concentration versus time curve extrapolated to infinity according to the following equation: $$AUC = AUC_{last} + \frac{C_{last}}{\hat{\lambda}_Z}$$ |
| CL/F | AVE0010 | Apparent Total Body Clearance of a drug from the plasma calculated using the following equation: $$CL/F = \frac{Dose_{EV}}{AUC_{EV}}$$ |

(continued)

| Parameters | Drug/Analyte | Definition/Calculation |
| --- | --- | --- |
| $V_z/F$ | AVE0010 | Aparent Volume of Distribution during the terminal ($\lambda_z$) phase calculated using the following equation:<br><br>$$V_z/F = \frac{CL/F}{\lambda_z}$$ |
| $t_{1/2Z}$ | AVE0010 | Terminal half-life associated with the terminal slope ($\lambda z$) determined according to the following equation:<br><br>$$t_{1/2Z} = \frac{0.693}{\lambda_z}$$<br><br>where $\lambda z$ is the slope of the regression line of the terminal phase of the plasma concentration versus time curve, in semi-logarithmic scale. Half-life is calculated by taking the regression of at least three points. |

- Pharmacodynamics: For pharmacodynamics (PD) of insulin glargine and lixisenatide given separately simultaneously and given as premixed formulations, the blood glucose concentration and glucose infusion rate was continuously recorded during the clamp procedure.
  PD parameters: The area under the body weight standardized glucose infusion rate (GIR) within 24 h (GIR-$AUC_{0-24h}$) and the time to 50% of the total GIR-AUC within 24 h (T50%-GIR-$AUC_{0-24h}$) was calculated. In addition, the maximum of the smoothed GIR ($GIR_{max}$) and the time to $GIR_{max}$, GIR-$T_{max}$, was assessed.
- Safety: Adverse events (AEs) reported by the subject or noted by the investigator; standard hematology and blood chemistry; urinalysis; vital signs and ECG; anti-AVE0010 antibodies; injections site tolerability.

Statistical methods:

Pharmacokinetics

Pharmacokinetic parameters are summarized by treatment using descriptive statistics. Statistical analyses are comparing both test treatments with the reference (R). Supplemental analyses compare the two test treatments.
AVE0010: For log transformed AUC, $AUC_{last}$ and $C_{max}$ the relative bioavailabilities between test treatment (Strengths A and B) and reference treatment (separate application) were assessed using a linear mixed effects model. Estimate and 90% confidence interval (CI) for the ratio of geometric means between test and reference treatment were provided for AUC, $AUC_{last}$ and Cmax.
Insulin glargine: For log transformed $AUC_{0-24h}$ the relative bioavailability between test and reference treatment were assessed using a linear mixed effects model. Estimate and 90% CI for the ratio of geometric means between test and reference treatment were provided for $AUC_{0-24h}$. Times to 50% of $AUC_{0-24h}$ (T50%-$AUC_{0-24h}$) were compared non-parametrically between test and reference treatment.

Pharmacodynamics

Statistical analyses compare both test treatments with the reference (R). Supplemental analyses compare the 2 test treatments.
For log transformed GIR-$AUC_{0-24h}$, the ratios between test (Strengths A and B) and reference treatment (versus separate application) were assessed using a linear mixed effects model. Estimate and 90% CI for the ratio of geometric means between the 2 treatments were provided for GIR-$AUC_{0-24h}$- $GIR_{max}$ was subject to corresponding analysis albeit a supplemental parameter.
Times to 50% of GIR-$AUC_{0-24h}$ were compared non-parametrically between each test and reference treatment. GIR-$t_{max}$ was subject to corresponding analysis albeit a supplemental parameter.

Safety and tolerability

The safety analysis is based on the review of the individual values (clinically significant abnormalities) and descriptive statistics by treatment.

For AEs, frequencies of treatment emergent adverse events (TEAEs) classified by MedDRA system organ class and preferred term is tabulated by treatment.

For vital signs and ECG, frequency of subjects with abnormalities and potentially clinically significant abnormalities (PCSAs) were summarized by treatment.

Summary

The primary objective of the study was to assess the relative bioavailabilities of lixisenatide and insulin glargine and the secondary objective was to compare activities between test and reference treatments (T1 versus R, T2 versus R). In order to position findings equivalence criteria were employed. The conclusion of equivalence in the formal sense requires the CI of the ratio test/reference to be within the limits of 0.80 and 1.25. For the following, the term "comparable" is used when the acceptance criteria are only marginally violated.

Lixisenatide exposure, $AUC_{last}$, total AUC and maximum concentration $C_{max}$, were equivalent with T2 and R. Exposure was not equivalent for T1 and R. T1 produced lower lixisenatide exposure as compared to R.

Insulin glargine exposure, $AUC_{0-24h}$, was equivalent with T2 and R and not equivalent with T1 and R. Insulin glargine exposure was 26 % higher after T1 as compared to R. Time to 50% of $AUC_{0-24h}$ was about 1 h shorter with T1 as compared to R and T2.

The glucodynamic effect, $GIR\text{-}AUC_{0-24h}$, was comparable between T2 and R and not equivalent between T1 and R. The effect was 72 % higher after T1, as compared to R while the times to 50% of $GIR\text{-}AUC_{0-24h}$ did not indicate differences between T1 and R, and T2 and R.

*Table 50 - Overview - Results*

| Comparison vs R | T1 | T2 |
|---|---|---|
| Bioavailability of lixisenatide (AUC) | ↓ | = |
| Bioavailability of insulin glargine ($AUC_{0-24h}$) | ↑ | = |
| Comparative glucodynamics ($GIR\text{-}AUC_{0-24h}$) | ↑ | ≈ |

Pharmacokinetic results

Lixisenatide exposure, $AUC_{last}$, total AUC and maximum concentration $C_{max}$, was equivalent with T2 and R. The point estimates for the ratio T2/R for $AUC_{last}$ were 1.04 (0.95 to 1.14), for AUC 1.04 (0.95 to 1.14), and for $C_{max}$ 0.91 (0.82 to 1.02). Exposure was not equivalent for T1 and R. T1 produced lower lixisenatide exposure as compared to R.

Insulin glargine exposure, $AUC_{0-24h}$, was equivalent with T2 and R and not equivalent with T1 and R. The point estimate for the ratio T2/R for $AUC_{0-24h}$ was 1.02 (0.92 to 1.14). Insulin glargine exposure was 26 % higher after T1 as compared to R. Time to 50% of $AUC_{0-24h}$ was about 1 h shorter with T1 as compared to R and T2.

Pharmacodynamic results

The glucodynamic effect, $GIR\text{-}AUC_{0-24h}$, was comparable between T2 and R and not equivalent between T1 and R. The point estimate for the ratio T2/R was 0.97 (0.74 to 1.27). The effect was 72 % higher after T1, as compared to R while the times to 50% of $GIR\text{-}AUC_{0-24h}$ did not indicate differences between T1 and R, and T2 and R.

Safety results

Headache was the most common AE (1, 3 and 3 subjects on R, T1 and T2 respectively), known to be study specific (clamp procedure).

Drug specific AEs were gastrointestinal symptoms (3, 4 and 3 subjects on R, T1 and T2 respectively), common for lixisenatide.

All treatments, R, T1 and T2 were well tolerated and judged as safe for the study population.

Conclusions

Combined medication of insulin glargine and lixisenatide of Strength A (T1), given as an on-site mixed formulation of Lantus U100 and an unbuffered version of the clinically developmental lixisenatide formulation, yielding around U60 insulin glargine concentration, results in greater insulin glargine exposure and overall greater glucodynamic effects at lower lixisenatide exposure compared to insulin glargine and lixisenatide given separately simultaneously (R) from Lantus U100 and current clinically developmental lixisenatide formulation.

In contrast, combined medication of insulin glargine of Strength B (T2), given as an on-site mixed formulation from a developmental U300 insulin glargine and an unbuffered version of the clinically developmental lixisenatide formulation, yielding a final U90 insulin glargine concentration, achieves pharmacokinetic equivalence and comparable glucodynamic effect (point estimate T2/R 0.97) compared to Lantus and lixisenatide given separately but simultaneously (R).

Of note, all treatments, R, T1 and T2 were well tolerated and judged as safe for the study population.

1. STUDY SUBJECTS

1.1 SUBJECTS ACCOUNTABILITY

*Table 51 - Overview of Study Populations*

|  | All (N=26) |
|---|---|
| Safety population | 26 |
| Pharmacokinetic population | 26 |
| - lixisenatide | 26 |
| - insulin glargine | 26 |
| Pharmacodynamic population | 25 |

1.2 STUDY DISPOSITION

*Table 52 - Overview of Subject Disposition*

| Status | All |
|---|---|
| Included | 26 |
| Randomized | 26 |
| Exposed | 26 |
| Treated Period 1 | 26 |
| Treated Period 2 | 25 |
| Treated Period 3 | 24 |
| Discontinued treatment | 2 |
| Completed study treatment period | 24 |
| Reason for treatment/study discontinuation | |
| Adverse event | 2 |

1.3 DEMOGRAPHIC AND BASELINE CHARACTERISTICS

*Table 53 - Summary of Demographic Data - Safety Population*

|  | All (N=26) |
|---|---|
| Sex [n (%)] | |
| Male | 20 (76.9%) |
| Female | 6 (23.1%) |
| Race [n (%)] | |

(continued)

|  | All (N=26) |
|---|---|
| Caucasian / white | 26 (100%) |
| Age (yrs) | |
| Number | 26 |
| Mean (SD) | 38.1 (11.1) |
| Median | 39.0 |
| Min : Max | 19:57 |
| Height (cm) | |
| Number | 26 |
| Mean (SD) | 177.2 (8.4) |
| Median | 175.5 |
| Min : Max | 164: 197 |
| Weight (kg) | |
| Number | 26 |
| Mean (SD) | 78.75 (9.20) |
| Median | 75.45 |
| Min : Max | 66.2 : 94.8 |
| BMI (kg/m2) | |
| Number | 26 |
| Mean (SD) | 25.04 (1.95) |
| Median | 25.00 |
| Min : Max | 21.7: 29.9 |

Note: Number corresponds to the count of subjects with non missing data used for the calculation of percentages.

### 1.4 DOSAGE AND DURATION

*Table 54 - Overview of Treatment Exposure - Safety Population*

| Treatment duration (in days) | R (separate) (N=25) n (%) | T1 (mix A) (N=24) n (%) | T2 (mix B) (N=26) n (%) |
|---|---|---|---|
| 1 | 25 (100%) | 24 (100%) | 26 (100%) |

n(%)=number and percentage of subject having the corresponding treatment duration. The denominator is N, the number of subjects treated within each group. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

### 2 PHARMACOKINETIC EVALUATION

### 2.1 PLASMA CONCENTRATIONS

### 2.2.1 Lixisenatide

Mean (SD) plasma lixisenatide concentration-time profiles for each treatment are shown in the Figure 11.

2.2.2 Insulin Glargine

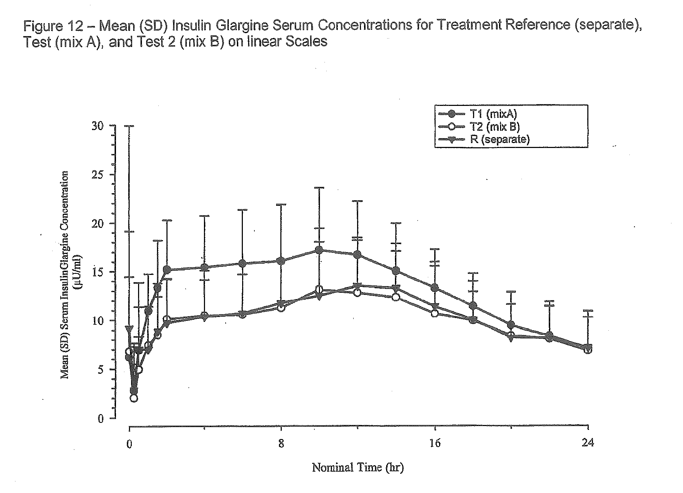

Mean (SD) serum insulin glargine concentration-time profiles for each treatment are shown in the Figure 12. For mean concentration calculation < LLOQ values were taken as zero.

2.2 PHARMACOKINETIC PARAMETERS

2.2.1 Lixisenatide

Pharmacokinetic parameters for lixisenatide for each treatment are summarized in the table below.

*Table 55 - Lixisenatide Pharmacokinetic Parameters*

| Mean ± SD (Geometric Mean) | Lixisenatide | | |
|---|---|---|---|
| [CV%] | 20 μg Lixisenatide R (separate) | 20 μg Lixisenatide T1 (mix A) | 20 μg Lixisenatide T2 (mix B) |
| N | 25 | 24 | 26 |
| $C_{max}$ (pg/ml) | 137 ± 33.5 (133) [24.3] | 104 ± 39.8 (97.6) [38.1] | 126 ± 34.2 (122) [27.1] |
| $t_{max}{}^a$ (hr) | 2.00 (0.50 - 3.00) | 2.00 (1.50 - 5.00) | 2.50 (1.50 - 4.00) |
| $t_{lag}{}^a$ (hr) | 0.00 (0.00 - 0.25) | 0.25 (0.00 - 0.50) | 0.25 (0.00 - 0.50) |
| $t_{1/2z}$ (hr) | 2.17 ± 0.41 (2.13) [18.9] | 2.36 ± 0.78 (2.27) [32.9] | 2.33 ± 0.47 (2.29) [20.4] |
| $AUC_{last}$ (pg•hr/ml) | 609 ± 172 (585) [28.3] | 486 ± 190 (453) [39.2] | 628 ± 180 (604) [28.6] |
| AUC (pg•hr/ml) | 685 ± 190 (659) [27.8] | 570 ± 250 (527) [43.8] | 708 ± 192 (683) [27.1] |
| a Median (Min - Max) | | | |

2.2.2 Insulin Glargine

Pharmacokinetic parameters for insulin glargine for each treatment are summarized in the table below. According to data handling conventions and justifications in the statistical analysis plan (SAP) the concentration of insulin glargine was to be set to zero at t=0 in all samples. Thereafter, < LLOQ concentration results were set to LLOQ/2, that is 2.5 μU/mL for calculation of $AUC_{0-24}$ and other PK parameters. For calculation of mean concentration values including $C_{max}$ < LLOQ concentration results were set to zero.

*Table 56 - Insulin Glargine Pharmacokinetic Parameters*

| Mean ± SD (Geometric Mean) | Serum Insulin glargine | | |
|---|---|---|---|
| [CV%] | 0.4 U/kg Insulin glargine R (separate) | 0.4 U/kg Insulin glargine T1 (mix A) | 0.4 U/kg Insulin glargine T2 (mix B) |
| N | 23[a] | 24 | 22[b] |
| $C_{max}$ (μU/ml) | 15.0 ± 5.76 (14.0) [38.5] | 18.6 ± 5.59 (17.7) [30.1] | 14.5 ± 5.32 (13.2) [36.6] |
| $t_{max}{}^c$ (hr) | 12.00 (0.25 - 15.00) | 10.00 (2.00 - 18.00) | 11.06 (0.25 - 24.00) |
| $AUC_{0-24}$ | 253 ± 78.4 | 320 ± 95.4 | 270 ± 63.6 |

(continued)

| Mean $\pm$ SD (Geometric Mean) | Serum Insulin glargine | | |
|---|---|---|---|
| [CV%] | 0.4 U/kg Insulin glargine R (separate) | 0.4 U/kg Insulin glargine T1 (mix A) | 0.4 U/kg Insulin glargine T2 (mix B) |
| (pU•hr/ml) | (241) [31] | (305) [30] | (261) [24][d] |

[a] Profile of Subject 276001006 and 276001019 was excluded. [b] Profile of Subject 276001003, 276001005, 276001006, 276001011 was excluded. [c] Median (Min - Max). [d] n=20, Subject 276001007 and 276001014 not included in calculation of summary statistics.

*Table 57 - $T_{50\%}$-$AUC_{(0-24h)}$ for Insulin Glargine - Descriptive Statistics*

| | R (separate) | T1 (mix A) | T2 (mix B) |
|---|---|---|---|
| $T_{50\%}$-$AUC_{(0-24h)}$ (h) | | | |
| Number | 23 | 24 | 20 |
| Mean (SD) | 11.860 (0.962) | 10.888 (0.693) | 11.655 (1.405) |
| Median | 12.100 | 11.050 | 11.835 |
| Min: Max | 9.70 : 13.29 | 9.29 : 11.84 | 7.95 : 14.15 |

AUC = Area under the insulin glargine concentration versus time curve. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

2.3 RESULTS OF STATISTICAL ANALYSIS OF PHARMACOKINETIC DATA

2.3.1 Lixisenatide (AVE0010)

*Table 58 - Parametric Analyses - Treatment Ratios (T1/R, T2/R and T2/T1) - Estimates of Treatment Ratio with 90% Confidence Interval*

| Parameter | Comparison | Estimate | 90% CI |
|---|---|---|---|
| $C_{max}$ (pg/ml) | T1 (mix A) / R (separate) | 0.74 | (0.64 to 0.84) |
| | T2 (mix B) / R (separate) | 0.91 | (0.82 to 1.02) |
| | T2 (mix B) / T1 (mix A) | 1.24 | (1.11 to 1.40) |
| $AUC_{last}$ (pg.hr/ml) | T1 (mix A) / R (separate) | 0.78 | (0.69 to 0.88) |
| | T2 (mix B) / R (separate) | 1.04 | (0.95 to 1.14) |
| | T2 (mix B) / T1 (mix A) | 1.33 | (1.18 to 1.49) |
| AUC (pg.hr/ml) | T1 (mix A) / R (separate) | 0.80 | (0.71 to 0.91) |
| | T2 (mix B) / R (separate) | 1.04 | (0.95 to 1.14) |
| | T2 (mix B) / T1 (mix A) | 1.30 | (1.15 to 1.47) |

Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide. PK parameters are for lixisenatide (AVE0010).

2.3.2 Insulin Glargine

*Table 59 - Parametric Analyses - Treatment Ratios (T1/R, T2/R and T2/T1) - Estimates of Treatment Ratio with 90% Confidence Interval*

| Parameter | Comparison | Estimate | 90% CI |
|---|---|---|---|
| $AUC_{(0-24h)}$ ($\mu$U.hr/ml) | T1 (mix A) / R (separate) | 1.26 | (1.14 to 1.40) |
| | T2 (mix B) / R (separate) | 1.02 | (0.92 to 1.14) |
| | T2 (mix B) / T1 (mix A) | 0.81 | (0.71 to 0.92) |

Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide. PK parameters are for insulin glargine.

*Table 60 - Nonparametric Analyses - Treatment Differences (T1-R, T2-R and T2-T1) - $T_{50\%}$-$AUC_{(0-24h)}$ [hours] - Estimate of Treatment Differences*

| Exact Hodges-Lehmann | | |
|---|---|---|
| Comparison | Point estimate | 90% confidence interval |
| T1 vs. R | -0.9 | (-1.2;-0.5) |
| T2 vs. R | -0.2 | (-0.7; 0.3) |
| T2 vs. T1 | 0.9 | (0.4; 1.3) |

Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide. PK parameters are for insulin glargine.

3. PHARMACODYNAMIC EVALUATION

3.1 MAIN ANALYSIS - DESCRIPTIVE STATISTICS AND PLOTS

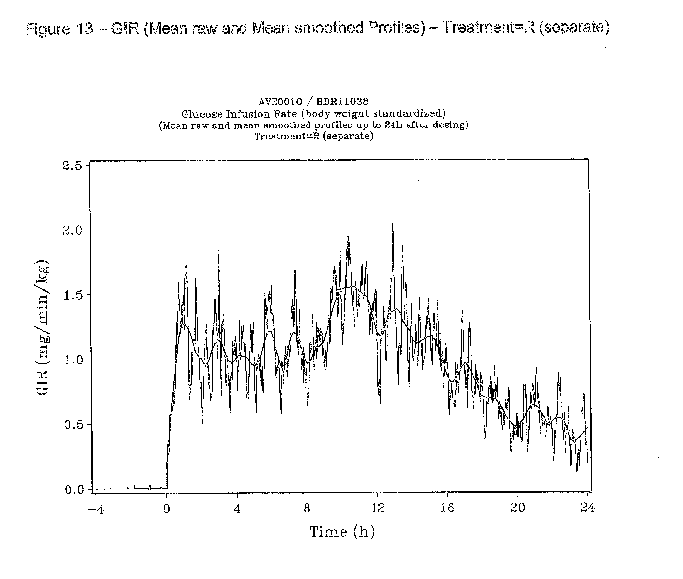

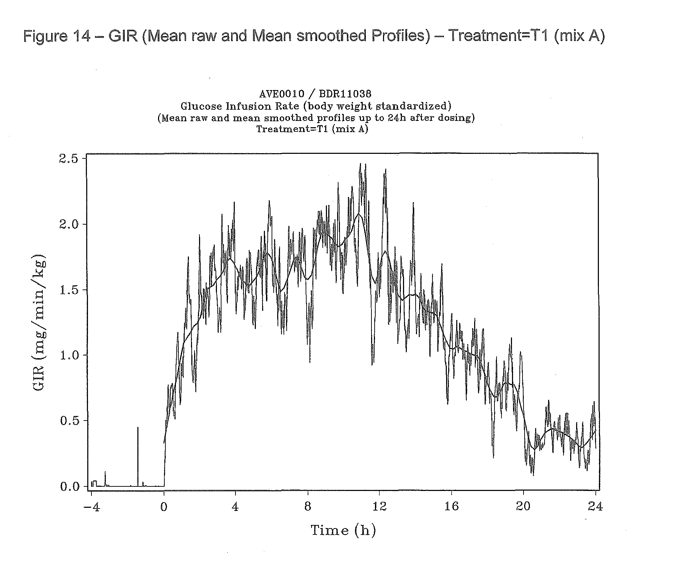

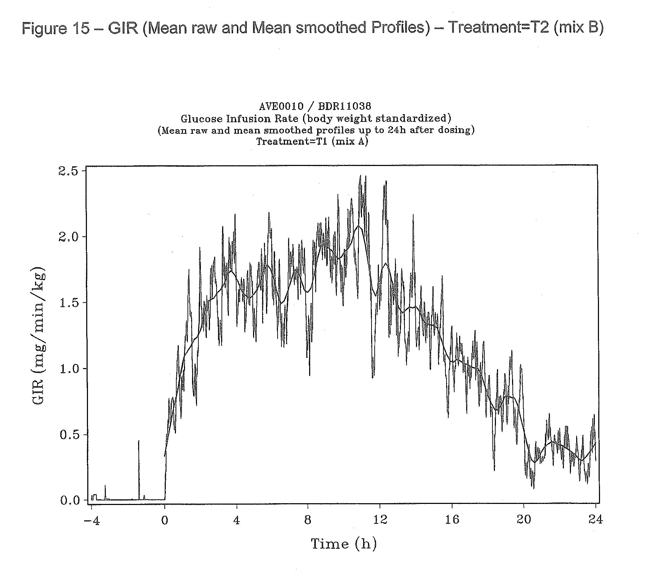

The time course of glucose infusion rate is plotted in Figures 13-15

*Table 61 - Descriptive Statistics of GIR-$AUC_{(0-24h)}$ (mg/kg)*

| | R (separate) | T1 (mix A) | T2 (mix B) |
|---|---|---|---|
| GIR-$AUC_{(0-24h)}$ (mg/kg) | | | |
| Number | 24 | 24 | 23 |
| Geometric Mean | 996.73 | 1484.32 | 1019.57 |
| CV% | 65.975 | 57.184 | 68.507 |
| Mean (SD) | 1405.65 (927.38) | 1766.68 (1010.26) | 1465.37 (1003.88) |
| Median | 1358.85 | 1537.50 | 1408.00 |
| Min : Max | 81.4 : 3332.5 | 234.6: 4801.6 | 12.9 : 3767.6 |

GIR = body weight standardized glucose infusion rate. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

*Table 62 - Descriptive Statistics of Time (h) to 50% of GIR-$AUC_{(0-24h)}$*

| | R (separate) | T1 (mix A) | T2 (mix B) |
|---|---|---|---|
| $T_{50\%}$-GIR-$AUC_{(0-24)}$ (h) | | | |
| Number | 24 | 24 | 23 |
| Mean (SD) | 9.412 (2.968) | 9.661 (1.987) | 10.566 (3.016) |
| Median | 10.235 | 9.850 | 10.780 |

(continued)

|  | R (separate) | T1 (mix A) | T2 (mix B) |
|---|---|---|---|
| Min : Max | 1.77 : 12.60 | 4.02 : 14.75 | 4.70: 18.47 |

GIR = body weight standardized glucose infusion rate. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

*Table 63 - Descriptive Statistics of $GIR_{max}$ (mg/kg/min)*

|  | R (separate) | T1 (mix A) | T2 (mix B) |
|---|---|---|---|
| $GIR_{max}$ (mg/kg/min) |  |  |  |
| Number | 24 | 24 | 23 |
| Geometric Mean | 2.331 | 2.827 | 2.337 |
| CV% | 44.6792 | 43.5224 | 49.1224 |
| Mean (SD) | 2.588(1.156) | 3.070(1.336) | 2.706(1.329) |
| Median | 2.415 | 2.930 | 2.560 |
| Min: Max | 0.68: 5.10 | 1.07: 7.73 | 0.24: 5.84 |

GIR = body weight standardized glucose infusion rate. GIRmax and GIR-tmax are based on smoothed GIR profiles. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

*Table 64 - Descriptive Statistics of Time to $GIR_{max}$ (h)*

|  | R (separate) | T1 (mix A) | T2 (mix B) |
|---|---|---|---|
| GIR-$t_{max}$ (h) |  |  |  |
| Number | 24 | 24 | 23 |
| Mean (SD) | 7.372 (5.427) | 9.654 (5.172) | 10.938 (7.130) |
| Median | 6.940 | 10.130 | 11.820 |
| Min : Max | 0.75 : 24.00 | 0.75 : 22.13 | 0.00: 24.00 |

GIR = body weight standardized glucose infusion rate. $GIR_{max}$ and GIR-tmax are based on smoothed GIR profiles. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

3.2 MAIN ANALYSIS - COMPARATIVE ANALYSES

*Table 65- Parametric Analyses - Treatment ratios (T1/R, T2/R and T2/T1) - Estimates of Treatment Ratio with 90% Confidence Interval\**

| Parameter | Comparison | Estimate | 90% CI |
|---|---|---|---|
| GIR-AUC$_{(0-24h)}$ (mg/kg) | T1 (mix A) / R (separate) | 1.72 | (1.33 to 2.22) |
|  | T2 (mix B) / R (separate) | 0.97 | (0.74 to 1.27) |
|  | T2 (mix B) / T1 (mix A) | 0.56 | (0.42 to 0.76) |
| $GIR_{max}$ (mg/kg/min) | T1 (mix A) / R (separate) | 1.28 | (1.15 to 1.43) |
|  | T2 (mix B) / R (separate) | 0.98 | (0.83 to 1.17) |
|  | T2 (mix B) / T1 (mix A) | 0.77 | (0.67 to 0.89) |

(continued)

| Parameter | Comparison | Estimate | 90% CI |
|---|---|---|---|
| Parameter | Comparison | Estimate | 90% CI |

Treatments are dosings of 20 $\mu$g Lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide. $GIR_{max}$ and GIR-tmax are based on smoothed GIR profiles. *$GIR_{max}$ and GIR-$T_{max}$ are secondary endpoints

*Table 66 - Nonparametric Analyses - Treatment Differences (T1-R, T2-R and T2-T1) - $T_{50\%}$-GIR-$AUC_{(024h)}$ [hours] - Estimate of Treatment Differences*

| Exact Hodges-Lehmann | | |
|---|---|---|
| Comparison | Point estimate | 90% confidence interval |
| T1 vs. R | -0.5 | (-1.2 ; 0.9) |
| T2 vs. R | 0.2 | (-0.3; 2.0) |
| T2 vs. T1 | 1.0 | (-0.1 ; 1.8) |

Treatments are dosings of 20 $\mu$g lixisenatide and per Kg body weight of 0.4 U/Kg insulin glargine. R denotes separate injections of insulin glargine (U100) and lixisenatide. T1 (mix A) and T2 (mix B) denote on-site mixes of insulin glargine (U100 and U300 respectively) with lixisenatide.

*Table 67 - Nonparametric Analyses - Treatment Differences (T1-R, T2-R and T2-T1) - GIR-$t_{max}$ [hours] - Estimate of Treatment Differences\**

| Exact Hodges-Lehmann | | |
|---|---|---|
| Comparison | Point estimate | 90% confidence interval |
| T1 vs. R | 2.2 | (-0.7; 4.8) |
| T2 vs. R | 3.3 | (0.1 ; 7.2) |
| T2 vs. T1 | 0.8 | (-1.9 ; 4.3) |

Treatments are dosings of 20 $\mu$g Lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide. GIRmax and GIR-tmax are based on smoothed GIR profiles. * $GIR_{max}$ and GIR-$T_{max}$ are secondary endpoints

## 4. SAFETY EVALUATION

### 4.1 TREATMENT EMERGENT ADVERSE EVENTS

A total of 26 adverse events (AEs) occurred in 12 subjects..

The majority of AEs were related to the gastrointestinal system (15 cases in 8 subjects) with mild to moderate nausea being the most frequent event (9 cases in 6 subjects). Onset of nausea was between approximately 1 h and 6 h after study drug administration. In all cases, symptoms completely abated within 7 h without any specific treatment. Incidence of nausea did not apparently differ between treatments.

Three cases of vomiting were reported for 2 subjects. Other gastrointestinal events were single cases of abdominal discomfort, heartburn or diarrhea.

Eight cases of mild or moderate headache occurred in 4 subjects which is a common finding in clamp studies regardless of the medication used.

According to (evaluation by treatment) headache was the most common AE (1, 3 and 3 subjects on R, T1 and T2 respectively), known to be study specific (clamp procedure). Drug specific AEs were gastrointestinal symptoms (3, 4 and 3 subjects on R, T1 and T2 respectively), common to lixisenatide.

All treatments, R, T1 and T2 were well tolerated and judged as safe for the study population.

*Table 68 - Overview of Treatment-Emergent Adverse Events (TEAEs) - Safety Population*

| | R (separate) (N=25) n (%) | T1 (mix A) (N=24) n (%) | T2 (mix B) (N=26) n (%) |
|---|---|---|---|
| AnyTEAE | 4 (16.0%) | 8 (33.3%) | 5 (19.2%) |
| Any severe TEAE | 0 | 0 | 0 |
| Any serious TEAE | 0 | 0 | 0 |
| Any TEAE leading to permanent treatment discontinuation | 0 | 0 | 2 (7.7%) |

TEAE = Treatment Emergent Adverse Event. N = Number of subjects exposed, n(%) = Number and % of subjects with at least one TEAE. An adverse event is considered as treatment emergent if it occurred from the time of investigational product (IP) administration within the period up to 72 hours later. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

*Table 69 - Number (%) of Subjects with TEAE by System Organ Class and Preferred Term - Safety Population*

| Primary System Organ Class Preferred Term | R (separate) (N=25) n (%) | T1 (mix A) (N=24) n (%) | T2 (mix B) (N=26) n (%) |
|---|---|---|---|
| Any class | 4 (16.0%) | 8 (33.3%) | 5 (19.2%) |
| Nervous system disorders | 1 (4.0%) | 3 (12.5%) | 3 (11.5%) |
| Headache | 1 (4.0%) | 3 (12.5%) | 3 (11.5%) |
| Gastrointestinal disorders | 3 (12.0%) | 4 (16.7%) | 3 (11.5%) |
| Nausea | 2 (8.0%) | 3 (12.5%) | 2 (7.7%) |
| Vomiting | 0 | 2 (8.3%) | 1 (3.8%) |
| Dyspepsia | 0 | 0 | 1 (3.8%) |
| Abdominal discomfort | 1 (4.0%) | 0 | 0 |
| Diarrhoea | 1 (4.0%) | 0 | 0 |
| General disorders and administration site conditions | 0 | 1 (4.2%) | 1 (3.8%) |
| Injection site phlebitis | 0 | 0 | 1 (3.8%) |
| Injection site erythema | 0 | 1 (4.2%) | 0 |

TEAE = Treatment Emergent Adverse Event. N = Number of subjects exposed, n(%) = Number and % of subjects with at least one TEAE. An adverse event is considered as treatment emergent if it occurred from the time of investigational product (IP) administration within the period up to 72 hours later. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide. MedDRA version: 12.0

4.2 SERIOUS ADVERSE EVENTS

There were no serious TEAEs.

4.3 DISCONTINUATION DUE TO ADVERSE EVENTS

Subject 276001011 discontinued due to nausea and headache during period 1. In Subject 276001012, a phlebitis of the left lower arm (v. cephalica) occurred after the second trial period which required antibiotic treatment and prompted exclusion by Investigator's decision. For both subjects, one additional subject each was enrolled.

4.4 LOCAL TOLERABILITY AT INJECTION SITE

Local reactions at the injection site were observed in 4 out of 75 dosing occasions with in total 100 injections. During TP2 of subject 276001003, a moderate erythema with a diameter of 4 cm occurred at the injection site 15 mins after administration of investigational product strength A (T1) (Global irritation score 2). This finding had completely resolved by 160 mins post-dose. This case was reported as an AE.

In trial period 1 (for subjects 276001007, 276001008 and 276001009), a barely perceptible erythema (global irritation score of 0.5) was found 15 mins after study drug administration which had completely resolved by 1 h post-dose. These findings were not considered to be of any clinical relevance and not to be an AE according to Clinical Study Protocol definitions.

*Table 70 - Frequency Table for Global Irritation Score and Pain - Safety Population*

| | R (separate) (N=25) n (%) | T1 (mix A) (N=24) n (%) | T2 (mix B) (N=26) n (%) |
|---|---|---|---|
| Global irritation score [1] | | | |
| No | 24 (96.0%) | 22 (91.7%) | 25 (96.2%) |
| Hardly | 1 (4.0%) | 1 (4.2%) | 1 (3.8%) |
| Moderate | 0 | 1 (4.2%) | 0 |
| Spontaneous pain | | | |
| No | 25 (100%) | 24 (100%) | 26 (100%) |

n (%) = number and % of subjects with the maximum score. Maximum score per subject and treatment is analysed as well as pain at any time per subject and treatment. The denominator is N, the number of subjects treated within each group. [1] No = No reaction; Hardly = Hardly perceptible erythema; Mild = slight erythema with or without slight edema; Moderate = moderate erythema, edema, with or without papules; Intense = marked erythema, edema, induration, with or without papules; Severe = intense erythema with edema, vesicles or blisters. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

4.5 OTHER SAFETY PARAMETERS

*Table 71 - Number of Subjects with on-treatment Abnormalities (PCSA) in ECG Parameters - Safety Population*

| | Subjects with at least one PCSA (On treatment)/ Evaluable subjects [a] | | |
|---|---|---|---|
| Electrocardiogram (PCSA definition) | R (separate) (N=25) [b] | T1 (mix A) (N=24) [b] | T2 (mix B) (N=26) [b] |
| HR ≤40 bpm & decr. ≥20 bpm versus B | 0/25 | 0/24 | 0/26 |
| HR ≥100 bpm & incr. ≥20 bpm versus B | 1/25 | 0/24 | 0/26 |
| PR ≥220 ms | 0/25 | 2/24 | 1/26 |
| QRS ≥120 ms | 0/25 | 0/24 | 0/26 |
| 431 ≤QTc ≤450 (Males) or 451 ≤QTc ≤4.70 (Females) | 14/25 | 10/24 | 9/26 |
| QTc > 450 (Males) or QTc > 470 (Females) | 2/25 | 5/24 | 4/26 |
| QTc ≥500 | 0/25 | 0/24 | 0/26 |
| 30 ≤delta QTc ≤60 ms | 2/25 | 4/24 | 3/26 |
| Delta QTc > 60 ms | 0/25 | 1/24 | 0/26 |

PCSA = Potentially Clinically Significant Abnormality. [a] Count of subjects evaluable for a given parameter. [b] Total count of subjects exposed to study drugs (i.e. all subjects who took at least one dose of study drug). A PCSA is considered to be on-treatment if it occurred from the time of investigational product (IP) administration within the period up to 72 hours later. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) *with* lixisenatide.

The large numbers of subject with QTc prolongation with either treatment can be explained by the duration of the euglycaemic clamp where subjects are recumbent without physical activity. This is not uncommon for prolonged clamps.

*Table 72 - Number of Subjects with on-treatment Abnormalities (PCSA) in Vital Signs Parameters - Safety Population*

| Vital signs (PCSA definition) | Subjects with at least one PCSA (On treatment)/ Evaluable subjects [a] | | |
|---|---|---|---|
| | R (separate) (N=25) [b] | T1 (mix A) (N=24) [b] | T2 (mix B) (N=26) [b] |
| HR $\leq$ 40 bpm & decr. $\geq$ 20 bpm versus B | 0/25 | 0/24 | 0/26 |
| HR $\geq$ 100 bpm & incr. $\geq$ 20 bpm versus B | 0/25 | 0/24 | 0/26 |
| SBP $\leq$ 95 mmHg & decr. $\geq$ 20 mmHg versus B | 0/25 | 0/24 | 0/26 |
| SBP $\geq$ 140 mmHg & incr. $\geq$ 20 mmHg versus B | 2/25 | 2/24 | 1/26 |
| DBP $\leq$ 45 mmHg & decr. $\geq$ 10 mmHg versus B | 0/25 | 0/24 | 0/26 |
| DBP $\geq$ 90 mmHg & incr. $\geq$ 10 mmHg versus B | 1/25 | 1/24 | 0/26 |
| St - Su SBP $\leq$ -20 mmHg | 0/25 | 0/24 | 1/26 |
| St - Su DBP $\leq$ -10 mmHg | 0/25 | 0/24 | 1/26 |

PCSA = Potentially Clinically Significant Abnormality. [a] Count of subjects evaluable for a given parameter. [b] Total count of subjects exposed to study drugs (i.e. all subjects who took at least one dose of study drug). A PCSA is considered to be on-treatment if it occurred from the time of investigational product (IP) administration within the period up to 72 hours later. Treatments are dosings of 20 $\mu$g lixisenatide and per kg body weight of 0.4 U/kg insulin glargine. R denotes separate injections of insulin glargine (U100) and lixisenatide. T1 (mix A) and T2 (mix B) denote on-site mixes of insulin glargine (U100 and U300 respectively) with lixisenatide.

*Table 73 - Shift Table for Results of Anti-Lixisenatide Antibody Testing - up to first Post-Study Visit*

| Baseline | All (N = 26) [a] | | |
|---|---|---|---|
| | pos. [b] | neg. [b] | mis. [b] |
| positive | 0/0 | 0/0 | 0/0 |
| negative | 0/26 | 24/26 | 2/26 |
| missing | 0/0 | 0/0 | 0/0 |

[a] Total count of subjects exposed to study drugs (i.e., all subjects who took at least one dose of study drug). [b] After baseline (missing data for at least one study visit). pos. = at least one positive result; neg. = all results negative; mis. = missing data

EXAMPLE 3

A randomized, cross-over, open, euglycemic clamp study on the relative bioavailability and activity of 0.6 U/kg insuline glargine and 20 $\mu$g lixisenatide, given as on-site mix compared to separate simultaneous injections in subjects with diabetes mellitus type 1.

CLINICAL TRIAL SUMMARY

COMPOUND:

Lixisenatide / Insulin

Glargine

| TITLE | A randomized, cross-over, open, euglycemic clamp study on the relative bioavailability and activity of 0.6 U/kg insulin glargine and 20 μg lixisenatide, given as on-site mix compared to separate simultaneous injections in subjects with diabetes mellitus type 1 |
|---|---|
| STUDY OBJECTIVE(S) | Primary objective:<br><br>• to assess the relative bioavailability of a single dose of insulin glargine and lixisenatide given subcutaneously as on-site mix vs separate and simultaneous injections of each drug<br>Secondary objectives:<br>• to compare the activity of a single dose of insulin glargine and lixisenatide given subcutaneously as on-site mix vs separate and simultaneous injections of each drug<br>• to asses the safety and tolerability of insulin glargine and lixisenatide given subcutaneously as on-site mix |
| STUDY DESIGN | Phase I, single-center, open-label, randomized, cross-over (2 treatments, 2 treatment periods and 2 sequences), active control, single dose of lixisenatide and insulin glargine with a wash-out duration between treatment periods (5-18 days, preferred 7 days) |
| STUDY POPULATION Main selection criteria: | Male and female subjects, aged 18 to 65 years old, with type 1 diabetes mellitus |
| Total expected number of subjects: Expected number of sites: | 22 subjects are to be enrolled to have 18 subjects for final PK evaluation 1 |
| INVESTIGATIONAL PRODUCT(S) Formulation(s): | Insulin glargine (Lantus®) and lixisenatide (AVE0010)<br><br>• Lantus U100 solution for injection (commercially available, purchased through CRO)<br>• Lixisenatide (100 μg/mL) solution for injection (provided by Sanofi-Aventis)<br>• Solution for injection prepared on-site (by the CRO) mixing:<br>  - a fixed volume of the insulin glargin/lixisenatide pre-mix solution for injection (800 μg/mL lixisenatide in Lantus U100; provided by Sanofi-Aventis)<br>  - diluted with a variable volume of Lantus U100 (commercially available, purchased through CRO) depending on subject's body weight. |
| Route(s) of administration: Dose regimen/duration: | Subcutaneous administration into a periumbilical site of the abdomen T (Test): Single dose injection of an on-site mix of Lantus U100 and lixisenatide (800 μg/mL in Lantus U100) atone peri-umbilical site under fasting conditions, final volumes of on-site mix and lixisenatide concentrations are provided in Table 2.<br><br>R (Reference): Single dose, separate simultaneous injections of Lantus U100 and lixisenatide (100 μg/mL) at opposite peri-umbilical sites within 1 min under fasting conditions<br><br>T (Test) |

(continued)

| TITLE | A randomized, cross-over, open, euglycemic clamp study on the relative bioavailability and activity of 0.6 U/kg insulin glargine and 20 $\mu$g lixisenatide, given as on-site mix compared to separate simultaneous injections in subjects with diabetes mellitus type 1 |
|---|---|
|  | Lixisenatide 20 $\mu$g<br>insulin glargine 0.6 U/kg<br>on-site mixed lixisenatide and insulin glargine<br>(25 $\mu$l of pre-mix [800 $\mu$g/mL lixisenatide in Lantus U100] + body weight adjusted volume of Lantus U100)<br><br>R (Reference)<br>Lixisenatide 20 $\mu$g (clinical formulation), 100 $\mu$g/mL for injection<br>Lantus U100 0.6 U/kg, 100 U/mL for injection |
| PRIMARY ENDPOINT(S) AND MAIN SECONDARY ENDPOINT(S) | Primary endpoints<br>The area under the plasma lixisenatide concentration curve LIX-$AUC_{last}$ and peak concentration LIX-$C_{max}$<br>The area under the serum insulin glargine concentration curve up to 24 h (INS-$AUC_{0-24}$), time to 50% of $AUC_{0-24}$ (T50% $AUC_{0-24}$)<br>Main secondary endpoints<br>AUC and Time to $C_{max}$ ($T_{max}$) for lixisenatide<br>The area under the body weight standardized glucose infusion rate curve (GIR) within 24 h (GIR-$AUC_{0-24}$) and the time to 50% of the GIR-AUC within 24 h ($T_{50\%}$-GIR-$AUC_{0-24}$)<br>Maximum smoothed body weight standardized glucose infusion rate $GIR_{max}$, and time to $GIR_{max}$ (GIR-$T_{max}$)<br>Safety and tolerability |
| ASSESSMENT SCHEDULE | One screening visit (Day -28 to Day -3), 2 treatment visits (trial periods 1 and 2, Day 1 to Day 2), and one end-of-study (EOS) visit (one day between Day 5 and Day 9 after last dosing) with final assessment of safety parameters, and post-study antibody check at week 4 - 6.<br>Euglycemic clamp setting for 24 hours after dosing in trial periods 1 and 2 (in-house-days, dosing).<br>Blood to be collected for the determination of plasma lixisenatide concentrations on Days 1-2 in both treatment periods<br>Blood to be collected for the determination of serum insulin glargine concentrations on Days 1-2 in both treatment periods<br>Blood glucose will be continuously monitored for 24 hours after IP administration (about 28 h including pre-dose clamp time).<br>Anti-lixisenatide antibody check: At screening (if necessary), before each dosing, 4 to 6 weeks after last dosing and in addition 3 to 6 months after post study visit in case of positive result. |
| STATISTICAL CONSIDERATIONS | Pharmacokinetics:<br>PK parameters will be summarized by treatment using descriptive statistics.<br>Statistical analyses will compare test treatment (T) with the reference treatment (R).<br>Lixisenatide: For log transformed AUC, $AUC_{last}$ and $C_{max}$ the relative bioavailability between test treatment (T, on-site mix) and reference treatment (R, separate injections) will be assessed using a linear effects model. Estimate and 90% confidence interval (CI) for the ratio of geometric means between test and reference treatment will be provided for AUC, $AUC_{last}$ and $C_{max}$.<br>Insulin glargine: For log transformed $AUC_{0-24}$ the relative bioavailability between test (T) and reference treatment (R) will be assessed using a linear effects model. Estimate and 90% confidence interval (CI) for the ratio of geometric means between test and reference treatment will be provided for $AUC_{0-24}$. Time to 50% of $AUC_{0-24}$ ($T_{50\%}$-$AUC_{0-24}$) will be compared non-parametrically between test and reference treatment. Pharmacodynamics:<br>PD parameters will be summarized by treatment using descriptive statistics. |

(continued)

| TITLE | A randomized, cross-over, open, euglycemic clamp study on the relative bioavailability and activity of 0.6 U/kg insulin glargine and 20 µg lixisenatide, given as on-site mix compared to separate simultaneous injections in subjects with diabetes mellitus type 1 |
|---|---|
| | Statistical analysis will compare test treatment (T) with the reference (R). For log transformed $GIR-AUC_{0-24}$, the ratios between test (T) and reference treatment (R) will be assessed using a linear effects model. Estimate and 90% confidence interval (CI) for the ratio of geometric means between test and reference treatment will be provided for $GIR-AUC_{0-24}$. $GIR_{max}$ will be subject to corresponding analysis albeit a supplemental parameter. Times to 50% of $AUC_{0-24}$ will be compared non-parametrically between test (T) and reference treatment (R). $GIR-T_{max}$ will be subject to corresponding analysis albeit a supplemental parameter. <br><br> Safety and tolerability: <br><br> The safety analysis will be based on the review of the individual values (clinically significant abnormalities) and descriptive statistics by treatment. <br><br> For adverse events, frequencies of treatment emergent adverse events (TEAEs) classified by MedDRA system organ class and preferred term will be tabulated by treatment. All adverse events will be listed. For vital signs and ECG, frequency of subjects with abnormalities and Potentially Clinically Significant Abnormalities (PCSAs) will be summarized by treatment. |
| | Frequencies for signs of local intolerance will be analyzed per treatment. |
| DURATION OF STUDY PERIOD (see Figure 16) (per Subject) | Total study duration for one subject: about 1 month (up to 7 months) Duration of each part of the study for one subject: <br> Screening: 3 to 28 days (D-28 to D-3) <br> Period 1: 2 days (1 overnight stay) <br> Washout: 5-18 days (preferentially 7 days between consecutive dosings) <br> Period 2: 2 days (1 overnight stay) <br> End-of-study visit: 1 day between D5 and D9 of trial period 2 <br> Post-study visit (for anti lixisenatide anti-body check): 4 to 6 weeks after last dosing <br> Follow-up visit (for anti lixisenatide anti-body check): 3-6 months after PSV, if necessary |

## 1 FLOW CHARTS

### 1.1 STUDY FLOW CHART (SCREENING TO POST-STUDY VISIT)

| Phase Trial period (TP) | Screening | Treatment Period TP1 | | Washout 5-18 days, preferred 7 days between consecutiv e dosing | Treatment Period TP2 | | End-of-study visit | Post-study visit |
|---|---|---|---|---|---|---|---|---|
| Days | D-28 to D-3 (I) | D-2/D-1 | D1 to D2 | | D-2/ D-1 | D1 to D2 | D5 to D9 after TP2 | 4-6 weeks after D2 of TP2 |
| Informed Consent | X | | | | | | | |
| Institutionalization | | | X | | | X | | |
| Discharge | | | D2 14:00h | | | D2 14:00h | | |
| Inclusion/exclusion Criteria | X | | X (a) | | (a) | (a) | | |
| Prior/Concomitant medication | | | <------------------------------------------------------------------------------------------------------> | | | | | |
| Medical/surgical History | X | | | | | | | |
| Physical examination | X | | X | | | X | X | |
| Height | X | | | | | | | |
| Body weight | X | | X | | | X | X | |
| Archival blood sampling | | | X (D1) | | | | | |
| Serologies | X | | | | | | | |
| Urine Drug Screen (b) | X | | X | | | X | | |
| Alcohol breath test | X | | X | | | X | | |
| Change of basal insulin (i) | | X | X | | X | X | | |
| Randomization | | | X | | | | | |
| Investigational Product Administration | | | X | | | X | | |

EP 2 898 895 A1

| Phase Trial period (TP) | Screening | Treatment Period TP1 | | Washout 5-18 days, preferred 7 days between consecutive dosing | Treatment Period TP2 | | End-of-study visit | Post-study visit |
|---|---|---|---|---|---|---|---|---|
| Days | D-28 to D-3 (I) | D-2/D-1 | D1 to D2 | | D-2/ D-1 | D1 to D2 | D5 to D9 after TP2 | 4-6 weeks after D2 of TP2 |
| SAFETY | | | | | | | | |
| Blood pressure/Heart rate (c) | X | | X | | | X | X | |
| Body temperature (d) | X | | X | | | X | X | |
| ECG monitoring | | | X | | | X | | |
| 12-lead ECG (e) | X | | X | | | X | X | |
| Blood Laboratory Examination (f,m) | X | | X | | | X | X | |
| Urinalysis (g) | X | | X | | | X | X | |
| Local tolerability | | | X | | | X | | |
| AE/SAE collection | | | <------------------------------------------------------------------------------------------------------> | | | | | |
| PHARMACOKINETICS | | | | | | | | |
| Blood collection for lixisenatide | | | X | | | X | | |
| insulin glargine | | | X | | | X | | |
| anti lixisenatide antibody | PEY00(j) | | PE00 (h) | | | PE01 (h) | | PE02 (k) |
| PHARMACODYNAMICS - CLAMP PROCEDURES | | | | | | | | |
| Glucose infusion on demand (clamp) | | | X | | | X | | |
| Blood glucose monitoring | | | X | | | X | | |

47

(continued)

| Phase Trial period (TP) | Screening | Treatment Period TP1 | | Washout 5-18 days, preferred 7 days between consecutiv e dosing | Treatment Period TP2 | | End-of-study visit | Post-study visit |
|---|---|---|---|---|---|---|---|---|
| Days | D-28 to D-3 (I) | D-2/D-1 | D1 to D2 | | D-2/ D-1 | D1 to D2 | D5 to D9 after TP2 | 4-6 weeks after D2 of TP2 |
| Phase Trial period (TP) | Screening | Treatment Period TP1 | | Washout 5-18 days, preferred 7 days between consecutiv e dosing | Treatment Period TP2 | | End-of-study visit | Post-study visit |
| Days | D-28 to D-3 (I) | D-2/D-1 | D1 to D2 | | D-2/ D-1 | D1 to D2 | D5 to D9 after TP2 | 4-6 weeks after D2 of TP2 |

a period 1 D1 inclusion, Period 2 just check absence of infection and adherence to study restrictions

b Urine drug screen: amphetamines/metamphetamines, barbiturates, benzodiazepines, cannabinoids, cocaine, opiates

c Vital signs (heart rate, respiratory rate and blood pressure, measured after 10 min in supine resting position, and after 3 min in standing position [except when connected to Biostator])

d Body temperature will be measured aurally

e 12 lead ECG will be recorded after at least 10 min in supine position. Automatic reading will be performed

f Hematology: Hemoglobin, HbA1c (screening, D1 TP1 and EOS), Hematocrit, Red Blood Cell Count, White Blood Cell Count with differential (Neutrophils, Lymphocytes, Monocytes, Basophils, Eosinophils), Platelets, INR and aPTT; Serum Chemistry: Sodium, Potassium, Chloride, Bicarbonate, Calcium, Glucose, Albumin, total Protein, total Cholesterol, Triglycerides, Creatinine, BUN, AST (SGOT), ALT (SGPT), Gamma-Glutamyl Transferase (GGT); Alkaline Phosphatase (ALP), Lactate Dehydrogenase (LDH), total and conjugated Bilirubin, CPK, Amylase, Lipase;; C-peptide; HIV, Hepatitis B and C at screening

g Urinalysis: proteins, glucose, blood, ketone bodies, pH

h Antibody determination in each Treatment Period before dosing j Subjects on long-acting basal insulin change to short acting insulins no later than 48 hrs pre dose. Subject on NPH or other intermediate insulin change to short acting insulins no later than 24 hrs pre dose. Subjects resume normal pre-study insulin medication after discharge on D2 TP1/TP2

j only for subjects who received a GLP-1 agonist before (e.g. exenatide, lixisenatide)

k if positive for anti-lixisenatide antibodies, follow-up samples will be taken within a 3 to 6 month period after post study visit

l For subjects having received GLP-1 agonist treatment before, the screening visit should be no later than 7 days before inclusion (D1 / TP1) due to the time required to perform the test on antibodies

m if female: plasma-$\beta$-HCG at screening, at TP1 and TP2 urine $\beta$-HCG only; plasma-FSH/estradiol if postmenopausal less than 2 years and at screening only

## 1.2 TREATMENT PERIOD FLOW CHART (PERIOD 1 AND 2)

| Day per trial period | D-2/ D-1 | D1 | | | | | | | | | | | | | | | | | | D2 | | | | | | | | |
|---|---|---|---|---|---|---|---|---|---|---|---|---|---|---|---|---|---|---|---|---|---|---|---|---|---|---|---|---|
| Indicative clock time | 12:00 | 07:00 | 08:00 | 09:00 | 10:00 | 11:00 | 12:00 | 12:15 | 12:30 | 13:00 | 13:30 | 14:00 | 14:30 | 15:00 | 16:00 | 17:00 | 18:00 | 20:00 | 22:00 | 00:00 | 02:00 | 04:00 | 06:00 | 08:00 | 10:00 | 12:00 | 13:00 | 14:00 |
| PK time (hour/minute) (m) | | | | | | | 0H | 0H15 | 0H30 | 1H | 1H30 | 2H | 2H30 | 3H | 4H | 5H | 6H | 8H | 10H | 12H | 14H | 16H | 18H | 20H | 22H | 24H | | |
| **Procedures** | | | | | | | | | | | | | | | | | | | | | | | | | | | | |
| Change of basal insulin (if applicable) | X | | | | | | | | | | | | | | | | | | | | | | | | | | | |
| Institutionalization | | < | --- | --- | --- | --- | --- | --- | --- | --- | --- | --- | --- | --- | --- | --- | --- | --- | --- | --- | --- | --- | --- | --- | --- | > | | |
| Discharge | | | | | | | | | | | | | | | | | | | | | | | | | | | | X |
| Inclusion/exclusion criteria (a) | | X | | | | | | | | | | | | | | | | | | | | | | | | | | |
| Physical examination | | X | | | | | | | | | | | | | | | | | | | | | | | | | | |
| Concomitant medication | < | --- | --- | --- | --- | --- | --- | --- | --- | --- | --- | --- | --- | --- | --- | --- | --- | --- | --- | --- | --- | --- | --- | --- | --- | --- | --- | > |
| Body weight | | X | | | | | | | | | | | | | | | | | | | | | | | | | | |
| Archival blood sampling (TP1 only) | | | | | | X | | | | | | | | | | | | | | | | | | | | | | |
| Urine Drug Screen (b) | | X | | | | | | | | | | | | | | | | | | | | | | | | | | |
| Alcohol Breath Test | | X | | | | | | | | | | | | | | | | | | | | | | | | | | |
| Randomization (TP1 only) after Investigators confirmation of subjects eligibility | | X | | | | | | | | | | | | | | | | | | | | | | | | | | |
| Meals (c) | X | | | | | | | | | | | | | | | | | | | | | | | | | | | ad lib |

| Day per trial period | D-2/ D-1 | D1 | | | | | | | | | | | | | | | | | | D2 | | | | | | | | |
|---|---|---|---|---|---|---|---|---|---|---|---|---|---|---|---|---|---|---|---|---|---|---|---|---|---|---|---|---|
| Indicative clock time | 12:00 | 07:00 | 08:00 | 09:00 | 10:00 | 11:00 | 12:00 | 12:15 | 12:30 | 13:00 | 13:30 | 14:00 | 14:30 | 15:00 | 16:00 | 17:00 | 18:00 | 20:00 | 22:00 | 00:00 | 02:00 | 04:00 | 06:00 | 08:00 | 10:00 | 12:00 | 13:00 | 14:00 |
| PK time (hour/minute) (m) | | | | | | | 0H | 0H15 | 0H30 | 1H | 1H30 | 2H | 2H30 | 3H | 4H | 5H | 6H | 8H | 10H | 12H | 14H | 16H | 18H | 20H | 22H | 24H | | |
| Fasting (l) | | <-------------------------------------------------------------------------------> | | | | | | | | | | | | | | | | | | | | | | | | | | |
| Investigational Product Administration (e) | | | | | | | X s c | | | | | | | | | | | | | | | | | | | | | |
| **SAFETY** | | | | | | | | | | | | | | | | | | | | | | | | | | | | |
| Blood Pressure/Heart Rate (f) | | X | | | | X | | | | | | | | | | | | | | | | | | | | | X | |
| Body temperature (g) | | X | | | | | | | | | | | | | | | | | | | | | | | | | X | |
| ECG monitoring (telemetry) | | <-------------------------------------------------------------------------------> | | | | | | | | | | | | | | | | | | | | | | | | | | |
| 12-lead ECG (h) | | X | | | | | | | | | | X | | | | | | | | X | | | | | | X | X | |
| Blood Laboratory Examination (i,n) | | X | | | | | | | | | | | | | | | | | | | | | | | | | | |
| Urinalysis (j) | | X | | | | | | | | | | | | | | | | | | | | | | | | | | |
| Local tolerability | | | | | | | | X | | X | | | | | | | | | | | | | | | | | | |
| Anti-lixisenatide antibodies (k) | | | | | | | X | | | | | | | | | | | | | | | | | | | | | |
| AE/SAE collection | | <-------------------------------------------------------------------------------> | | | | | | | | | | | | | | | | | | | | | | | | | | |
| **PHARMACOKINETICS** | | | | | | | | | | | | | | | | | | | | | | | | | | | | |
| Blood collection for lixisenatide | | | | | | | P0 | P0 | P0 | P0 | P0 | P0 | P0 | P0 | P0 | P0 | P0 | P0 | P0 | | | | | | | P1 | | |
| Blood collection for insulin glargine | | | | | | | S0 | S0 | S0 | S0 | S0 | S0 | | S0 | S0 | | S0 | | | S1 | S1 | S1 | S1 | S1 | S1 | S1 | | |

EP 2 898 895 A1

| Day per trial period | D-2/ D-1 | D1 | | | | | | | | | | | | | | | | | | D2 | | | | | | | | |
|---|---|---|---|---|---|---|---|---|---|---|---|---|---|---|---|---|---|---|---|---|---|---|---|---|---|---|---|---|
| Indicative clock time | 12:00 | 07:00 | 08:00 | 09:00 | 10:00 | 11:00 | 12:00 | 12:15 | 12:30 | 13:00 | 13:30 | 14:00 | 14:30 | 15:00 | 16:00 | 17:00 | 18:00 | 20:00 | 22:00 | 00:00 | 02:00 | 04:00 | 06:00 | 08:00 | 10:00 | 12:00 | 13:00 | 14:00 |
| PK time (hour/minute) (m) | | | | | | | 0H | 0H15 | 0H30 | 1H | 1H30 | 2H | 2H30 | 3H | 4H | 5H | 6H | 8H | 10H | 12H | 14H | 16H | 18H | 20H | 22H | 24H | | |
| **PHARMACODYNAMICS / BIOMARKERS** | | | | | | | | | | | | | | | | | | | | | | | | | | | | |
| Insulin-glucose infusion (pre-clamp) (d) | | < ----- > | | | | | | | | | | | | | | | | | | | | | | | | | | |
| Glucose infusion (clamp) | | | | | < ------- continuously on demand ------- > | | | | | | | | | | | | | | | | | | | | | | | |
| Blood glucose monitoring | | | < -------- continuously -------- > | | | | | | | | | | | | | | | | | | | | | | | | | |
| Glucose calibration | | | < -------- at least every 30 min -------- > | | | | | | | | | | | | | | | | | | | | | | | | | |

a Immediately before dosing in Treatment Period 1 (TP1), in Treatment Period 2 just check absence of infection and adherence to study restrictions. b Urine drug screen: amphetamines/metamphetamines, barbiturates, benzodiazepines, cannabinoids, cocaine, opiates. c Fluid = (water) ad libitum. d Pre-clamp on D1 TP1 and TP2 from 08:00 to 12:00. e Subcutaneous abdominal administration, either separately simultaneously, horizontally 5 cm right and left of the umbilicus, or single sc administration into one of either site according to randomized sequence. f Vital signs (heart rate, respiratory rate and blood pressure, measured after 10 min in supine resting position, and after 3 minutes in standing position [except when connected to Biostator] ). g Body temperature will be measured aurally. h 12 lead ECG will be recorded after at least 10 min in supine position. Automatic reading will be performed. I Hematology: Hemoglobin, HbA1c (screening, D1 TP1 and EOS), Hematocrit, Red Blood Cell Count, White Blood Cell Count with differential (Neutrophils, Lymphocytes, Monocytes, Basophils, Eosinophils), Platelets, INR and aPTT; Serum Chemistry: Sodium, Potassium, Chloride, Bicarbonate, Calcium, Glucose, Albumin, total Protein, total Cholesterol, Triglycerides, Creatinine, BUN, AST (SGOT), ALT (SGPT), Gamma-Glutamyl Transferase (GGT); Alkaline Phosphatase (ALP), Lactate Dehydrogenase (LDH), total and conjugated Bilirubin, CPK, Amylase, Lipase; if female: plasma-FSH and β-HCG, at TP1 and TP2 urine β-HCG only. j Urinalysis: proteins, glucose, blood, ketone bodies, pH. k Antibody determination in each Treatment Period before dosing. l starting 10hrs before admission and during clamp. m time (hour/minute) is expressed in reference to the last administration of IP (T0H). n if female: plasma-β-HCG at screening, at TP1 and TP2 urine β HCG only; plasma-FSH/estradiol if postmenopausal less than 2 years and at screening only

EP 2 898 895 A1

2. List of abbreviations

| | |
|---|---|
| °C | Degrees Celsius |
| AE | Adverse Event |
| AEPM | Adverse Event with pre-specified monitoring |
| ALT | Alanine aminotransferase |
| ARAC | Allergic Reaction Assessment Committee |
| ARF | Acute Renal Failure |
| AST | Aspartate Aminotransferase |
| AUC | Area under the curve |
| β-HCG | β-Human Chorionic Gonadotrophin |
| BID | bis in die (twice daily) |
| BMI | Body mass index |
| BP | Blood pressure |
| bpm | Beats per minute |
| CPK | Creatine phosphokinase |
| CRF | Case Report Form |
| D | Day |
| DRF | Discrepancy Resolution Form |
| ECG | Electrocardiogram |
| EDTA | Ethylenediaminetetra-acetic acid |
| EOS | End-of-study |
| FSH | Follicle Stimulation hormone |
| GCP | Good Clinical Practice |
| GIR | (body weight standardized) glucose infusion rate |
| GLP-1 | Glucagon like Peptide 1 |
| h | Hour(s) |
| Hb | Hemoglobin |
| HBs | Hepatitis B surface |
| Hct | Hematocrit |
| HCV | Hepatitis C virus |
| HIV | Human immunodeficiency virus |
| HR: | Heart Rate |
| IP: | Investigational Product |
| IRB/IEC | Institutional Review Board/Independent Ethics Committee |
| iv | intravenous |
| kg | Kilogram |
| NPH | Neutral Protamine Hagedorn (NPH-insulin) |
| NTEAE | Non-treatment emergent adverse event |
| QD | quaque die (once daily) |
| QTc | QT interval automatically corrected by the ECG machine |
| R | Reference medication |
| RBC | Red blood cell count |
| SAE | Serious adverse event |
| SBP | Systolic blood pressure |
| sc | subcutaneous |
| T1(2)DM | Type 1 (2) diabetes mellitus |
| TP1 / TP2 | Treatment Periods |
| TEAE | Treatment emergent adverse event |
| TP | Trial Period |
| UDS | Urine drug screen |
| ULN | Upper Limit of Normal range |
| WBC | White blood cell count |

Pharmacokinetic parameters definitions provided in Section 9.3.5.

3. INTRODUCTION

Lixisenatide (drug code: AVE0010) is a polypeptide with pronounced Glucagon like Peptide 1 (GLP-1) agonistic activities and is being developed for the treatment of type 2 diabetes to improve glycemic control. GLP-1 is the acronym for the mammalian incretin hormone Glucagon like Peptide 1. Lixisenatide is being given subcutaneously and predominantly addresses post-prandial glucose control.

Lixisenatide has been shown to provide optimum efficacy to tolerance ratio at 20 $\mu$g QD, which defines the current clinically relevant dose in clinical trials.

Lantus is an insulin product containing insulin glargine. Lantus provides 24 hour basal insulin supply after single dose subcutaneous injection and predominantly addresses fasting blood glucose control. Insulin glargine is 31$^B$-32$^B$-Di-Arg human insulin, an analogue of human insulin, with further substitution of asparagine in position A21 by glycine. Lantus is the current gold standard for basal insulin supplementation, titrated to target and efficacious when given once daily. Lantus is marketed since June 2000 in Europe, since May 2001 in USA, and other parts of the world.

Further details on Lantus can be also found in the EU SmPc http://www.ema.europa.eu/humandocs/PDFs/EPAR/Lantus/emea-combined-h284en.pdf.

More detailed information for lixisenatide (AVE0010) is provided in the Investigator's Brochure.

4. RATIONALE

4.1 Study rationale

Both insulin glargine and lixisenatide are efficacious when given once daily, they are administered subcutaneously and share similar physicochemical features, such as good solubility at low pH. This would allow the preparation of a pre-mix solution in which lixisenatide is mixed with Lantus U100, so that separate simultaneous injections may be replaced by one single injection delivering both components as a mixed formulation.

The combined drug regimen of this Example fixes the lixisenatide dose to 20 $\mu$g for any subject while leaving the Lantus dose to be adjusted as needed. To match this with a single injection, different medical device options are designed which deliver both drugs from two sources via one needle. These options will use a premixed solution of lixisenatide at a high concentration in Lantus U100 as one source, which will be mixed in the device with Lantus U100, the other source, while being injected.

The purpose of this study is to assess the exposure and the glucodynamic activity of such a mixture ('on-site mix') consisting of an insulin glargine/lixisenatide pre-mix diluted with Lantus U100, compared to the exposure and activity of both drugs when given separately. A Lantus dose of 0.6 U/kg will be given together with a fixed dose of 20 $\mu$g lixisenatide.

4.2 Design rationale and risk assessment

The present study is designed to assess the relative bioavailability and activity of 0.6 U/kg insulin glargine (0.6 U/kg Lantus U100) and 20 $\mu$g lixisenatide (200 $\mu$L lixisenatide 100 $\mu$g/mL) given separately simultaneously (= Reference R) and given as on-site mixed formulation of lixisenatide (800 $\mu$g/mL in Lantus U100) and Lantus U100 (= Test T) in an euglycemic clamp setting in subjects with diabetes mellitus type 1. The study will comprise 2 treatment periods

(TP). There will be one screening visit (D-28 to D-3), 2 treatment visits (D1 to D2 in TP1 and TP2), and one end-of-study visit (TP2, D5 to D9) with final assessment of safety parameters. A post study visit will follow to check if subjects have developed anti-lixisenatide antibodies.

Subjects will be exposed to each treatment R and T once in a cross-over and randomized manner. Subjects will be randomized (1:1) to sequences R T, T R such that each subject receives the reference (R, separate simultaneous injection) and the on-site mix (T). This design is considered appropriate to evaluate the relative bioequivalence of the on-site mix compared to the reference treatment.

The Lantus dose of 0.6 U/kg selected for the planned study is the typical strength to be used in type 2 diabetes patients, the intended target population for the pre-mix of Lantus and lixisenatide. Furthermore, the selected dose of 0.6 U/kg is above the average basal need in the study population which in turn will produce some measurable effects in the euglycemic clamp (i.e. glucose demand reflected in a sizeable GIR up to and even beyond 24 h).

The lixisenatide dose of 20 $\mu$g QD is considered the clinically relevant dose as it has been shown during clinical development that it provides optimum efficacy with good tolerability.

The primary objective of this study will be to determine insulin glargine and lixisenatide exposure. The lack of an assay specific for insulin glargine forces to use an assay which reads all endogenous insulin. Thus, any added source of insulin other than exogenous insulin glargine will cause falsely too high insulin concentrations. Therefore, subjects without endogenous insulin production (i.e. with diabetes mellitus type 1) will be included in this trial. Also, insulin release mediated by lixisenatide could be excluded in type 1 diabetes patients, while effects on gastric emptying, glucagon secretion and satiety remain.

Assessment of glucodynamic activity requires a euglycaemic clamp setting for up to 24 hours owed to the long duration of action.

Risk-benefit assessment

The population to be treated in this study are patients with type 1 diabetes already adjusted to standard medical care (incl. insulin treatment) and will thus have no general benefit from participating in this study. Furthermore, due to the objectives and duration of this study, involved patients will not immediately benefit from the outcome of this study.

Both, insulin glargine and lixisenatide are well characterized with respect to preclinical and clinical safety.

In clinical studies conducted with lixisenatide, the most frequent treatment-emergent adverse events (TEAEs) of lixisenatide were nausea, vomiting, and diarrhea. They were usually mild in intensity, appearing transiently at the beginning of the treatment. A low incidence of hypoglycemia was reported, mainly with administration of sulfonylurea with lixisenatide.

The risk associated with being exposed first time to lixisenatide is confined to gastrointestinal adverse reactions such as nausea and vomiting, which may be related to central and peripheral effects, such as slowing of gastric emptying. However, the participants are fasting and do not receive a meal prior to the end of clamp, which reduces the risk. Similarly, other predominant effects such as inhibiting glucagon secretion and increasing satiety remain, which do not pose problems as no meal is given. Also, the lack of effects mediated by release of endogenous insulin prevents adverse reactions associated with stimulated insulin release.

Lantus (insulin glargine) has been marketed since June 2000 in Europe and since May 2001 in the USA and other parts of the world and is one of the most widely used insulins by patients with T1DM and T2DM. According to the prescribing information the TEAEs of Lantus are hypoglycemia, injection site reaction, and immediate-type allergic reactions. Insulin glargine induced severe hypoglycemia at high doses in non-diabetic animals. An overall risk of hypoglycemia is not completely excluded, but can be controlled by glucose clamp design with continues glucose monitoring as planned in this study.

Based on the different pharmacological mode of actions (GLP-1 agonism versus insulin action), no safety-relevant interaction between the compounds is expected in the frame of the planned clinical study.

A combined parallel treatment with lixisenatide and insulin glargine has been evaluated in two other studies (Example 1 and Example 2) with a similar study design and study population. None of those studies provided any safety concern for the combined treatment of both drugs.

In Example 1 two strengths of a pre-mixed solution containing lixisenatide and insulin glargine (0.66 or 0.25 $\mu$g lixisenatide per 1 U insulin glargine), given subcutaneously as a single injection, were compared to a separate simultaneous injection of both drugs in type 1 diabetes subjects (N=42). Study design was cross-over with two treatments, meaning that each subject received reference treatment and one of the two pre-mixes.

In Example 2 two on-site mix solutions containing lixisenatide and insulin glargine (100 $\mu$g/mL lixisenatide + 100 or 300 U/mL insulin glargine), given subcutaneously as a single injection, were compared to a separate simultaneous injection of both drugs in type 1 diabetes subjects (N=26). Study design was cross-over with three treatments, meaning that each subject received reference treatment and both of the two on-site mixes.

All treatments were well tolerated and judged safe for the study population in both studies. Headache was the most common AE, likely related to the clamp procedure. Drug specific AEs were gastrointestinal symptoms, linked to lixisenatide.

The predominant risk for participants is loss of blood glucose control while preparing for the clamp days. However, subjects with diabetes mellitus type 1 are dependent on basal-bolus insulin replacement therapy and are used to adapt to variations in life-style and food. To change insulin treatment regimen is not uncommon to these patients. Therefore, although demanding, self-preparation for the clamp and switching back to daily routine is well managed by subjects dependent on insulin.

The risks associated with the clamp procedure are limited to the inconvenience being attached to the experimental setting for more than a day. The predominantly reported adverse event is headache. Participants are hospitalized under close supervision with effective blood glucose control when receiving the study medication. Thus, the risks associated with medication and the experimental set up are limited.

The risk for developing anti-lixisenatide antibodies is considered very low, in view of the very short treatment duration. In a previous study (ACT6011) antibodies were not detected at the end of a two week period after repeated injections. Antibody formation impacts the exposure of lixisenatide, and thus if detected in a subject during the course of this study will lead to exclusion of the respective treatment period for PK evaluation of lixisenatide. Subjects will be assessed following end of treatment for the occurrence of anti-lixisenatide antibodies.

4.3 Specific parameters rationale

Pharmacodynamics

The pharmacodynamic activity of insulin glargine will be evaluated by the euglycemic clamp technology. This technology is the gold standard to evaluate the effect of exogenous administered insulins on blood glucose disposal in type 1 diabetes patients and provide reliable and accurate results.

Parameters specific for assessment of glucose disposition in euglycemic clamp settings are the body weight standardized glucose infusion rate (GIR) profiles, total GIR-AUC (GIR-AUC$_{0-24}$) and times to a given percentage of GIR-AUC$_{0-24}$ such as Time to 50% of GIR-AUC$_{0-24}$.

Further parameters are the Maximum smoothed body weight standardized GIR, GIR$_{max}$, and Time to GIR$_{max}$, GIR-T$_{max}$.

Safety

Adverse events considered potentially related to an allergic reaction will be documented on a specific form and reviewed by an independent Allergic Reaction Assessment Committee (ARAC) based on the information available (see 6.4.1).

Anti-lixisenatide antibodies will be assessed during the study and a follow-up visit after end of treatment and in addition 3 to 6 months after post study visit in case of positive result.

For subjects having received GLP-1 agonist treatment before, anti-lixisenatide antibodies will be assessed at screening visit.

Pharmacokinetics

As to lixisenatide, AUC$_{last}$ is preferred to AUC as primary endpoint since using AUC there is the risk of exclusion of some AUC values if the extrapolated portion is higher than 30%.

As to insulin glargine, lack of pronounced maximum concentration of insulin glargine due to the sustained release nature of the product prompts to employ instead of INS-T$_{max}$ the time to 50% of INS-AUC (T50% INS-AUC$_{0-24}$) as measure for the time location of the insulin glargine exposure profile.

5. STUDY OBJECTIVES

5.1 PRIMARY

To assess the relative bioavailability of a single dose of insulin glargine and lixisenatide given subcutaneously as on-site mix versus separate and simultaneous injections of each drug.

5.2 SECONDARY

To compare the activity of a single dose of insulin glargine and lixisenatide given subcutaneously as on-site mix versus given separately and simultaneously of each drug and to asses the safety and tolerability of insulin glargine and lixisenatide given subcutaneously as on-site mix.

6 STUDY DESIGN

6.1 DESCRIPTION OF THE PROTOCOL

Phase I, single-center, open-label, randomized, cross-over (2 treatments, 2 treatment periods and 2 sequences), active control study with a wash-out period between treatments (5-18 days, preferred 7 days) in male and female subjects with type 1 diabetes mellitus receiving single-doses of lixisenatide and insulin glargine as

- separate simultaneous injections of insulin glargine (Lantus U100) and lixisenatide (100 µg/mL) at opposite peri-umbilical sites (= Reference R) and
- on-site mix of a lixisenatide/insulin glargine pre-mix (lixisenatide 800 µg/mL in Lantus® U100) diluted with insulin glargine (Lantus U100) at one peri-umbilical site (= Test T).

The two treatments R and T will be given cross-over in two treatment periods (TP 1 and TP 2) with the two-sequences R-T or T-R randomly assigned to the subjects.

To prevent interference of subjects standard insulin treatment with the clamp measurement, subjects have to abstain from using basal insulins and switch to short-acting insulins from

- 48 hours prior to dosing at D1 of TP1 and TP2, if on long-acting insulin products, i.e. Lantus (insulin glargine), Levemir (detemir) or ultralente insulins,
- 24 hours prior to dosing at D1 of TP1 and TP2 if on intermediate acting insulin products, i.e. NPH-insulin

## 6.2 DURATION OF STUDY PARTICIPATION

- Total study duration for one subject: about 1 month (up to 7 months)
- Duration of each part of the study for one subject:

  - Screening: 3 to 28 days (D-28 to D-3)
  - Period 1: 2 days (1 overnight stay)
  - Washout: 5 - 18 days (preferentially 7 days between consecutive dosings)
  - Period 2: 2 days (1 overnight stay)
  - End-of-study visit (EOS): 1 day between D5 and D9 of TP2

- Post-study visit (PSV): 4 to 6 weeks after last dosing (test for anti-lixisenatide antibodies)
- Follow up visit (FUV): 3-6 months following post study visit; only for subjects who were tested positive for anti-lixisenatide antibodies at PSV

## 6.3 INTERIM ANALYSIS

No interim analysis is planned.

## 6.4 STUDY COMMITTEES

### 6.4.1 Allergic Reaction Assessment Committee

Since lixisenatide is a peptide that may potentially generate allergic reactions, an Allergic Reaction Assessment Committee (ARAC) has been set up. The ARAC is a committee of experts in the field of allergy, independent from the Sponsor and the investigators, implemented to assess allergic reactions or allergic-like reactions that may occur during the study. The mission of the ARAC is to adjudicate, in a timely manner all allergic or possible allergic events.

Sometimes transient, injection site reactions, irritant in nature may occur requiring no intervention and are of dubious significance. These reactions would not be considered to be allergic reactions, however they are to be documented as adverse events. Virtually all symptoms listed on the CRF "Allergic Reaction Complementary Form" are possible adverse reactions that may be allergic in nature and may need to be addressed after medical judgment, excluding another etiology than allergy.

Adverse events that are obviously not of allergic origin (e.g. local injection site reactions) should not be recorded on the Allergic Reaction Complementary Form.

Adverse events that may constitute an allergic reaction (e.g. generalized itch, nasal itch, swelling at injection site, flushing, hives, swelling at lips, eyes, face, tongue, hands, feet, lump in throat, difficulty to swallow, hoarseness, change in pitch of voice, incapacity to speak, wheezing, chest tightness, stridor, etc) should be considered to be reported on the Allergic Reaction Complementary Form.

The ARAC reviews the reported cases and determines the nature of the event, confirms the allergic nature of alternative diagnosis based on the information reported by the investigator.

## 7. SELECTION OF SUBJECTS

Subjects who qualify for the study based on inclusion and exclusion criteria may be enrolled. In addition to the inclusion rules, the information provided in Section 10.3 and Section 10.5 (stopping rules) should be considered from the screening throughout the end of the study.

7.1 NUMBER OF SUBJECTS PLANNED

At least 22 subjects are to be enrolled to have 18 evaluable male and female subjects.

7.2 INCLUSION CRITERIA

Demography

I 01.	Male or female subjects, between 18 and 65 years of age, inclusive, with diabetes mellitus type 1 for more than one year, as defined by the American Diabetes Association (Referenz 1)
I 02.	Total insulin dose of < 1.2 U/kg/day
I 03.	Body weight between 50.0 kg and 95.0 kg inclusive if male, between 50.0 kg and 85.0 kg inclusive if female, Body Mass Index between 18.0 and 30.0 $kg/m^2$ inclusive

Health Status

I 04.	Fasting negative serum C-peptide (< 0.3 nmol/L)
I 05.	Glycohemoglobin (HbA1c) $\leq$9.0%
I 06.	Stable insulin regimen for at least 2 months prior to study (with respect to safety of the subject and scientific integrity of the study)
I 07.	Normal findings in medical history and physical examination (cardiovascular system, chest and lungs, thyroid, abdomen, nervous system, skin and mucosae, and musculo-skeletal system), unless the investigator considers any abnormality to be clinically irrelevant and not interfering with the conduct of the study (with respect to safety of the subject and scientific integrity of the study)
I 08.	Normal vital signs after 10 minutes resting in the supine position: 95 mmHg < systolic blood pressure < 140 mmHg; 45 mmHg < diastolic blood pressure < 90 mmHg; 45 bpm < heart rate < 100 bpm
I 09.	Normal standard 12-lead ECG after 10 minutes resting in the supine position; 120 ms < PQ < 220 ms, QRS < 120 ms, QTc $\leq$440 ms if male, 450 ms if female
I 10.	Laboratory parameters within the normal range (or defined screening threshold for the Investigator site), unless the Investigator considers an abnormality to be clinically irrelevant for diabetes patients; however serum creatinine should be strictly below the upper laboratory norm; hepatic enzymes (AST, ALT) and bilirubin (unless the subject has documented Gilbert syndrome) should be not above 1.5 ULN

Female subjects only

I 11.	Women of childbearing potential (less than two years post-menopausal or not surgically sterile for more than 3 months), must have a negative serum $\beta$-HCG pregnancy test at screening and a negative urine $\beta$-HCG pregnancy test at Day 1 on TP1 and TP2 and must use a highly effective method of birth control, which is defined as those which result in a low failure rate (i.e. less than 1% per year) according to the Note for guidance on non-clinical safety studies for the conduct of human clinical trials for pharmaceuticals (CPMP/ICH/286/95, modifica-tions). During the entire study female subjects of child bearing potential must use two independent methods of contraception, e.g. diaphragm and spermicide-coated condom. The use of a condom and spermicidal creams is not sufficiently reliable.
For postmenopausal women with presence of less than two years post-menopausal, and not surgically sterile for more than 3 months, the hormonal status will be determined (FSH > 30 IU/L, estradiol < 20 pg/mL)

Regulations

I 12.	Having given written informed consent prior to any procedure related to the study
I 13.	Covered by a Health Insurance System where applicable, and/or in compliance with the recommendations of the national (German) laws in force relating to biomedical research

I 14.     Not under any administrative or legal supervision

## 7.3 EXCLUSION CRITERIA

Medical history and clinical status

E 1.     Any history or presence of clinically relevant cardiovascular, pulmonary, gastrointestinal, hepatic, renal, metabolic (apart from diabetes mellitus type 1), hematological, neurological, psychiatric, systemic (affecting the body as a whole), ocular, gynecologic (if female), or infectious disease; any acute infectious disease or signs of acute illness

E 2.     More than one episode of severe hypoglycemia with seizure, coma or requiring assistance of another person during the past 6 months

E 3.     Frequent severe headaches and / or migraine, recurrent nausea and / or vomiting (more than twice a month)

E 4.     Blood loss ($\geq$300 ml) within 3 months before inclusion

E 5.     Symptomatic hypotension (whatever the decrease in blood pressure), or asymptomatic postural hypotension defined by a decrease in SBP equal to or greater than 20 mmHg within three minutes when changing from the supine to the standing position

E 6.     Presence or history of a drug allergy or clinically significant allergic disease according to the Investigator's judgment

E 7.     Likelihood of requiring treatment during the study period with drugs not permitted by the clinical study protocol

E 8.     Participation in a trial with any investigational drug during the past three months

E 9.     Symptoms of a clinically significant illness in the 3 months before the study, which, according to the investigator's opinion, could interfere with the purposes of the study

E 10.     Presence of drug or alcohol abuse (alcohol consumption > 40 grams / day)

E 11.     Smoking more than 5 cigarettes or equivalent per day, unable to refrain from smoking during the study

E 12.     Excessive consumption of beverages with xanthine bases (> 4 cups or glasses / day)

E 13.     If female, pregnancy (defined as positive $\beta$-HCG blood test), breast-feeding

Interfering substance

E 14.     Any medication (including St John's Wort) within 14 days before inclusion, or within 5 times the elimination half-life or pharmacodynamic half-life of that drug, whichever the longest and regular use of any medication other than insulins in the last month before study start with the exception of thyroid hormones, lipid-lowering and antihypertensive drugs, and, if female, with the exception of hormonal contraception or menopausal hormone replacement therapy, any vaccination within the last 28 days

General conditions

E 15.     Subject who, in the judgment of the Investigator, is likely to be non-compliant during the study, or unable to cooperate because of a language problem or poor mental development

E 16.     Subject in exclusion period of a previous study according to applicable regulations

E 17.     Subject who cannot be contacted in case of emergency

E 18.     Subject is the investigator or any sub-investigator, research assistant, pharmacist, study coordinator, or other staff thereof, directly involved in the conduct of the protocol.

Biological status

E 19.     Positive reaction to any of the following tests: hepatitis B surface (HBs Ag) antigen, anti-hepatitis B core antibodies (anti-HBc Ab) if compound having possible immune activities, anti-hepatitis C virus (anti-HCV2) antibodies, anti-human immunodeficiency virus 1 and 2 antibodies (anti-HIV1 and anti HIV2 Ab)

E 20. Positive results on urine drug screen (amphetamines / methamphetamines, barbiturates, benzodiazepines, cannabinoids, cocaine, opiates)

E 21. Positive alcohol test

Specific to the study

E 22. Subjects tested positive for antibodies against GLP-1 agonists (e.g. exenatide, lixisenatide) at screening

E 23. Known hypersensitivity to GLP-1 analogues (e.g. exenatide), insulin glargine and exipients

E 24. History of unexplained pancreatitis, chronic pancreatitis and/or pancreatectomy

E 25. Clinically relevant known gastroparesis

E 26. Personal history or family history of medullary thyroid cancer or a genetic condition that predisposes to medullary thyroid cancer

E 27. Any history or presence of deep leg vein thrombosis or a frequent appearance of deep leg vein thrombosis in 1st degree relatives (parents, siblings or children)

8 TREATMENTS

8.1 INVESTIGATIONAL PRODUCT

Lixisenatide (product code: AVE0010)

Lixisenatide will be used as two different formulations:

- Solution for injection containing 800 $\mu$g/mL lixisenatide in Lantus U100 (pre-mix)
- Solution for injection containing 100 $\mu$g/mL lixisenatide
- Lixisenatide dose: 20 $\mu$g
- Container: 3 ml glass cartridges

Insulin glargine

Insulin glargine will be used as two different formulations:

- Lantus U100 solution for injection containing 100 U/mL insulin glargine (marketed product)
- Solution for injection containing 800 $\mu$g/mL lixisenatide in Lantus U100 (pre-mix)
- Insulin glargine dose: 0.6 U/kg
- Container: 3 mL glass cartridges

Route of application: Subcutaneously

Conditions: Fasted

Duration of treatment: 1 day at each period, single dose

Start: 12:00 on Day 1 (D1) in Treatment Periods 1 and 2 (TP1/2)

Additional treatments for 100% of included subjects will be provided

Dispensing materials should be kept by the Investigator up to the full documented reconciliation performed with the Sponsor at the end of the study. The Investigator should wait for the written approval of the Sponsor before proceeding with their destruction.

Preparation of Formulations

The present study will assess the relative bioavailability and activity of 0.6 U/kg insulin glargine and 20 $\mu$g lixisenatide given as two different treatments

*Table 74 - Treatments*

| | Reference (R) | | Test (T) | |
|---|---|---|---|---|
| | separate simultaneous injections of insulin glargine (Lantus® U100) and lixisenatide (100 µg/mL) at opposite peri-umbilical sites | | injection of an on-site mix of insulin glargine (Lantus® U100) and lixisenatide (800 µg/mL in Lantus® U100) at one peri-umbilical site | |
| Compound | Lixisenatide | Lantus U100 | Lixisenatide | Lantus U100 |
| Dose | 20 µg | 0.6 U/kg | 20 µg | 0.6 U/kg |
| Form | solution for injection containing 100 µg/mL lixisenatide[a] | solution for injection containing 100 U/mL insulin glargine [b] | on-site mixed [d] lixisenatide and insulin glargine using a pre-mix[c] (800 µg/mL lixisenatide in Lantus U100) diluted in Lantus U100 | |

a Lixisenatide (100 µg/mL) solution for injection will be provided by Sanofi-Aventis
b Lantus U100 solution for injection is commercially available and will be purchased through CRO
c Pre-mix solution (800 µg/mL lixisenatide in Lantus U100) will be provided by Sanofi-Aventis
d On-site mix consisting of a fixed volume of the insulin glargine/lixisenatide pre-mix solution diluted with a variable volume of Lantus U100 will be prepared by the CRO

As the individual insulin glargine dose is determined by body weight while the lixisenatide dose is fixed, for the on-site mix (Test, lixisenatide/insulin glargine pre-mix solution diluted in Lantus U100), a fixed volume of the pre-mix will be diluted with a variable volume of Lantus U100 solution, depending on subject's body weight

*Table 75 - Dosing and On-site mix preparation*

| Weight (kg) | Lantus dose (U) | Lantus U100 volume (µL) | Lixisenatide pre-mix[a] volume (µL) | On-site mix volume (µL)[b] | Lixisenatide conc. (µg/U) | Lixisenatide conc. (µg/mL) |
|---|---|---|---|---|---|---|
| 50 | 30 | 275 | 25 | 300 | 0.67 | 66.67 |
| 55 | 33 | 305 | 25 | 330 | 0.61 | 60.6 |
| 60 | 36 | 335 | 25 | 360 | 0.56 | 55.56 |
| 65 | 39 | 365 | 25 | 390 | 0.51 | 51.28 |
| 70 | 42 | 395 | 25 | 420 | 0.48 | 47.62 |
| 75 | 45 | 425 | 25 | 450 | 0.44 | 44.44 |
| 80 | 48 | 455 | 25 | 480 | 0.42 | 41.67 |
| 85 | 51 | 485 | 25 | 510 | 0.39 | 39.22 |
| 90 | 54 | 515 | 25 | 540 | 0.37 | 37.04 |
| 95 | 57 | 545 | 25 | 570 | 0.35 | 35.09 |
| 100 | 60 | 575 | 25 | 600 | 0.33 | 33.33 |

e lixisenatide 800 µg/mL in Lantus U100. On site mix = 25 µL of pre-mix (lixisenatide 800 µg/mL in Lantus U100) + Lantus U100 ([6 µL*bw kg] - 25 µL)

For the on-site mix, the pre-mix solution (lixisenatide 800 µg/mL in Lantus U100) will be provided by sanofi-aventis and Lantus U100 cartridges will be purchased through the CRO. The final individual on-site mix solutions shall be prepared by the CRO (Profil, Neuss), according to the pharmacy manual, on the day of dosing. Profil holds a license for IP manufacturing acc. to national law. For the on-site mix, there will be one vial per subject.

For preparation of individual doses of the on-site mix, a solution containing 4-fold of the calculated volume will be prepared in accordance with the pharmacy manual in order to ensure sufficient dose accuracy:

$$([24 \ (\mu L/kg)*body \ weight \ (kg)] - 100 \ \mu L) \ of \ Lantus^® \ U100 +$$

will be drawn and transferred into sterile glass vial with the mean of syringes.

The pre-mix (lixisenatide in Lantus® U100) will be added to Lantus® U100 to prevent adhesion of lixisenatide. The mixture is shaken to establish a homogenous solution before the individual dosing volume [6 (μL/kg) * body weight (kg)] is drawn for injection.

Dosing

This is a single dose study with in total 2 administrations of study medication, comprising in total 3 injections, such that subjects will be exposed to treatment 2 times. Subjects will be randomized (1:1) to sequences R T, T R such that each subject receives the reference treatment (R, separate simultaneous injection) and the test treatment (T, on-site mix).

Injections will be given left or right of the umbilicus, with both sites being used for separate simultaneous injections. A washout period of 5 to 18 days will separate consecutive dosing days, the preference will be 7 days (7 days between consecutive dosings). The length of the wash-out period may vary individually allowing both the participant and the investigator to adjust to their needs. By experience, 5 days comprise a minimum period for recovery enabling 1 clamp per week for a participant, while 18 days represent a break of 3 weeks between dosing days, allowing subjects the freedom to fulfill non-study related obligations, if unavoidable.

Under fasting conditions subjects will receive single s.c. injections of 0.6 U/kg Lantus and 20 μg lixisenatide separately simultaneously as reference medication R at opposite peri-umbilical sites within 1 minute, or an injection of an on-site mix formulation as test medications T at one peri-umbilical site.

Timing for administration is at around 12:00 h on Day 1 in Treatment Periods 1 and 2.

IP administration may be postponed for up to 2 h in case the target glucose level has not been met 4 hours after start of the run-in phase (pre-clamp).

If the target glucose level cannot be established within 6 hours after start of the run-in phase, the visit will be terminated and the subject may be scheduled for a new dosing visit 1 - 7 days later.

Calculation of Dose of IP (insulin glargine)

To calculate the amount of Lantus given for each subject (0.6 U/kg), the body weight (in kg) will be determined to one decimal place and the amount of insulin calculated will be rounded up or down to integer numbers as shown in the following examples:

a subject with a body weight of 75.3 kg will receive 45 U insulin (75.3 x 0.6 = 45.18 which is rounded down to 45); a subject with a body weight of 74.4 kg will receive 45 U insulin (74.4 x 0.6 = 44.64, which is rounded up to 45).

The body weight recorded during TP1 D1 will be used for calculation of study medication dose for all treatment periods. The study medication dose will not be changed if a subject's weight changes by less than or equal to 2 kg between TP 1 and TP2. If a subject's body weight changes by more than 2 kg between TP 1 and TP2, the study medication dose will be recalculated based on the weight at TP2/D1.

8.2 SYRINGES AND NEEDLES

The following syringes with needles attached will be used to administer IP: Becton Dickinson, Ref 305502, Dimensions: 1 ML 27G 3/8 0.40x10. The syringes will be supplied by the investigator.

8.3 OTHER PRODUCTS

Other products used during the clamp procedure are described in

*Table 76 - Preparation of infusion*

| Drug Code | INN | Formulation | Manufacturer | Dose/Routeof administration |
|-----------|-----|-------------|--------------|------------------------------|
| Glucose | Glucose | 20 % solution for infusion | Certified, selected by PROFIL | iv infusion |
| Intramed Heparin Sodium | Heparin | Vial containing 5 mL solution (5000 IU/mL) | Certified, selected by PROFIL | iv infusion |

(continued)

| Drug Code | INN | Formulation | Manufacturer | Dose/Routeof administration |
|---|---|---|---|---|
| 0.9 % Sodium Chloride | Sodium Chloride | Solution | Certified, selected by PROFIL | iv infusion |
| Apidra | Insulin glulisine | 100 U/mL for injection | sanofi-aventis | iv infusion |

Glucose solution, sodium chloride solution and heparin will be provided by the Investigator.

Glucose solution: 20 % glucose solution will be infused with the Biostator to keep subjects individual blood glucose at the determined target level. A second infusion pump (part of the Biostator) will deliver 0.9 % sodium chloride solution to keep the line patent. In case the amount of 20 % glucose solution needed exceeds the infusion capacity of the Biostator, a second glucose infusion pump will be engaged.

Pumps are validated once per year and the validation documents are kept in a central file on site.

Heparin: A low dose heparin solution (10.000 Units heparine/100 mL saline) will be infused via a double lumen catheter. The heparin solution will be taken up together with blood used for the Biostator's blood glucose measurement in the other lumen of the catheter and is aimed to prevent blood clotting in the system.

Insulin glulisine: 15 U Apidra [100 U/ml] will be given to 49 mL of saline solution, to which 1 mL of the subject's own blood is added to prevent adhesion, producing a concentration of 0.3 U/mL, which will be infused at an individual rate to achieve euglycemia.

8.4 DESCRIPTION OF BLINDING METHODS

Not applicable.

8.5 METHOD OF ASSIGNING SUBJECTS TO TREATMENT GROUP

IPs will be administered according to the Clinical Study Protocol only to subjects who have given written informed consent.

Subjects who comply with all inclusion/exclusion criteria will be assigned an incremental subject number according to the chronological order of inclusion on the morning of D1. The 9 digit subject number consisting of 3 components (276 001 001, 276 001 002, 276 001 003, etc.), of which the first 3 digits (276) are the country number, the middle 3 digits are the site number and the last 3 digits are the subject incremental number within the site. The subject number remains unchanged and allows the subject to be identified during the whole study.

IP administration will be in accordance with the randomization on the treatment sequence.

The randomized treatment kit number list is generated. Subjects who comply with all inclusion/exclusion criteria will be assigned a treatment number in a pre-planned order following a randomized treatment kit number list:

- The next eligible subject will always receive the next treatment number according to the randomization list
- Additional subjects will have a different identification number (i.e., 500 + the number of the subject who discontinued the study). Each subject will receive the same treatment and treatment sequence as the subject, who discontinued the trial
- Screen Failed subjects: e.g., 901, 902 (to be recorded in the CRF only in case of AE occurring during screening period after signing of informed consent)

Subjects withdrawn from the study retain their subject number and their treatment number, if already assigned. New subjects must always be allotted a new subject number and, if applicable, a new treatment number.

Notes: The randomization of a subject will occur after Investigators confirmation of subjects eligibility for this study. Baseline parameters will be the parameters available the closest before the randomization.

8.6 PACKAGING AND LABELING

IP will be provided in 3 mL cartridges (lixisenatide/Lantus pre-mix solution and lixisenatide 100 µg/mL solution). 10 cartridges of lixisenatide 100 µg/mL will be gathered in a regrouping box. 10 cartridges of Lantus pre-mix and lixisenatide 100 µg/mL will be gathered in a regrouping box. The respective number of IP will be packaged under the responsibility of Sanofi-Aventis according to good manufacturing practice and local regulatory requirement and provided to CRO. The content of the labeling is in accordance with the local regulatory specifications and requirements. Lantus U100 is commercially available and will be ordered by the CRO. The on-site mix will be prepared and labeled by the CRO on the day of dosing.

8.7 STORAGE CONDITIONS

All IP will be stored in an appropriate locked room under the responsibility of the Investigator, and must be accessible only to authorized personnel. The IP has to be stored at +2°C to +8°C, protected from light, and must not be frozen.

8.8 ACCESS TO THE RANDOMIZATION CODE DURING THE STUDY

Not applicable.

8.9 RESPONSIBILITIES

The Investigator, the clinical site pharmacist, or other personnel allowed to store and dispense IP will be responsible for ensuring that the IP used in the study is securely maintained as specified by the Sponsor and in accordance with the applicable regulatory requirements.
The clinical site pharmacist at the CRO (Profil) is responsible for the manufacturing of the on-site mix solution for injection (see
Table 75) according to the process described in the Clinical Trial Protocol and pharmacy manual. Profil is further responsible to apply for and maintain during the course of the study a manufacturing license according to national law, and fulfill all necessary requirements for manufacturing of IP.
All IP shall be dispensed in accordance with the Clinical Trial Protocol and Investigator's prescription and it is the Investigator's responsibility to ensure that an accurate record of IP issued and returned is maintained.

8.10 RETRIEVAL OF TREATMENTS AND/OR DESTRUCTION

A detailed treatment log of the returned or destroyed IP will be established with the Investigator (or the pharmacist) and countersigned by the Investigator and the Monitoring Team.

8.11 CONCOMITANT TREATMENT

The use of concomitant medication is not allowed after screening and signing Informed Consent Form until EOS with the exception of drugs mentioned under Exclusion Criteria 0 (Section 7.3).
Participants will have to abstain from using basal insulins and switch to short-acting insulins from

- 48 hours prior to dosing at D1 of TP1 and TP2, if on long-acting insulin products, i.e. Lantus (insulin glargine), Levemir (detemir) or ultralente insulins,
- 24 hours prior to dosing at D1 of TP1 and TP2 if on intermediate acting insulin products, i.e. NPH-insulin

Thereafter the blood glucose levels will be controlled solely by subcutaneous injection of the usual short-acting insulin prescribed by the subjects' treating physician. The last subcutaneous injection of short-acting insulin will be no later than 9 hours before study drug administration. Subjects on pump therapy may remain on their basal infusion rate until 06:00 on D1.
For symptomatic adverse events which are not jeopardizing the subjects's safety (e.g. headache) concomitant medication should be reserved for adverse events of severe intensity or of moderate intensity which persist for a long duration. In particular, the use of acetaminophen/paracematol is prohibited if there is a known risk of hepatotoxicity, or as soon as abnormalities of liver enzymes occur.
However, if a specific treatment is required for any reason, an accurate record must be kept on the appropriate record form, including the name of the medication (international nonproprietary name), daily dosage and duration for such use. The Sponsor must be informed within 48 h via e-mail or fax, with the exception of treatment of headache.
For oral treatments that are dependent on threshold concentrations for efficacy, such as contraceptives (pill), patients should be advised to take those treatments at least 1 hour before investigational product injection or about 11 hours after investigational product injection.
Treatment of potential allergic reactions will be in compliance with the recommendations as published elsewhere (reference 2). Dependent on the severity of the allergic reaction treatment with antihistamins, corticosteroids and epinephrine may be considered.

The subjects will not take any non-trial medication, which will interfere with the metabolic control or the insulin sensitivity of subjects throughout the study and in the two weeks before the study.

## 8.12 TREATMENT ACCOUNTABILITY AND COMPLIANCE

- IP compliance:

  - IP will be administered under direct medical supervision, and an appropriate record will be completed by the Investigator or his/her delegate
  - IP intake will be confirmed by measurable plasma/serum drug assay results

- IP accountability:

  - The Investigator counts the number of cartridges and vials remaining in the returned packs, then fills in the Treatment Log Form
  - The Investigator records the dosing information on the appropriate page(s) of the Case Report Form (CRF)
  - The Monitor Team in charge of the study then checks the CRF data by comparing them with the IP and appropriate accountability forms

Used cartridges and vials should be kept by the Investigator up to the fully documented reconciliation performed with the Sponsor at the end of the study.

## 9 ASSESSMENT OF INVESTIGATIONAL PRODUCT

The present study is designed to assess the relative bioavailability (exposure) and activity (glucose disposition) as well as the safety and clinical and biological tolerability of 0.6 U/kg insulin glargine and 20 $\mu$g lixisenatide given as on-site mix of lixisenatide (800 $\mu$g/mL lixisenatide in Lantus® U100 diluted in Lantus® U100) compared to lixisenatide (100 $\mu$g/mL) and Lantus® U100 given separately simultaneously in an euglycemic clamp setting in subjects with diabetes mellitus type 1.

## 9.1 PHARMACODYNAMICS

### 9.1.1 Euglycaemic Clamp

The pharmacodynamic effect of insulin glargine, mainly the duration of insulin action will be evaluated by the euglycemic clamp technique.

During the euglycemic clamp the blood glucose concentration, the glucose infusion rate (GIR) and the amount needed to keep a subject's blood glucose concentration at its target level will be continuously measured and recorded using the Biostator™ device (continuous glucose monitoring system, Life Sciences Instruments, Elkhart, IN, USA).

The amount of glucose required is a measure of insulin mediated glucose uptake into tissues (glucose disposal or glucose lowering activity). The Biostator™ determines blood glucose levels in 1 min intervals and adjusts the glucose infusion rate in response to changes in blood glucose using a predefined algorithm.

During the clamp arterialized venous blood glucose concentration, which reflects the supply for total glucose utilization of all tissues, as well as glucose infusion rates will be continuously monitored.

Venous blood samples will be taken for determination of plasma lixisenatide and insulin glargine concentration.

Clamp procedure

To prevent interference of subjects standard insulin treatment with the clamp measurement, subjects have to abstain from using basal insulins and switch to short-acting insulins from

- 48 hours prior to dosing at D1 of TP1 and TP2, if on long-acting insulin products, i.e. Lantus (insulin glargine), Levemir (detemir) or ultralente insulins,
- 24 hours prior to dosing at D1 of TP1 and TP2 if on intermediate acting insulin products, i.e. NPH-insulin

Thereafter the blood glucose levels will be controlled solely by subcutaneous injection of the usual short-acting insulin prescribed by the subjects' treating physician. The last subcutaneous injection of short-acting insulin will be no later than 9 hours before IP administration. Subjects on pump therapy may remain on their basal infusion rate until 06:00 on D1.

During Treatment Periods 1 and 2 (TP1, TP2), subjects are admitted to the clinic in the morning of D1 after an overnight fast of at least 10 h. In the morning of Day 1 the pre-clamp procedure starts and subjects are linked to the Biostator. Blood glucose concentration is adjusted to 4.4 - 6.6 mmol/L (80 - 120 mg/dL) and maintained within these limits by means of iv bolus-administrations of a rapid acting insulin analog (e.g. insulin glulisine) and subsequent individual infusions, with infusion of glucose as needed. Duration of the pre-clamp is 4 hours (approximately 08:00 until 12:00).

The subjects' blood glucose is to be adjusted 60 min before study medication administration to approximately 5.5 mmol/L (100 mg/dL), which is to be continuously maintained by means of iv infusion of glucose solution until clamp end. The insulin infusion is to be discontinued immediately prior to the administration of the study medication.

At time point 0 (TO on D1 in TP1 and TP2, around 12:00), subjects will receive reference or test medication (R, T, see Table 74) as assigned by randomization. Injections will be given left or right of the umbilicus, with both sites being used for separate simultaneous injections.

IP administration may be postponed for up to 2 h in case the target glucose level has not been met 4 hours after start of the run-in phase (pre-clamp). If the target glucose level cannot be established within 6 hours after start of the run-in phase, the visit will be terminated and the subject may be scheduled for a new dosing visit 1 - 7 days later.

The goal of any basal insulin supplementation is to add to or even to substitute endogenous insulin secretion between meals. In subjects without endogenous insulin secretion, as invited to participate in this study, exogenous insulin should provide for just the amount of insulin required to dispose hepatic glucose production. If perfectly matched, there is no need for extra glucose to compensate for excess insulin. The resulting glucose infusion rate approximates zero. Once insulin action ceases, blood glucose concentration rises. The times to onset of rise and to times blood glucose concentrations exceeding predefined thresholds can be read by the Biostator.

It is expected that the selected dose of 0.6 U/kg is above the average basal need which in turn will produce some glucose demand reflected in a sizeable GIR up to and even beyond 24 h.

The corresponding parameter indicative of the clamp performance, i.e. the precision for keeping blood glucose at baseline level, is the blood glucose variability over the clamp period. A measure for blood glucose variability is the coefficient of variation (CV%) per individual clamp.

A low coefficient of variation in blood glucose over 24 h is prerequisite to properly assess the insulin effect in clamp settings.

The clamp period is not to exceed 24 h post study medication injection, the predefined clamp end Subjects are to continue fasting during the whole glucose clamp (pre-clamp and clamp) period while having access to water ad libitum.

In case blood glucose passes 11.1 mmol/L (200 mg/dL) prior to 24 h for 30 minutes after cessation of glucose infusion and the investigator confirms that any possible errors leading to false blood glucose levels above 11.1 mmol/L (200 mg/dL) have been excluded, the rapid acting insulin analog (e.g. insulin glulisine) used in the pre-IP administration time of the clamp will be given to extend the observation period to 24 h for pharmacokinetic blood sampling. In that case, the sponsor has to be informed.

The subjects will be delinked from the clamp setting when blood glucose is well within the isoglycemic range.

Participants will resume their pre-study medication on the day of discharge at TP1 to TP2, i.e. Day 2. The TEAE observation period will be from dosing on Day 1 to 72 hours later, at TP1 to TP2.

The effect of the IPs is to last about 24 h, which is why the participants will be confined to the institute for 2 days.

A washout period of 5 to 18 days will separate consecutive clamp period days, the preference will be 7 days (7 days between consecutive dosings). The length of the wash-out period may vary individually allowing both the participant and the investigator to adjust to their needs. By experience, 5 days comprise a minimum period for recovery enabling 1 clamp per week for a participant, while 18 days represent a break of 3 weeks between dosing days, allowing subjects the freedom to fulfill non-study related obligations, if unavoidable.

Screening and D1 of TP1 should not be separated by more than 28 days, while the EOS should occur no earlier than D5 of TP2, or no later than D9 of TP2, respectively.

9.1.2 Pharmacodynamic Sampling Times

Arterialized venous blood is to be continuously drawn at a rate of 2 mL/h for determination of arterial blood glucose concentration every minute from 4 hours (maximum 6 hours) prior to IP administration (pre-clamp) up to 24 h after medication (clamp).

Arterialized venous blood samples (0.2 mL) for concurrent Biostator calibration, which is a technical requirement, will be collected at least in 30 minute intervals after connection to the Biostator up to 24 hours after medication.

9.1.3 Number of Pharmacodynamic Samples

Blood glucose will be continuously measured during the clamp procedure. In addition, 52 samples per subject and treatment period will be collected for calibration of the Biostator. In total 52*2*22 samples or 2288 samples will be collected (see table below).

*Table 77 - Number of blood samples and aliquots per subject*

| Periods | Glucose [a] | Glucose [b] |
| --- | --- | --- |
| TP1 | Continuously | 52 |
| TP2 | Continuously | 52 |
| Total number of samples per subject | Continuously | 104 |
| Total number of samples [c] | Continuously | 2288 |

[a] continuous glucose monitoring at 2 mL/h for a maximum of 30hrs
[b] calibration
[c] assuming 22 subjects completed the study; number could be smaller due to drop outs

9.1.4 PD Handling Procedure

*Table 78 - Sample Handling Procedures*

| Analyte | Glucose |
| --- | --- |
| Blood Sample Volume | 200$\mu$L |
| Handling Procedures | Blood to be filled into capillary and then into sample cup for immediate analysis |

9.1.5 PD Parameters

The area under the body weight standardized GIR within 24 h (GIR-AUC$_{0-24}$) and the time to 50% of the total GIR-AUC within 24 h (T50%-GIR-AUC$_{0-24}$) will be calculated. In addition, the maximum GIR (GIR$_{max}$) and the time to GIR$_{max}$, GIR-T$_{max}$, will be assessed.
Further supplemental parameters might be derived as appropriate.

9.2 SAFETY

9.2.1 Baseline Demographic characteristics

The baseline demographic characteristics will consist of:

- Age (years)
- Body weight (kg)
- Height (cm)
- Body Mass Index (BMI) (kg/m$^2$)

9.2.2 Safety Assessment at Baseline and during the study

- Physical examination at screening: cardiovascular system, chest and lungs, thyroid, abdomen, nervous system, skin and mucosae, and musculo-skeletal system and relevant medical and surgical history, diabetes history (diagnosis of diabetes, onset of insulin treatment, late complications); only findings relevant to the study are to be documented, past and current smoking status
- Physical examination at pre-dose and during the study: cardiovascular system, abdomen and lungs; only findings relevant to the study are to be documented
- Body temperature (aural)

- Vital signs: Heart rate, respiratory rate and systolic and diastolic blood pressure measured after 10 minutes in supine resting position, heart rate and systolic and diastolic blood pressure-also after 3 minutes in standing position (except for unscheduled measurements when connected to Biostator)
- Laboratory tests (in fasted conditions for blood samples):
- Hematology: Red blood cell count (RBC), hematocrit (Hct), hemoglobin (Hb), white blood cell count (WBC) with differential (neutrophils, eosinophils, basophils, monocytes and lymphocytes), platelets, INR and aPTT
- Biochemistry:

  - Plasma electrolytes: Sodium, potassium, bicarbonate, chloride, calcium
  - Liver function: AST, ALT, alkaline phosphatase, gamma-glutamyl transferase (yGT), total and conjugated bilirubin
  - Renal function: creatinine, BUN
  - Metabolism: Glucose, albumin, total proteins, total cholesterol, triglycerides, HbA1c (at screening, D1 TP1 and EOS), LDH, amylase, lipase
  - Potential muscle toxicity: Creatinine phosphokinase (CPK)
  - Serology: Hepatitis B antigen (HBs Ag), anti-hepatitis B core antibodies (anti-HBc Ab), anti-hepatitis C antibodies (anti-HCV2), anti-HIV1 and anti-HIV2 antibodies

- Archival blood sample: a 15 mL blood sample will be collected into a dry, red topped tube, centrifuged at approximately 1500 g for 10 minutes at 4°C; the serum will then be transferred into three storage tubes, which will be immediately capped and frozen in an upright position at -20°C. This sample will be used if any unexpected safety issue occurs to ensure that a pre drug baseline value is available for previously non-assessed parameters (e.g., serology). If this sample is not used, the Investigator will destroy it after the Sponsor's approval
- Urinalysis: Proteins, glucose, blood, ketone bodies, pH

  - Qualitative: A dipstick is to be performed on a freshly voided specimen for qualitative detection using a reagent strip;
  - Quantitative: A quantitative measurement for glucose, protein, erythrocytes and leucocytes count will be required in the event that the urine sample test is positive for any of the above parameters by urine dipstick (e.g., to confirm any positive dipstick parameter by a quantitative measurement).

- Urine drug screen: Amphetamines/metamphetamines, barbiturates, benzodiazepines, cannabinoids, cocaine, opiates
- Alcohol breath test
- Pregnancy/hormone test (if female): Plasma β-HCG at screening, at TP1 and TP2 urine β HCG only; plasma-FSH/estradiol if postmenopausal less than 2 years and at screening only
- Adverse Events: Spontaneously reported by the subject or observed by the Investigator. As lixisenatide may cause nausea and vomiting, particular attention will be given to gastrointestinal symptoms
- ECG telemetry (single lead)
- 12-lead ECG (automatic)
- Anti-lixisenatide antibodies: Anti-lixisenatide antibodies will be determined at baseline of each trial period (D1), and a final blood sample will be drawn after an additional 4-6 weeks at the post-study visit (PSV). If a subject is tested positive for anti-lixisenatide antibodies during post study visit, a follow-up sample will be taken within a 3 to 6 month period after post study visit. For subjects having received GLP-1 agonist treatment before, anti-lixisenatide antibodies test will be performed at the screening visit
- Blood samples for laboratory tests for should be taken under fasted conditions.

ECG Methodology

ECG telemetry

- ECG telemetry will be continuously monitored by medical personnel. All arrhythmic events will be documented by printing and included in the subject's CRF. This documentation must allow for diagnosis of the event, time of occurrence, and duration, and will be signed by the Investigator or delegate. The ECG telemetry records must be kept for a potential re-analyze taking account the Investigational Product exposure.

Twelve-lead ECGs

- Twelve-lead ECGs will be recorded after at least 10 minutes in supine position using an electrocardiographic device (MAC 5500). The electrodes will be positioned at the same place for each ECG recording throughout the study (attachment sites of the leads will be marked with an indelible pen).
- ECGs should always be recorded before the PK sampling (if any). PK samples need to be drawn as soon as possible (within 15 minutes) after ECG.
- Each ECG consists of a 10 second recording of the 12 leads simultaneously, leading to:

  - a single 12-lead ECG (25 mm/s, 10mm/mV) print-out with HR, PR, QRS, QT, QTc automatic correction evaluation, including date, time, initials and number of the subject, signature of the investigator, and at least 3 complexes for each lead. The Investigator medical opinion and automatic values will be recorded in the CRF. This print-out will be retained at the site level
  - a digital storage that enables eventual further reading by an ECG central lab: each digital file will be identified by theoretical time (day and time DxxTxxHxx), real date and real time (recorder time), Sponsor study code, subject number (i.e., 3 digits) and site and country numbers if relevant.

- The digital recording, data storage and transmission (whenever requested) need to comply with all the applicable regulatory requirements (i.e., FDA 21 CFR, part 11).

Warning: when vital signs, ECG, and blood samples are scheduled at the same time as an Investigational Product administration and/or a meal, they should be done prior to Investigational Product administration and/or meal. Whenever measurements of vital signs, ECG, and blood samples for PK, PD, or safety coincide, the following order will be respected: ECG, vital signs, PD, PK, and safety samples; in order to respect exact timing of PK samples (refer to flow-chart for time window allowance for PK samples), the other measures will be done ahead of the scheduled time. The assessment schedule should be adapted to the design of the study

### 9.2.3 Anti-Lixisenatide Antibodies

Anti-lixisenatide antibodies will be determined during the study.

*Table 79 - Number of blood samples and aliquots per subject*

| Periods | Anti-lixisenatide antibodies / sample ID |
|---|---|
| Screening [c] | 1/ PEY00 |
| TP1 | 1 / PE00 |
| TP2 | 1 / PE01 |
| Post study visit | 1 / PE02 |
| Follow up visit [c] | 1 / PE03 |
| Total number of samples / subject | 3-5 |
| Total number of samples [a] | 3-5*22 = 66 (min) -110 (max) [b] |

[a] Assuming 22 subjects completed the study; number could be smaller due to drop outs
[b] For subjects having treated with lixisenatide before, an additional sample will also be drawn at screening; a follow-up sample will be taken 3 to 6 months after post study visit (PSV) for subjects tested positive for anti-lixisenatide antibodies at PSV
[c] not required for every subject

*Table 80 - Anti-Lixisenatide antibody - Sample Handling Procedures*

| Analyte | Anti-Lixisenatide antibodies |
|---|---|
| Blood Sample Volume | 3mL |
| Anticoagulant Tube Type | $K_2$-EDTA |
| Handling Procedures | Blood storage until centrifugation: +4°C |

(continued)

| Analyte | Anti-Lixisenatide antibodies |
|---------|------------------------------|
| Centrifuge Conditions | Within 1 hour of collection, at 1500 g for 10 min at +4°C |
| Plasma Aliquot Split [a] | 1 mL + remaining |
| Plasma Storage Conditions | -20°C |
| Plasma Shipment Conditions | Dry ice |

[a] Plasma samples will be split in two aliquots with abundant volume in the first aliquot to allow for multiple analyses. The first aliquot with a volume as specified will be sent to the bioanalytical laboratory, the second aliquot will remain at the site.

*Table 81 - Bioanalytical Method*

| Analyte | Anti- Lixisenatide antibodies |
|---------|-------------------------------|
| Matrix | Plasma |
| Analytical Technique | BIAcore |
| Lower Limit of Quantification | cut-off |
| Assay Range | not relevant |
| Assay Volume | 100 $\mu$L |
| Site of Bioanalysis | Biomarker/Biologicals, Department of Global Metabolism and Pharmacokinetics, sanofi-aventis, Frankfurt |
| Method Reference | RPSMPK-DOH0754-BM1-EN-E01 |

9.2.4 Local tolerability at injection site

The evaluation of injection site reaction following IP injection will be standardized according to Section 14 (Evaluation of skin response). Findings at the site of injection (such as erythema, edema, papules, induration, vesicles, blisters) will be graded mainly according to a Global Irritation Score. A local injection site reaction with a score of $\geq 3$ according to the rating scale will be documented additionally as an adverse event. The subjects are asked to report sensations at the injection site.

9.3 PHARMACOKINETIKS

For the assessment of lixisenatide pharmacokinetics, the area under the plasma lixisenatide concentration curve (AUC) as $AUC_{last}$ and AUC, apparent clearance (CL/F), apparent volume of distribution (Vz/F), and terminal half life $t_1/_{2\lambda z}$ will be derived, and peak concentration $C_{max}$, and time to $C_{max}$ ($T_{max}$) will be observed.
For the assessment of insulin glargine pharmacokinetics, the area under the serum insulin concentration curve (AUC) up to 24 hours, $AUC_{0-24}$ and the time to 50% of $AUC_{0-24}$ will be derived. In addition, $C_{max}$ and time to $C_{max}$ ($T_{max}$) will be observed.

9.3.1 Sampling Times

Blood is to be collected for the determination of plasma lixisenatide concentrations at time points 0H and 0H15 0H30, 1 H, 1H30, 2H, 2H30, 3H, 4H, 5H, 6H, 8H, 12H and 24H after injection of study medication.
Blood is to be collected for the determination of serum insulin glargine concentrations at time points 0H and 0H15, 0H30, 1H, 1H30, 2H, 4H, 6H, 8H, 10H, 12H, 14H, 16H, 18H, 20H, 22H and 24H after injection of study medication.
The sampling times for blood collection can be found in the Period Flow Chart (Section 1.2).

9.3.2 Number of Pharmacokinetic Samples

Lixisenatide: 14 blood samples per subject (N=22) and treatment period (2) will be collected, in total 616 samples (see Table 82).
Insulin Glargine: 17 blood samples per subject (N=22) and treatment period (2) will be collected, in total 748 samples (see Table 82).
In total 31*2*22 = 1364 samples will be collected.

*Table 82 - Number of blood samples per subject*

| Periods | Lixisenatide | Insulin (glargine) |
|---|---|---|
| Period 1 | 14 | 17 |
| Period 2 | 14 | 17 |
| Total number of samples per subject | 28 | 34 |
| Total number of samples [a] | 28*22=616 | 34*22=748 |

[a] assuming 22 subjects completed the study; number could be smaller due to drop outs

9.3.3 PK Handlung procedure

The exact time of IP administration and sample collection must be recorded in CRF.

*Table 83 - Sample Handling Procedures*

| Analyte | Insulin | Lixisenatide |
|---|---|---|
| Blood Sample Volume | 3mL | 3 mL |
| Anticoagulant Tube Type | Vacutainer PET blood collection tubes containing no additives | $K_2$-EDTA |
| Handling Procedures | Blood storage until centrifugation: at room temperature (allow clotting for 30 min, but not exceeding 1 hour) | Blood storage until centrifugation: +4°C |
| Centrifuge Conditions: | Within 45 min of collection, at 2000 g for 10 min at +4°C | Within 1 hours of collection, at 1500 g for 10 min at +4°C |
| Plasma/Serum Aliquot Split [a] | 1 mL + remaining | 1 mL + remaining |
| Storage Conditions | -20°C (freeze aliquots immediately) | -20°C |
| Shipment Conditions | Dry ice | Dry ice |

[a] Plasma/serum samples will be split in two aliquots with abundant volume in the first aliquot to allow for multiple analyses. The first aliquot with a volume as specified will be sent to the bioanalytical laboratory, the second aliquot will remain at the site.

9.3.4 Bioanalytical Methods

*Table 84 - Bioanalytical Method*

| Analyte | Lixisenatide | Insulin |
|---|---|---|
| Matrix | Plasma | Serum |
| Analytical Technique | double-antibody sandwich ELISA | Radioimmunoassay |
| Lower Limit of Quantification | 12 pg/ml | 5.02 ($\mu$U/mL 0.18 ng/mL or 30 pmol/L |
| Assay Range | 12- 220 pg/mL | 5.02 - 150 $\mu$U/mL |
| Assay Volume | 100 $\mu$L | 300 $\mu$L |
| Site of Bioanalysis | Biomarker/Biologicals, Department of Global Metabolism and Pharmacokinetics, sanofi aventis, Frankfurt | Parexel, Bloemfontein;South Africa |
| Method Reference | VAA43648CH-IB-02 | VAL030/01 |

No analytical interference of insulin glargine on lixisenatide assay, or vice versa, was observed.

9.3.5 PK Parameters

The following pharmacokinetic parameters will be calculated, using non-compartmental methods for lixisenatide plasma and insulin glargine serum concentrations after single dose. The parameters will include, but may not be limited to the following.

Table 85 - List of pharmacokinetic parameters and definitions

| Paramet ers | Drug/Analyte | Definition/Calculation |
|---|---|---|
| $C_{max}$ | Lixisenatide / insulin | Maximum plasma/serum concentration observed |
| $T_{max}$ | Lixisenatide / insulin | First time to reach $C_{max}$ |
| $AUC_{last}$ | Lixisenatide | Area under the plasma concentration versus time curve calculated using the trapezoidal method from time zero to the real time, $t_{last}$ (time corresponding to the last concentration above the limit of quantification, $C_{last}$) |
| $AUC_{0-24}$ | Insulin | Area under the serum concentration versus time curve calculated using the trapezoidal method from time zero to 24 hours post dosing |
| AUC | Lixisenatide | Area under the plasma concentration versus time curve extrapolated to infinity according to the following equation: $$AUC = AUC_{last} + \frac{C_{last}}{\lambda_z}$$ |
| CL/F | Lixisenatide | Apparent Total Body Clearance of a drug from the plasma calculated using the following equation: $$CL/F = \frac{Dose_{EV}}{AUC_{EV}}$$ |
| $V_z/F$ | Lixisenatide | Aparent Volume of Distribution during the terminal ($\lambda_z$) phase calculated using the following equation: $$V_z/F = \frac{CL/F}{\lambda_z}$$ |
| $t_{1/2Z}$ | Lixisenatide | Terminal half-life associated with the terminal slope ($\lambda z$) determined according to the following equation: $$t_{1/2Z} = \frac{0.693}{\lambda_z}$$ where $\lambda z$ is the slope of the regression line of the terminal phase of the plasma concentration versus time curve, in semi-logarithmic scale. Half-life is calculated by taking the regression of at least three points. |

9.4 SAMPLED BLOOD VOLUME

Table 86 - Sampled Blood Volume

| Type | Volume per Sample | Sample Number | Total |
|---|---|---|---|
| Serology | 2 mL | 1 | 2 mL |
| Hematology | 2.7 mL | 4 | 10.8 mL |
| Coagulation | 2 mL | 4 | 8 mL |
| Biochemistry | 5 mL | 4 | 20 mL |
| Archival Sample | 15 mL | 1 | 15 mL |
| Insulin | 3 mL | 34 | 102 mL |
| Lixisenatide | 3 mL | 28 | 84 mL |
| Lixisenatide antibody | 3 mL | 3 up to 5 [b,c] | 9 mL up to 15 mL |

(continued)

| Type | Volume per Sample | Sample Number | Total |
|---|---|---|---|
| Glucose calibration | 0.2 mL | 104 | 20.8 mL |
| Glucose continuously | 2 mL/h | Max. 52 | 104 mL |
| β-HCG (if female) [a] | 0 mL | 1 | 0 mL |
| plasma-FSH/estradiol (if female) [a,d] | 0 mL | 1 | 0 mL |
| Total | | 233 up to 235 | 375.6 mL |
| | | | 378.6 mL [b] |
| | | | 381.6 mL [c] |

[a] included in serology

[b] For subjects having treated with GLP-1 agonists before, an additional sample will be drawn at screening

[c] For subjects tested positive for anti-lixisenatide antibodies at post study visit an additional sample will be taken 3 to 6 month after PSV

[d] if postmenopausal less than 2 years

Table 87 - Additional sampled blood volume for repeated blood laboratory examinations

| Type | Volume per Sample | Sample Number | Total |
|---|---|---|---|
| Serology | 2 mL | 1 | 2 mL |
| Hematology + HbA1C | 2.7 mL | 1 | 5.4 mL |
| Coagulation | 2 mL | 1 | 4 mL |
| Biochemistry | 5 mL | 1 | 10 mL |
| In addition total | | 4 | 21,4 mL |

9.5 MEASURES TO PROTECT BLINDING OF THE TRIAL

Not applicable.

10 SUBJECT SAFETY

The Investigator is the primary person responsible for taking all clinically relevant decisions in case of safety issues. If judged necessary, the opinion of a Specialist should be envisaged in a timely manner (e.g. acute kidney failure, convulsions, skin rashes, angioedema, cardiac arrest, electrocardiographic modifications, etc).

10.1 ADVERSE EVENT MONITORING

All events will be managed and reported in compliance with all applicable regulations, and included in the final clinical study report.

10.2 DEFINITIONS OF ADVERSE EVENT (AE) AND SERIOUS ADVERSE EVENT (SAE)

An Adverse Event is any untoward medical occurrence in a subject administered a pharmaceutical product and which does not necessarily have to have a causal relationship with this treatment.

A Serious Adverse Event is any untoward medical occurrence that at any dose:

- Results in death or;
- Is life-threatening or;
  Note: The term "life-threatening" in the definition of "serious" refers to an event in which the subject was at risk of death at the time of the event; it does not refer to an event which hypothetically might have caused death if it were more severe.
- Requires inpatient hospitalization or prolongation of existing hospitalization or;
- Results in persistent or significant disability/incapacity or;
- Is a congenital anomaly/birth defect;

- Is a medically important event:

Medical and scientific judgment should be exercised in deciding whether expedited reporting is appropriate in other situations, such as important medical events that may not be immediately life-threatening or result in death or hospitalization but may jeopardize the subject or may require intervention to prevent one of the other outcomes listed in the definition above.

Note: Examples of such events are intensive treatment in an emergency room or at home for allergic bronchospasm, blood dyscrasias, convulsions, ALT > 3 ULN + total bilirubin > 2 ULN or asymptomatic ALT increase ≥ 10ULN that does not result in hospitalization, or development of drug dependency or drug abuse.

## 10.3 OBLIGATION OF THE INVESTIGATOR REGARDING SAFETY REPORTING

### 10.3.1 Adverse Events

All AEs regardless of seriousness or relationship to IP, spanning from the signature of the informed consent form until the end of the study, as defined by the protocol for that subject, are to be recorded on the corresponding page(s) or screen(s) included in the CRF.

Whenever possible, diagnosis or single syndrome should be reported instead of symptoms. The Investigator should specify the date of onset, intensity (see definitions below), action taken with respect to IP, corrective treatment/therapy given, additional investigations performed (e.g. in case of dermatologic lesions photographs are required), outcome and his/her opinion as to whether there is a reasonable possibility that the Adverse Event was caused by the IP.

Severity of an AE is assessed as:

- Mild = no modification of daily activities and does not require mandatory corrective/symptomatic treatment
- Moderate = hinders normal daily activities and/or requires mandatory corrective/symptomatic treatment
- Severe = prevents daily activities and requires mandatory corrective/symptomatic treatment

Laboratory, vital signs or ECG abnormalities are to be recorded as AEs only if:

- symptomatic, and/or
- requiring either corrective treatment or consultation, and/or
- leading to IP discontinuation or modification of dosing, and/or
- fulfilling a seriousness criterion, and/or
- defined as an AE with pre-specified monitoring (AEPM) with immediate notification.

### 10.3.2 Serious Adverse Events

In the case of a Serious Adverse Event the Investigator must immediately:

- SEND (within 1 working day, preferably by fax or e-mail) the signed and dated provided paper Case Report Form page(s) to the representative of the Monitoring Team whose name, fax number and e-mail address appear on the Clinical Trial Protocol.
- ATTACH the photocopy of all examinations carried out and the dates on which these examinations were performed. Care should be taken to ensure that the subject's identity is protected and the subject's identifiers in the Clinical Trial are properly mentioned on any copy of source document provided to the Sponsor. For laboratory results, include the laboratory normal ranges.
- ENTER the information related to the Serious Adverse Event in the appropriate screens of the e-CRF
- All further documentation as well as additional information (for laboratory data, concomitant medication, subject status) should be sent (by fax or e-mail) to the Monitoring Team within 1 working day of knowledge. In addition, any effort should be made to further document each Serious Adverse Event that is fatal or life threatening within the week (7 days) following initial notification.

### 10.3.3 Safety observations

The Investigator should take all appropriate measures to ensure the safety of the subjects, notably he/she should follow up the outcome of SAE /AEs with pre-specified monitoring until clinical recovery is complete and laboratory results have returned to normal or until progression has been stabilized or death. In all cases, this may imply that observations will continue beyond the last planned visit per protocol, and that additional investigations may be requested

# EP 2 898 895 A1

by the Monitoring Team up to as noticed by the sponsor.

When treatment is prematurely discontinued, the subject's observations will continue until the end of the study as defined by the protocol for that subject.

In case of any Serious Adverse Event/AEPM with immediate notification brought to the attention of the Investigator at any time after the clinical trial and considered by him/her to be caused by the Investigational Product with a reasonable possibility, this should be reported to the Monitoring Team.

10.3.4 Adverse events with pre-specified monitoring (AEPM)

Adverse events requiring pre-specified monitoring are AEs (serious or non-serious) that need to be monitored, documented, and managed in a pre-specified manner described in the protocol.

For each defined AEPM, consider carefully the need to collect additional specific information that would impact the study and/or the CRF design, such as:

- Pre-existing related condition or lifestyle of interest for the AE (e.g., habits, cardiovascular risk factor...),
- Expected list of associated signs and symptoms,
- Corrective actions (e.g., treatment discontinuation, concomitant treatment...),
- Diagnostic actions (e.g., test(s) or procedure(s) results...),
- Additional descriptive factors,
- Sequelae.

*10.3.4.1 AEPM with immediate notification*

For these AEs, the Sponsor will be informed immediately (i.e. within 1 working day), as per SAEs notification described in Section 0, even if not fulfilling a seriousness criterion, using the corresponding pages in the CRF (to be sent) or screens in the e-CRF.

- QTc ≥500 ms

    - In occurrences of prolongation of QTc automatic measurement ≥500 ms, confirmed by a manual reading by the Investigator, or a physician delegated by the Investigator, using the Fridericia formula for correcting QT, the subject should be placed under supervision in a specialized setting. Stop Investigational Product administration and appropriate blood samples will be collected. Subsequent ECG monitoring of the subject should then be performed on a regular and clinically responsible basis until the QTc interval returns to a safe value as determined by the Investigator in agreement with the Sponsor.

- Pregnancy

    - Pregnancy occurring in a female subject included in the clinical trial. Pregnancy will be recorded as an AE with pre-specified monitoring with immediate notification in all cases. It will be qualified as an SAE only if it fulfills the SAE criteria.
    - In the event of pregnancy, IP should be discontinued.
    - The follow-up of the pregnancy will be mandatory until the outcome has been determined.

- Symptomatic Overdose with IP

    - An overdose (accidental or intentional) with the IP is an event suspected by the Investigator or spontaneously notified by the subject (not based on systematic IP count) and defined as at least twice of the intended dose within the intended therapeutic interval, adjusted according to the tested drug.

- Pancreatitis and/or increase of pancreatic enzymes (amylase, lipase) 2 > ULN (see also Section 10.4.2)

*10.3.4.2 AEPM without immediate notification*

- Asymptomatic overdose with IP (see also section before)
- Local tolerability

- The evaluation of injection site will be standardized according to Section 14 (Evaluation of skin response). Findings (such as erythema, edema, papules, induration, vesicles, blisters) will be graded mainly according to a Global Irritation Score. A local injection site reaction with a score of ≥3 according to the rating scale will be documented additionally as an AE. Further, a dermatologist must be consulted if a score is > 3.

- Allergic or allergic-like reaction

  - In case a subject experiences an allergic reaction or an allergic-like reaction this has to be reported as an adverse event. Additional information is collected on specific allergic reaction forms. Allergic, or possible allergic reactions will be adjudicated by the Allergic Reaction Assessment Committee (ARAC, see Section 6.4.1).

10.3.5 Laboratory abnormalities with pre-specified monitoring

Laboratory abnormalities should be monitored, documented, and managed according to the related flowchart in appendices.

Laboratory abnormalities with pre-specified monitoring which are non study-specific:

- Neutropenia,
- Thrombocytopenia,
- Acute renal insufficiency,
- Suspicion of rhabdomyolysis.

10.4 SPECIFIC TO THE TRIAL

10.4.1 Stopping rules

IP administration may be postponed for up to 2 h in case the target glucose level has not been met 4 hours after start of the run-in phase (pre-clamp) on Day 1. If the target glucose level cannot be established within 6 hours after start of the run-in phase, the visit will be terminated and the subject may be scheduled for a new dosing visit 1 - 7 days later.

Subjects experiencing a confirmed allergic reaction which is considered closely related to the administration of IP by the investigator will be withdrawn from further treatment.

10.4.2 Monitoring of suspected pancreatitis

Because some cases of acute pancreatitis have been reported with the GLP-1 agonist exenatide (Byetta), subjects enrolled in this study should be followed for any suspected acute pancreatitis, ie, with symptoms and/or signs of acute abdominal distress.

In case of severe, persistent abdominal pain, which can radiate to the back, often with characteristic positional features, with possible occurrence of nausea, vomiting, fever and leucocytosis, further measurement of amylase and lipase should be performed. The diagnosis of pancreatitis may be supposed also if other causes of abdominal pain are excluded (i.e., gallbladder disease, etc) and elevated amylase/lipase is seen and in addition pancreatic changes are seen on ultrasound and/or CT or MRI (with contrast, as appropriate).

Amylase and lipase values greater than 2-fold ULN should be repeated within 7 days. Amylase and lipase values greater than 3-fold ULN should be repeated within 48 hours. If the value remains above 2-fold ULN, it should be repeated weekly until it is less than 2-fold ULN. Amylase and lipase elevations without associated clinical symptoms should receive a gastroenterologic evaluation with additional imaging, as appropriate. All the laboratory or clinical documentations should be collected. As soon as there are signs, symptoms and results of investigations exploring suspected pancreatitis (eg, laboratory results, imaging reports, gastroenterologist's evaluations, etc) related to suspected pancreatitis, the investigator must document and report them on a specific e-CRF form.

With any diagnosis of acute pancreatitis, the investigational treatment and other potentially suspect drugs should be stopped and the subject followed further clinically.

10.4.3 Local tolerability

All skin reactions at the site of IP injection are to be documented as an adverse event if a score of ≥3 is observed according to the scoring system described in (Section 14). Further, a dermatologist must be consulted if a score is > 3.

10.4.4 Allergic or allergic-like reaction

In case a subject experiences an allergic reaction or an allergic-like reaction this has to be reported as an adverse event. Additional information is collected on specific allergic reaction forms. Allergic, or possible allergic reactions will be adjudicated by the Allergic Reaction Assessment Committee (ARAC, see Section 6.4.1).

## 11. STATISTICAL CONSIDERATIONS

The material of Section 13 of the Clinical Trial Protocol is the basis for the Statistical Analysis Plan or a Statistical Technical Document for the study. This plan will be drafted before first enrollment and may be revised during the study to accommodate Clinical Trial Protocol amendments and to make changes to adapt to unexpected issues in study execution and data that affect planned analyses. These revisions will be based on review of the study and data, and a final plan will be issued prior to data lock.

## 11.1 DETERMINATION OF SAMPLE SIZE

The primary objective of the study is to assess the relative bioavailability for insulin glargine and lixisenatide given as on-site mix and separately simultaneously. Based on the statistical analysis of Examples 1 and 2, a value of approximately 0.300 can be expected for the $SD_{within}$ of $AUC_{last}$ of lixisenatide on the natural log-transformed scale, while that for insulin glargine is expected to be lower. For the purpose of the sample size calculation within-subject SDs between 0.25 and 0.35 were used.

Table 88 shows the maximum imprecision (in terms of the 90 % confidence interval) for the ratio of adjusted geometric means (onsite mix versus separately simultaneously) that will obtained with 90 % assurance, for total number of subject N between 16 and 20, assuming a true within-subject SD of values between 0.25 and 0.35 for log AUC.

*Table 88 - Maximum imprecision for any pairwise ratio*

| Confidence level: 90% Assurance: 90% | | | Maximum width 90% CI for an observed ratio equal to | | |
|---|---|---|---|---|---|
| Within-subject SD on log scale | Total number of subjects | Maximum imprecision (%) | 0.9 | 0.95 | 1 |
| 0.250 | 16 | 17.4 | (0.74;1.09) | (0.78;1.15) | (0.83;1.21) |
| | 18 | 16.2 | (0.75;1.07) | (0.80;1.13) | (0.84;1.19) |
| | 20 | 15.2 | (0.76;1.06) | (0.81;1.12) | (0.85;1.18) |
| 0.275 | 16 | 18.9 | (0.73;1.11) | (0.77;1.17) | (0.81;1.23) |
| | 18 | 17.6 | (0.74;1.09) | (0.78;1.15) | (0.82;1.21) |
| | 20 | 16.6 | (0.75;1.08) | (0.79;1.14) | (0.83;1.20) |
| 0.300 | 16 | 20.5 | (0.72;1.13) | (0.76;1.19) | (0.80;1.26) |
| | 18 | 19.1 | (0.73;1.11) | (0.77;1.17) | (0.81;1.24) |
| | 20 | 17.9 | (0.74;1.10) | (0.78;1.16) | (0.82;1.22) |
| 0.325 | 16 | 22.0 | (0.70;1.15) | (0.74;1.22) | (0.78;1.28) |
| | 18 | 20.5 | (0.72;1.13) | (0.76;1.19) | (0.79;1.26) |
| | 20 | 19.3 | (0.73;1.11) | (0.77;1.18) | (0.81;1.24) |
| 350 | 16 | 23.5 | (0.69;1.18) | (0.73;1.24) | (0.77;1.31) |
| | 18 | 21.9 | (0.70;1.15) | (0.74;1.22) | (0.78;1.28) |
| | 20 | 20.6 | (0.71;1.13) | (0.75;1.20) | (0.79;1.26) |

Imprecision is in terms of the relative distance (%) of the lower 90% confidence limit from the observed ratio. The distance of the upper 90% confidence limit from the observed ratio will be greater due to asymmetry. Study design: 2-sequence 2-treatment 2-period cross-over.

With 18 subjects, if the true within-subject SD is as much as 0.3, the treatment ratio will be estimated with a maximum imprecision of 19.1 % (i.e. the 90 % CI will be 0.81 and 1/0.81 = 1.24 times the observed ratio), with 90 % assurance. 22 subjects will be included in order to have 18 completed subjects.

11.2 SUBJECT DESCRIPTION

11.2.1 Disposition of subjects

A detailed summary of subject accountability including count of subjects included, randomized, exposed (i.e. received any amount of study medication), completed (i.e. subjects who completed all study treatment periods), discontinued along with the main reasons for discontinuation will be generated for each sequence and for all subjects in total.
Subject disposition at the final visit will be presented in a listing including sequence group, disposition status at the end of the study with the date of last administration of study drug, date of final visit, reason for discontinuation. All withdrawals from the study, taking place on or after the start of the first study drug administration, will be fully documented in the body of the clinical study report (CSR).

11.2.2 Protocol deviations

Prior to data lock of the study, Clinical Trial Protocol deviations will be examined relative to criteria defined for definition of populations (see Section 0) and other study criteria including:

- Inclusion and exclusion criteria;
- Treatment compliance;
- Compliance with the Clinical Trial Protocol with regard to prohibited therapies;
- Compliance with the Clinical Trial Protocol with regard to intervals between visits and total treatment duration; and
- Whether planned activity and safety evaluation were performed, etc.

Deviations covered will include but not be limited to:

- Subjects without any evaluation (of any variables) after randomization;
- Subjects not exposed;
- Subject without any evaluation of the primary variable (if relevant);
- Subjects who entered the study even though they did not satisfy the inclusion criteria;
- Subjects who developed withdrawal criteria during the study but were not withdrawn;
- Subjects who received the wrong treatment or incorrect dose;
- Subjects who received a prohibited concomitant medication.

Major deviations will be listed and summarized.

11.3 ANALYSIS POPULATION

All exclusions from any analysis populations (pharmacodynamic, pharmacokinetic and/or safety) will be fully documented in the CSR.
Subjects excluded from any analysis population will be listed with treatment sequence, and with reason for exclusion. Any relevant information will be fully documented in the CSR. Frequencies of subjects, overall and per treatment, for the analysis populations will be tabulated.
For the event of subjects having received treatments that differed from those assigned according to the randomization schedule, analyses will be conducted according to the treatment received rather than according to the randomized treatment.

11.3.1 Pharmacodynamic population

All subjects without any major deviations related to study drug administration, and for whom PD parameters are available, will be included in the pharmacodynamic population. For subjects with insufficient PD profiles in one but not both treatment periods, parameters of the sufficient profiles will be included in the analysis.
The pharmacodynamic data for insulin glargine of those subjects will be excluded from evaluation, who will receive (for safety reasons) insulin glulisine within the observation period of 24 h after IP administration.

11.3.2 Safety population

All subjects who were exposed to any comparative study treatment, regardless of the amount of treatment administered, will be included in the safety population.

11.3.3 Pharmacokinetic populations

All subjects without any major deviations related to study drug administration, and for whom PK parameters will be available, will be included in the corresponding pharmacokinetic population (see below).

*11.3.3.1 Pharmacokinetic population for insulin glargine*

All subjects without any major deviations related to study drug administration, and for whom insulin PK parameters will be available, will be included in the pharmacokinetic population for insulin glargine. For subjects with insufficient insulin PK profiles at one but not both treatment periods, parameters of the sufficient profiles will be included in the analysis.
The bioanalytical assay for insulin glargine is interfered by other insulins like insulin glulisine.
Therefore, the pharmacokinetic data for insulin glargine of those subjects will be excluded from evaluation, who will receive (for safety reasons) insulin glulisine within the observation period of 24 h after IP administration

*11.3.3.2 Pharmacokinetic population for lixisenatide*

All subjects without any major deviations related to study drug administration, and for whom lixisenatide PK parameters will be available, will be included in the pharmacokinetic population for lixisenatide. For subjects with insufficient lixisenatide PK profiles at one but not both study treatment periods, parameters of the sufficient profiles will be included in the analysis. Antibody formation impacts the exposure of lixisenatide, and thus if detected in a subject during the course of this study will lead to exclusion of the respective treatment period for PK evaluation of lixisenatide.

11.4 DEMOGRAPHIC AND BASELINE CHARACTERISTICS

11.4.1 Subject demographic characteristics, medical history and diagnoses The following data will be collected: sex, age, height, weight, and race. Baseline body mass index (BMI) per subject will be calculated from pre-dose body weight and height data:

$$BMI = body\ weight\ [kg]\ /\ (height\ [m])^2$$

All variables concerning demographic and background characteristics will be listed individually and summarized for the safety population.
Deviations from inclusion criteria related to medical history and diagnoses will be listed and described individually.

11.4.2 Baseline pharmacodynamic parameters

None.

11.4.3 Baseline safety parameters

For safety variables, the latest scheduled value before study drug administration within the period or within the study, whatever is applicable for the variable, will be taken as the baseline value. If the baseline pre-dosing value is rechecked before dosing, the rechecked value will be considered as the baseline and used in statistics.

11.5 EXTENT OF STUDY TREATMENT EXPOSURE AND COMPLIANCE

Details of study drug dosing and complementary information will be listed individually and summarized if appropriate.
Individual total doses of insulin glargine will be summarized by treatment.

11.6 PRIOR/CONCOMITANT MEDICATION/THERAPY

Prior and concomitant medications/therapies (if any) will be coded according to the World Health Organization-Drug Reference List (WHO-DRL, latest version in use at time of database lock) and will be listed individually.
Concomitant insulin medication will be listed separately.

11.7 ANALYSIS OF PHARMACODYNAMIC VARIABLES

All pharmacodynamic analyses will encompass data of the pharmacodynamic population (defined in Section 0). No adjustment of the alpha-level will be made for multiple analyses.

For pharmacodynamics of insulin glargine given with lixisenatide, the blood glucose concentration and glucose infusion rate (GIR) will be continuously recorded during the clamp procedure.

11.7.1 Description of pharmacodynamic variables

In order to achieve comparability between the subjects under the body weight depending insulin dosing, all values for GIR will be divided by the subject's body weight in kg for analysis. Thus in the below, if not stated otherwise, GIR always refers to the body weight standardized glucose infusion rate.

*11.7.1.1 Primary PD variable*

None of the PD variables will be considered primary.

*11. 7.1.2 Secondary PD variables*

The following PD variables will be derived and considered secondary:

- Area under the body weight standardized glucose infusion rate time curve [GIR-AUC$_{(0-24)}$ (mg/kg)]
- Time (h) to 50% of GIR-AUC$_{(0-24)}$ [T50%-GIR-AUC$_{(0-24h)}$ (h)]
  GIR-AUC$_{(0-24)}$ will be calculated according to the rectangular rule for the stepwise constant function with timescale in minutes.

*11.7.1.3 Additional PD variables*

The following additional PD variables will be also derived:

- Maximum smoothed body weight standardized glucose infusion rate [GIR$_{max}$ (mg/kg/min)]
- Time to GIR$_{max}$ [GIR-T$_{max}$ (h)]

The maximum of the raw body weight standardized GIR will be subject to the noise in the GIR adjustment. Thus, the derivation of GIR$_{max}$ and the time to GIR$_{max}$, will be based upon a LOESS (locally weighted regression in smoothing scatterplots) smoothing technique for the raw body weight standardized GIR data. Due to the expected morphology of the GIR-profiles as known under Lantus, a smoothing factor of 6% will be used (SAS, PROC LOESS, factor 0.06).

Further supplemental PD variables

Further supplemental parameters will be derived, as:

- Time to end of glucose infusion will be derived as the latest time after dosing with GIR above zero.
- Time to end of effect will be derived as the latest time with blood glucose at 130 mg/dL or below. In particular, it will be defined as time of end of clamp (censoring) where blood glucose is at 130 mg/dL or below at end of clamp.

Additional PD variables may be derived if deemed necessary for interpretation of results.

11.7.2 Primary PD analysis

None of the PD analyses will be considered primary.

11.7.3 Secondary analysis / analysis of secondary variables

Statistical analyses will compare test treatment (T) with the reference treatment (R).

Prior to the analysis described below, GIR-AUC$_{(0-24)}$ will be log-transformed (natural log).

Log-transformed GIR-AUC$_{(0-24)}$ will be analyzed with a linear mixed effects model with fixed terms for sequence, period and treatment

$$log(parameter) = sequence + period + treatment + error,$$

and with an unstructured R matrix of treatment (i, i) variances and covariances for subject within sequence blocks, using SAS PROC MIXED.

90% confidence interval (CI) for the ratio of treatments geometric means (T/R) will be obtained by computing estimate and 90% CI for the difference between treatment means within the linear mixed effects model framework, and then converting to ratio of geometric means by the antilog transformation. Equivalence will be concluded if the 90% CI for the ratio is entirely within the 0.80 to 1.25 equivalence reference interval.

Listings of individual ratios (test treatment versus reference treatment, T/R) will be provided with the corresponding descriptive statistics.

T50%-GIR-AUC$_{(0-24)}$ (h) will be analysed non-parametrically. CIs for the treatment difference in medians will be derived by Hodges-Lehmann method.

The distribution of T50%-GIR-AUC$_{(0-24)}$ values will be represented by histogram plots for each treatment. In addition, a histogram of differences in T50%-GIR-AUC$_{(0-24)}$ between treatments (T-R) will be provided.

*11.7.3.1 Descriptive presentations for GIR profiles*

Individual body weight standardized GIR (mg/kg/min) will be plotted for raw, smoothed and cumulative raw values.

Mean and median body weight standardized GIR-profiles as well as mean and median percentage cumulative profiles (24 h) over time will be plotted by treatment.

*11.7.3.2 Descriptive presentations for derived PD parameters*

PD parameters will be listed individually, and descriptive statistics will be generated by treatment.

*11.7.3.3 Treatment comparison for further PD parameters*

Treatment ratios (T/R) with confidence limits will be derived for maximum standardized glucose infusion rate [GIR$_{max}$ (mg/kg/min)] using the corresponding linear mixed effects model as described above. Exploratory comparisons between treatments will be based on conventional bioequivalence criteria (90% confidence limits 0.80 to 1.25).

The distribution of GIR-T$_{max}$ values will be represented by histogram plots for each treatment. In addition, a histogram of differences in GIR-T$_{max}$ between treatments will be provided.

*11.7.3.4 Performance of clamp*

Individual profiles of blood glucose concentration will be plotted.

Duration of clamp will be derived per clamp as the time between dosing and end of clamp in hours.

Individual variability of blood glucose per clamp will be derived as the coefficient of variation (CV%) of blood glucose values between individual start and individual end of clamp. Individual average blood glucose level per clamp will be derived as the arithmetic mean of blood glucose values between individual start and individual end of clamp.

Parameters will be listed individually and summarized descriptively within treatment.

11.8 ANALYSIS OF SAFETY DATA

The safety evaluation will be based upon the review of the individual values (potentially clinically significant abnormalities), descriptive statistics (summary tables, graphics) and if needed on statistical analysis (appropriate estimations, confidence intervals). "Potentially Clinically Significant Abnormalities" (PCSA) criteria will be used according to standard criteria of sanofi-aventis. Criteria will be documented in the statistical analysis plan of this study. The safety analysis will be conducted according to the sanofi-aventis standards related to analysis and reporting of safety data from clinical trials.

All safety analyses will encompass data of the safety population.

For all safety data, the observation period will be divided into segments of three different types:

- the pre-treatment period is defined as the time between when the subject gives informed consent and the first administration of comparative study medication.

- the on-treatment period per period is defined as the time from (first) study medication administration up to 72 hours later.
- the post-treatment period is defined as the time after on-treatment period to either the (first) administration of study medication in the next period or the end of the follow-up period.

### 11.8.1 Adverse events

All AEs will be coded using MedDRA (latest version in use at time of database lock).
The following listings will be provided for all adverse events:

- Listing of all adverse events (by subject)
- Listing of comments related to adverse events

#### *11.8.1.1 Definitions*

For safety data, the observation period will be divided into segments of three different types:

- the pre-treatment period is defined as the time between when the subject gives informed consent and the first administration of comparative study medication.
- the on-treatment period per period is defined as the time from (first) study medication administration up to 72 hours later.
- the post-treatment period is defined as the time after on-treatment period to either the (first) administration of study medication in the next period or the end of the follow-up period.

### Treatment emergent adverse events

All AEs will be classified as follows:

- Treatment-emergent adverse events (TEAEs): Any AE with an onset (incl. worsening) during an on-treatment period
- Non-treatment-emergent adverse events (NTEAEs): Any AE not classified as TEAE#
- Pre-treatment AEs, defined as AEs that developed (or worsened) during the pre-treatment period before the first dose of comparative study medication
- Post-treatment AEs, defined as AEs that developed during a post-treatment period without worsening during an on-treatment phase.

### Assignment to treatments

For analysis purposes, each TEAE will be assigned to the last treatment given before onset (or worsening) of the AE. If a TEAE develops on one treatment and worsens under a later treatment, it will be considered treatment emergent for both treatments.

### Missing information

In case of missing or inconsistent information, an AE will be counted as a TEAE, unless it can clearly be ruled out that it is not a TEAE (e. g. by partial dates or other information).
If the start date of an AE is incomplete or missing, it will be assumed to have occurred after the first administration of study medication except if an incomplete date indicates that the AE started prior to treatment.

#### *11.8.1.2 Treatment-emergent adverse events*

Treatment emergent adverse events will be listed and summarized by treatment:

- Overview of TEAEs (number and percentage of subjects with at least one TEAE, severe TEAE, TEAE leading to discontinuations, death (if any))
- Summary of all treatment-emergent adverse events by primary system organ class and preferred term (number and percentage of subjects with at least one TEAE) ("in-text table")

  - Table without number of events (for body of the clinical study report)

- Table with number of events (for appendix of the clinical study report)

• Listing of subjects presenting treatment emergent adverse events by treatment, system organ class and preferred term

*11. 8.1.3 Deaths, serious and other significant adverse events*

In case of any occurrences, deaths, serious AEs, and other significant AEs will be listed individually and described in the study report in detail.

*11.8.1.4 Adverse events leading to treatment discontinuation*

In case of any occurrences, individual subject listings will be generated for all adverse events leading to treatment discontinuation.

11.8.2 Clinical laboratory evaluations

*11.8.2.1 Hematology and biochemistry data*

Laboratory safety parameters will be measured on D1 of each period and at EOS. Per schedule, these safety parameters will not be assessed during the on-treatment period.
The values to be used as baseline (hematology and biochemistry) will be the values collected on D1 predose in the first treatment period. If any of the scheduled baseline tests are repeated for any subject, the last rechecked values will be considered as baselines, provided they were done before the first IP administration.
The following tables and listings will be provided:

• A specific listing of individual data from subjects with post-baseline PCSAs will be provided, sorted by function and time of measurement
• All individual data, including rechecked values, for planned hematology and biochemistry, will be listed by biological function and time of measurement. If any, data from unscheduled laboratory tests will also be included in this listing. In these listings, individual data will be flagged when lower or higher than the lower or upper laboratory limits and/or when reaching the absolute limit of PCSA criteria, when defined
• A listing of liver function data for subjects, who experienced at least one occurrence of ALT > 3ULN and at least one occurrence of total bilirubin > 2 ULN during the study with at least one of them being post first dose, will be also provided. Liver function data will be expressed as multiple of the corresponding ULN
• A listing with subjects with conjugated bilirubin > 35% total bilirubin and total bilirubin > 1.5 ULN will be provided
• A listing related to increase in ALT $\geq$2 ULN will be provided, including notably the information on drug intake, medical and surgical history, alcohol habits, trigger factors, event details with ALT values, associated signs and symptoms.
• Descriptive statistics per treatment for raw data and changes from previous baseline
• A listing of out-of-range definitions will be provided.

In the listings of subjects with PCSAs, liver function data, CPK, and eosinophils will be expressed as multiple of the corresponding ULN.

*11.8.2.2 Urinalysis data*

All qualitative urinary test results (dipstick), including rechecked values, will be listed.

11.8.3 Vital signs

*11.8.3.1 Blood pressure and heart rate*

Heart rate and systolic and diastolic blood pressure (SBP and DBP) are measured after 10 minutes in supine resting position and also after 3 minutes in standing position, except when connected to the Biostator.
The values to be used as the baselines will be the D1 pre-dose assessment value of each treatment period. If any of the scheduled baseline tests are repeated for any subject, the last rechecked values will be considered as baselines, provided they were done before the IP administration.
For heart rate and blood pressures, orthostatic differences will be calculated as the change from supine to standing position.

For all parameters, an "On-Treatment" analysis will be performed including all unplanned values and rechecked values.

The following tables and listings will be provided:

- Summary tables of counts of subjects with PCSAs will be provided as incidence tables of post-baseline PCSAs, regardless of the normal or abnormal status of the baseline

- For heart rate and blood pressures (supine and standing positions), raw data and changes from baseline (supine position only) will be summarized in descriptive statistics, for type of measurement (position) each parameter and time point, based on planned pre-dose measurements and the baseline defined

- All individual data, including unplanned and rechecked values, will be listed (supine, standing orthostatic difference). In the listings, values will be flagged when reaching the limits of the PCSA criteria when defined

- A data listing of individual post-baseline PCSAs will be provided

- Comments related to vital sign evaluations will also be listed in the Appendix, if any.

*11.8.3.2 Body weight, body mass index, and body temperature*

The values to be used as baselines for body weight and BMI will be the values collected on D1 of TP1. The values to be used as baselines for body temperature will be the values collected on D1 of each TP. Individual data will be listed including flags (weight only) for values when reaching the limits of the PCSA criteria.

11.8.4 ECG

Heart rate, PQ-, QRS-, and QT-intervals and corrected QT (QTc) from automatic reading will be analyzed as raw parameter value and change from baseline.

The values to be used as the baseline will be the Day 1 predose value of each period. If any of the scheduled baseline tests are repeated for any subject, the rechecked values will be considered as baselines, provided they were done before the drug administration of the period.

For all parameters, an on-treatment analysis will be performed using all post-baseline assessments done during the on-treatment period, including rechecked values. Counts of subjects with postbaseline PCSAs will be provided in summary tables regardless of the normal or abnormal status of the baseline, by treatment group.

Raw data for all parameters will be summarized in descriptive statistics by parameter, treatment, visit and time of measurement.

Individual data, including rechecked values, will be listed, sorted by treatment, subject, visit and time of measurement. In the listings, values reaching the limits of the PCSA criteria will be flagged.

A listing of individual data from subjects with post-baseline PCSAs will be provided, sorted by type of measurement and sorted by period, subject, visit and time of measurement.

Additionally, a separate listing of the cardiac profile for subjects with prolonged QTc (>450 ms for Males and >470 ms for Females) or changes from baseline in QTc >60 ms (for males and females) and a listing of subjects with at least one abnormality in qualitative assessment (i.e., abnormal ECG) after the 1st dosing will be also provided.

11.8.5 Other related safety parameters

*11.8.5.1 Anti-lixisenatide antibodies*

If appropriate, a summary table will be provided with the number of subjects developing anti-lixisenatide antibodies during the study. Individual subject listing will be provided. Comments, if any, will be listed in the appendix.

Handling of data for anti-lixisenatide antibodies

Anti-lixisenatide antibody data will be provided by sanofi-aventis / Global Metabolism and Pharmacokinetics.

Database will be locked after availability of anti-lixisenatide antibody levels at post-study visit. If subjects are lixisenatide antibody positive at this post-study visit (PSV), database will be unlocked for uploading of anti-lixisenatide antibody data obtained from follow-up visit (FUV).

All results for anti-lixisenatide antibodies, including data from PSV and FUV will be contained in the final database for this study and will be covered by final analysis of this study

*11.8.5.2 Physical Examination*

Listing of comments related to physical examination will be provided, if any.

*11.8.5.3 Local tolerability at injection site*

Frequency distributions by treatment will be provided for levels of local tolerability at injection site. Individual data will be listed.

*11.8.5.4 Allergic reactions*

Listings for allergic reactions

Any cases of allergic reaction will be documented as adverse events with detailed complementary information. All cases will be described in detail in the clinical study report.
Individual cases and all complementary data will be listed.

Allergic medical history and family medical history

Allergic medical history and family medical history is to be documented for subjects with any occurrence of potential allergic reaction. All details of allergic medical history and of allergic family medical history will be listed on an individual basis.

11.9 ANALYSIS OF PHARMACOKINETIC DATA

11.9.1 Pharmacokinetic parameters

The list of PK parameters is listed in Section 0. In addition, $T_{50\%}$-$AUC_{(0-24)}$ for insulin will be derived in the context of the statistical analysis.

11.9.2 Statistical analysis

Pharmacokinetic parameters of lixisenatide and insulin glargine will be listed and summarized using at least arithmetic and geometric means, standard deviation (SD), standard error of the mean (SEM), coefficient of variation (CV%), minimum, median and maximum for each treatment.
All pharmacokinetic analyses will encompass data of the corresponding pharmacokinetic populations as defined in Section 0. No adjustment of the alpha-level will be made for multiple analyses.
Statistical analyses will compare test treatment (T) versus reference treatment (R).

*11.9.2.1 Analysis of treatment ratios*

Prior to all analysis described below, $C_{max}$, $AUC_{last}$ and AUC values will be log-transformed (natural log). The analysis will be performed for $C_{max}$, $AUC_{last}$ and AUC for lixisenatide and for $AUC_{0-24}$ for insulin glargine.
Log-transformed parameters will be analyzed with a linear mixed effects model with fixed terms for sequence, period and treatment

$$log(parameter) = sequence + period + treatment + error,$$

and with an unstructured R matrix of treatment (i, i) variances and covariances for subject within sequence blocks, using SAS PROC MIXED.
For these parameters, estimate and 90% confidence interval (CI) for the ratio of treatments geometric means (Test / Reference) will be obtained by computing estimate and 90% CI for the difference between treatment means within the linear mixed effects model framework, and then converting to ratio of geometric means by the antilog transformation.

Bioequivalence will be concluded if the 90% CI for the ratio is entirely within the 0.80 to 1.25 equivalence reference interval.

Listings of individual treatment ratios (T/R) will be provided with the corresponding descriptive statistics.

### 11.9.2.2 $T_{50\%}$-$AUC_{(0-24)}$ for insulin

The distribution of $T_{50\%}$-$AUC_{(0-24)}$ values for insulin will be represented by histogram plots for each treatment. In addition, a histogram of differences in $T50\%$-$AUC_{(0-24)}$ between treatments (T-R) will be provided.

$T50\%$-$AUC_{(0-24)}$ (h) will be analysed non-parametrically. CIs for the treatment difference in medians will be derived by Hodges-Lehmann method.

### 11.9.2.3 $T_{max}$ for lixisenatide

$T_{max}$ will be summarized by range and median for each treatment.

The distribution of $T_{max}$ values will be represented by histogram plots for each treatment. In addition, a histogram of differences in $T_{max}$ between treatments (T-R) will be provided.

### 11.10 PK/PD ANALYSIS

If appropriate, graphical displays (e. g. scatter plots) will be generated to explore PK/PD relationship.

### 11.11 INTERIM ANALYSIS

No interim analysis is planned.

### 12 ETHICAL AND REGULATORY STANDARDS

### 12.1 ETHICAL PRINCIPLES

This Clinical Trial will be conducted in accordance with the principles laid down by the 18th World Medical Assembly (Helsinki, 1964) and all applicable amendments laid down by the World Medical Assemblies, and the ICH guidelines for Good Clinical Practice (GCP).

### 12.2 LAWS AND REGULATIONS

This Clinical Trial will be conducted with all international laws and regulations and, national laws and regulations of the country(ies) in which the Clinical Trial is performed, as well as any applicable guidelines.

### 12.3 INFORMED CONSENT

The Investigator (according to applicable regulatory requirements), or a person designated by the Investigator and under the Investigator's responsibility, should fully inform the subject of all pertinent aspects of the Clinical Trial including the written information giving approval/favorable opinion by the Ethics Committee (IRB/IEC) and Health Authorities. All participants should be informed to the fullest extent possible about the study, in language and terms they are able to understand.

Prior to a subject's participation in the Clinical Trial, the written Informed Consent Form should be signed, name filled in and personally dated by the subject or by the subject's legally acceptable representative, and by the person who conducted the informed consent discussion. A copy of the signed and dated written Informed Consent Form will be provided to the subject. The Informed Consent Form used by the Investigator for obtaining the subject's informed consent must be reviewed and approved by the Sponsor prior to submission to the appropriate Ethics Committee (IRB/IEC) and Health Authorities for approval/favorable opinion.

### 14.3.1 INSTITUTIONAL REVIEW BOARD/INDEPENDENT ETHICS COMMITTEE (IRB/IEC)

As required by local regulation, the Investigator or the Sponsor must submit this Clinical Trial Protocol to the appropriate Ethics Committee and Health Authorities, and is required to forward to the respective other party a copy of the written and dated approval/favorable opinion signed by the Chairman with Ethics Committee composition and Health Authorities.

The Clinical Trial (study code, Clinical Trial Protocol title and version number), the documents reviewed (Clinical Trial Protocol, Informed Consent Form, Investigator's Brochure, Investigator's CV, etc.) and the date of the review should be clearly stated on the written (IRB/IEC) and Health Authorities approval/favorable opinion.

Investigational Product will not be released at the study site and the Investigator will not start the study before the written and dated approval/favorable opinion is received by the Investigator and the Sponsor.

During the Clinical Trial, any amendment or modification to the Clinical Trial Protocol should be submitted to the Ethics Committee (IRB/IEC) and Health Authorities before implementation, unless the change is necessary to eliminate an immediate hazard to the patients, in which case the IRB/IEC should be informed as soon as possible. It should also be informed of any event likely to affect the safety of subjects or the continued conduct of the Clinical Trial, in particular any change in safety. All updates to the Investigator's Brochure will be sent to the Ethics Committee (IRB/IEC).

A progress report is sent to the Ethics Committee (IRB/IEC) and Health Authorities at least annually and a summary of the trial's outcome at the end of the Clinical Trial.

## 13. USE OF COMPUTERIZED SYSTEMS

Computerized systems used during the different steps of the study are:

- For data entry and management activities, Oracle Clinical RDC version 4.5.3
- For pharmacokinetic activities, WinNonlin, PKDMS
- For bioanalytical data, WATSON
- For statistical activities, SAS® (version 9.1, SAS Institute, NC USA)
- For pharmacovigilance activities, Clintrace
- For monitoring activities, IMPACT
- For medical writing activities, DOMASYS.

Data loading is planned for PD, lab and for anti-lixisenatide antibody data of this clinical trial.

## 14. EVALUATION OF SKIN RESPONSES

Reactions to the respective injections will be documented on case record forms through a numerical scoring system defined below:

Global irritation score:

| Score | Definition |
|-------|-----------|
| 0 | No reaction |
| 1 | Hardly perceptible erythema |
| 2 | Mild : slight erythema with or without slight edema |
| 3 | Moderate : moderate erythema, edema, with or without papules |
| 4 | Intense : marked erythema, edema, induration, with or without papules |
| 5 | Severe : intense erythema with edema, vesicles or blisters |

All reactions with a score ≥3 will be reported as adverse events in the case record forms.

## 15. BIBLIOGRAPHIC REFERENCES

1. American Diabetic Association. Report of the Expert Committee on the Diagnosis and Classification of Diabetes Mellitus. Diabetes Care 1998;21:5-19

2. Samspon HA, Munoz-Furlong A, Campbell RL et al. Second symposion on the definition and management of anaphylaxis: summary report - Second National Institute of Allergy and Infectious Disease/Food Allergy and Anaphyiaxis Network symposium. Journal of Allergy and Clinical Immunology 2006;117(2):391-397

3. Pharmacy Manual

The subject-matter of the present application is further described by the following items:

1. A pharmaceutical combination comprising

    (a) desPro$^{36}$Exendin-4(1-39)-Lys$_6$-NH$_2$ or/and a pharmaceutically acceptable salt thereof, and
    (b) insulin glargine or/and a pharmaceutically acceptable salt thereof,

wherein the concentration of compound (a) is in the range of 20 - 120 µg/ml, and wherein the concentration of compound (b) is in the range of 40 - 200 U/ml.

2. The combination of item 1, for use in the treatment of diabetes mellitus type 1 or 2 by administration of a dose of 0.25 - 1.5 U/kg insulin glargine and 0.05 - 0.5 µg/kg desPro$^{36}$Exendin-4(1-39)-Lys$_6$-NH$_2$.

3. The combination of item 2, for use in the treatment of diabetes mellitus type 2.

4. Use of a combination of

    (a) desPro$^{36}$Exendin-4(1-39)-Lys$_6$-NH$_2$ or/and a pharmaceutically acceptable salt thereof, and
    (b) insulin glargine or/and a pharmaceutically acceptable salt thereof,

wherein the concentration of compound (a) is in the range of 20 - 120 µg/ml, and wherein the concentration of compound (b) is in the range of 40 - 200 U/ml, for the preparation of a medicament for the treatment of diabetes mellitus type 1 or 2.

5. The use of item 4, wherein diabetes mellitus type 1 or 2 is treated by administration of a dose of 0.25 - 1,5 U/kg insulin glargine and 0.05 - 0.5 µg/kg desPro$^{36}$Exendin-4(1-39)-Lys$_6$-NH$_2$.

6. The use of item 4 or 5, wherein the medicament is prepared for the treatment of diabetes mellitus type 2.

7. A method of treatment of diabetes mellitus type 1 or/and type 2, comprising administering to a subject in need thereof a composition comprising

    (a) desPro$^{36}$Exendin-4(1-39)-Lys$_6$-NH$_2$ or/and a pharmaceutically acceptable salt thereof, and
    (b) insulin glargine or/and a pharmaceutical acceptable salt thereof,

wherein the concentration of compound (a) is in the range of 20 - 120 µg/ml, and wherein the concentration of compound (b) is in the range of 40 - 200 U/ml.

8. The method of item 7, wherein diabetes mellitus type 1 or 2 is treated by administration of a dose of 0.25 - 1.5 U/kg insulin glargine and 0.05 - 0.5 µg/kg desPro$^{36}$Exendin-4(1-39)-Lys$_6$-NH$_2$.

9. The method of item 7, wherein diabetes mellitus type 2 is treated.

**Claims**

1. A pharmaceutical composition comprising

   (a) desPro$^{36}$Exendin-4(1-39)-Lys$_6$-NH$_2$ or/and a pharmaceutically acceptable salt thereof, and
   (b) insulin glargine or/and a pharmaceutically acceptable salt thereof,

   wherein the concentration of compound (a) is in the range of 20 - 120 $\mu$g/ml, and wherein the concentration of compound (b) is in the range of 40 - 200 U/ml.

2. The pharmaceutical composition of claim 1, for use in the treatment of diabetes mellitus type 1 or 2 by administration of a dose of 0.25 - 1.5 U/hg insulin glargine and 0.05 - 0.5 $\mu$g/kg desPro$^{36}$Exendin-4(1-39)-Lys$_6$-NH$_2$.

3. The pharmaceutical composition of claim 2, for use in the treatment of diabetes mellitus type 2.

4. The pharmaceutical composition of claim 2 or 3, wherein the composition is administered parenterally.

5. The pharmaceutical composition of any of the claims 2 to 4, wherein the subject to be treated is an adult subject.

6. The pharmaceutical composition of any of the claims 2 to 5, wherein the subject to be treated is obese.

7. The pharmaceutical composition of any of the claim 6, wherein the subject has a body mass index of at least 30.

8. The pharmaceutical composition of any of the claims 2 to 7, wherein diabetes mellitus type 2 is not adequately controlled with insulin alone.

9. The pharmaceutical composition of any of the claims 2 to 8, wherein the subject to be treated has a HbA1c value in the range of 7 % to 10% or/and a fasting plasma glucose concentration of at least 7 mmol/L or/and 2 hours postprandial plasma glucose of at least 11.1 mmol/L.

Figure 1 – Mean (SD) Lixisenatide Plasma Concentrations for Treatment R1 and T1 on  linear Scales

Figure 2 – Mean (SD) Lixisenatide Plasma Concentrations for Treatment R2 and T2 on linear Scales

Figure 3 – Mean (SD) Insulin Glargine Serum Concentrations for Treatment R1 and T1 on linear scales

Figure 4 – Mean (SD) Insulin Glargine Serum Concentrations for Treatment R2 and T2 on linear Scales

Figure 5 – GIR (Mean raw and Mean smoothed profiles) – Treatment=R1 (separate)

AVE0010 / BDR10880
Glucose Infusion Rate (body weight standardized)
(Mean raw and mean smoothed profiles up to 24h after dosing)
Treatment=R1 (separate)

GIR = body weight standardized Glucose Infusion Rate

Strength 1 (R1 and T1) and Strength 2 (R2 and T2) are treatments per kg body weight of 0.4 U/kg insulin glargine combined with 0.264 µg/kg and 0.100 µg/kg lixisenatide respectively.

R1 and R2 are separate simultaneous injections; T1 and T2 are injections of premixed formulation

Figure 6 – GIR (Mean raw and Mean smoothed profiles) – Treatment=T1 (mixed)

AVE0010 / BDR10860
Glucose Infusion Rate (body weight standardized)
(Mean raw and mean smoothed profiles up to 24h after dosing)
Treatment=T1 (mixed)

GIR = body weight standardized Glucose Infusion Rate

Strength 1 (R1 and T1) and Strength 2 (R2 and T2) are treatments per kg body weight of 0.4 U/kg insulin glargine combined with 0.264 μg/kg and 0.100 μg/kg lixisenatide respectively.

R1 and R2 are separate simultaneous injections; T1 and T2 are injections of premixed formulation

Figure 7 – GIR (Mean raw and Mean smoothed profiles) – Treatment=R2 (separate)

AVE0010 / BDR10880
Glucose Infusion Rate (body weight standardized)
(Mean raw and mean smoothed profiles up to 24h after dosing)
Treatment=R2 (separate)

GIR = body weight standardized Glucose Infusion Rate

Strength 1 (R1 and T1) and Strength 2 (R2 and T2) are treatments per kg body weight of 0.4 U/kg insulin glargine combined with 0.264 µg/kg and 0.100 µg/kg lixisenatide respectively.

R1 and R2 are separate simultaneous injections; T1 and T2 are injections of premixed formulation

Figure 8 – GIR (Mean raw and Mean smoothed profiles) – Treatment=T2 (mixed)

AVE0010 / BDR10880
Glucose Infusion Rate (body weight standardized)
(Mean raw and mean smoothed profiles up to 24h after dosing)
Treatment=T2 (mixed)

GIR = body weight standardized Glucose Infusion Rate

Strength 1 (R1 and T1) and Strength 2 (R2 and T2) are treatments per kg body weight of 0.4 U/kg insulin glargine combined with 0.264 µg/kg and 0.100 µg/kg lixisenatide respectively.

R1 and R2 are separate simultaneous injections; T1 and T2 are injections of premixed formulation

Figure 9

Screening | Period 1/2 D -2 | Period 1/2 D -1 | Period 1/2 D 1 | Period 1/2 D 2 | EOS | Post study visits
Informed consent | Preparation | Admission | Medication | Discharge | Follow up | Antibody check
| | Experimental set up | Randomization | | | * if anti-AVE0010
| | Inclusion check | PK sampling | | | antibody positive

R = reference (separate simultaneous injections), T = test (injection of premixed formulations)
1 = strength I, 2 = strength II

Figure 10

| Screening | Period 1/2/3 D -2 | Period 1/2/3 D1 | | Period 1/2/3 D 2 | EOS | Post study visit |
|---|---|---|---|---|---|---|
| Informed consent | Preparation | Admission | Medication | PK sampling ctd | Follow up | AB check |
| | | Inclusion check | Randomization | Discharge | | |
| | | Experimental set up | PK sampling | | | |

R = reference, T = test (1 strength A, 2 strenghth B)

Figure 11 – Mean (SD) Lixisenatide Plasma Concentrations for Treatment Reference (separate), Test (mix A), and Test 2 (mix B) on linear Scales

Figure 12 – Mean (SD) Insulin Glargine Serum Concentrations for Treatment Reference (separate), Test (mix A), and Test 2 (mix B) on linear Scales

Figure 13 – GIR (Mean raw and Mean smoothed Profiles) – Treatment=R (separate)

AVE0010 / BDR11038
Glucose Infusion Rate (body weight standardized)
(Mean raw and mean smoothed profiles up to 24h after dosing)
Treatment=R (separate)

Figure 14 – GIR (Mean raw and Mean smoothed Profiles) – Treatment=T1 (mix A)

AVE0010 / BDR11038
Glucose Infusion Rate (body weight standardized)
(Mean raw and mean smoothed profiles up to 24h after dosing)
Treatment=T1 (mix A)

Figure 15 – GIR (Mean raw and Mean smoothed Profiles) – Treatment=T2 (mix B)

AVE0010 / BDR11038
Glucose Infusion Rate (body weight standardized)
(Mean raw and mean smoothed profiles up to 24h after dosing)
Treatment=T1 (mix A)

Figure 16

R = reference, T = test (injection of on-site mix)

Europäisches
Patentamt

European
Patent Office

Office européen
des brevets

# EUROPEAN SEARCH REPORT

Application Number

EP 14 19 7154

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2008 053048 A1 (SANOFI AVENTIS DEUTSCHLAND [DE]) 29 April 2010 (2010-04-29) * paragraphs [0020], [0032], [0082], [0083], [0092], [0083], [0020] * * examples 1,11 * * claim 21 * ----- | 1-9 | INV. A61K38/26 A61K38/28 A61P3/10 A61P5/50 |

TECHNICAL FIELDS
SEARCHED      (IPC)

A61K

The present search report has been drawn up for all claims

| Place of search | Date of completion of the search | Examiner |
|---|---|---|
| The Hague | 30 March 2015 | Strack, Eberhard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

 

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 14 19 7154

This annex lists the patent family members relating to the patent documents cited in the above-mentioned European search report.
The members are as contained in the European Patent Office EDP file on
The European Patent Office is in no way liable for these particulars which are merely given for the purpose of information.

30-03-2015

| Patent document cited in search report | Publication date | Patent family member(s) | Publication date |
|---|---|---|---|
| DE 102008053048 A1 | 29-04-2010 | NONE | |

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0104156 A

**Non-patent literature cited in the description**

- Report of the Expert Committee on the Diagnosis and Classification of Diabetes Mellitus. *Diabetes Care,* 1998, vol. 21, 5-19

- **SAMSPON HA ; MUNOZ-FURLONG A ; CAMPBELL RL et al.** Second symposion on the definition and management of anaphylaxis: summary report - Second National Institute of Allergy and Infectious Disease/Food Allergy and Anaphyiaxis Network symposium. *Journal of Allergy and Clinical Immunology,* 2006, vol. 117 (2), 391-397